# EUROPEAN PATENT APPLICATION

(11) **EP 4 667 589 A1**
(43) Date of publication of application: **24.12.2025**
(21) Application number: 24382675.7
(22) Date of filing: 21.06.2024
(51) Int. Cl.: C12Q 1/689

(54) **IN VITRO METHOD FOR THE DIAGNOSIS OF A MYCOPLASMA PNEUMONIAE INFECTION AND/OR FOR THE DIFFERENTIAL DIAGNOSIS BETWEEN A MYCOPLASMA PNEUMONIAE PNEUMONIA AND OTHER BACTERIAL OR VIRAL PNEUMONIAS**

(71) Applicant: Fundación Pública Galega Instituto de Investigación Sanitaria de Santiago de Compostela, 15706 Santiago de Compostela A Coruña (ES); Servizo Galego De Saúde, 15703 Santiago de Compostela, A Coruna (ES); Universidade de Santiago de Compostela, 15782 Santiago de Compostela (ES)
(72) Inventor: GÓMEZ CARBALLA, Alberto, 15706 Santiago de Compostela (ES); VIZ LASHERAS, Sandra, 15706 Santiago de Compostela (ES); MARTINÓN TORRES, Federico, 15706 Santiago de Compostela (ES); SALAS ELLACURIAGA, Antonio, 15706 Santiago de Compostela (ES)
(74) Representative: Hoffmann Eitle

(57) **Abstract**

The present invention refers to an *in vitro* method for the diagnosis of a *Mycoplasma pneumoniae* infection and/or for the differential diagnosis between *a Mycoplasma pneumoniae* pneumonia and other bacterial or viral pneumonias and/or for assessing whether to treat a patient suffering from pneumonia with a macrolide antibiotic.

## Description

### FIELD OF THE INVENTION

The present invention refers to the medical field. Particularly, it refers to an *in vitro* method for the diagnosis of a *Mycoplasma pneumoniae* infection and/or for the differential diagnosis between a *Mycoplasma pneumoniae* pneumonia and other bacterial or viral pneumonias. The present invention also refers to the possibility of selecting the antibiotic treatment for a patient suffering from pneumonia. This comprises determining whether the patient is suffering from *Mycoplasma pneumoniae* pneumonia by following the method of the invention, wherein, if the patient is suffering from *Mycoplasma pneumoniae* pneumonia a treatment with a macrolide antibiotic may be recommended and a treatment with beta-lactam antibiotic might be initially discarded, and/or wherein if the patient is not suffering from *Mycoplasma pneumoniae* pneumonia other clinical decisions could be considered.

### STATE OF THE ART

*Mycoplasma pneumoniae* commonly causes upper respiratory infections in infants and young adults, presenting with clinical manifestations ranging from asymptomatic to pneumonia. *M*. *pneumoniae* is one of the most common causes of atypical pneumonia. The incidence of *M. pneumoniae* pneumonia is uncertain and is typically described as endemic, marked by cyclic epidemics occurring every 3 to 5 years. The COVID-19 pandemic, along with associated non-pharmaceutical interventions, had a significant impact on the circulation of respiratory pathogens, including *M. pneumoniae.* A global surveillance network noted a decrease in *M. pneumoniae* detection from April 2020 to March 2021. Despite a resurgence in other pathogens, *M. pneumoniae* infections remained low until March 2022-23. Concerns about a potential upsurge in mycoplasma infections have arisen due to waning herd immunity. Recent outbreaks of *M. pneumoniae* infections have been reported during the last months in different European countries, including Spain, Denmark, France and the Netherlands. In November 2023, the World Health Organization (WHO) reported an increase in outpatient consultations and hospitalizations for pneumonia in China since May, along with rises in RSV, adenovirus, and influenza virus *M. pneumoniae* cases since October.

Establishing the microbiological ethology of pneumonia remains a challenge for clinicians within healthcare facilities, primarily due to the complexity of obtaining direct microbiological samples that require invasive methods. Additionally, there is a delay in obtaining results from other indirect tests such as pneumococcal antigen in urine, PCR in respiratory swabs, or serology if available. In the case of *M. pneumoniae,* cultures are usually the gold-standard for bacterial diagnosis; however, due to the difficulty of obtaining a representative sample, the slow growth, and the low sensitivity, they are not useful for clinical and treatment decisions. Serology for *M. pneumoniae* is another common approach, but tests show low specificity, and IgM can remain positive for months after the infection, with frequent cross-reactivity with other pathogens. This has led to its gradual replacement by PCR. Specific diagnosis of *M. pneumoniae* is important since, due to its lack of cellular wall, it does not respond to beta-lactams such as amoxicillin, which is the first-line treatment for typical community acquired pneumonia CAP. Azithromycin is the first line of treatment instead. Thus, clinicians usually start with empirical antibiotic treatment and adjust it later based on microbiological test results.

So, there is an unmet medical need of finding reliable tools aimed at diagnosing *Mycoplasma pneumoniae* infection, giving the possibility of differentiating it from other bacterial or viral pneumonias, and of assessing whether a patient suffering from pneumonia should be treated with a beta-lactam antibiotic, discarding a treatment with beta-lactam antibiotic when it is confirmed that the patient has a *Mycoplasma pneumoniae* pneumonia.

The present invention is focused on solving this problem by exploring host gene expression mechanisms specifically triggered by *M. pneumoniae* and evaluating them in the context of pneumonia molecular patterns generated by other pathogens, thus giving rise to a host-transcriptome signature specific for *M. pneumoniae* infection that may be used to overcome the limitations of current clinical and non-clinical procedures.

### DESCRIPTION OF THE INVENTION

### Brief description of the invention

The present invention refers to an *in vitro* method for the diagnosis of a *Mycoplasma pneumoniae* infection and/or for the differential diagnosis between a *Mycoplasma pneumoniae* pneumonia and other bacterial or viral pneumonias and/or for selecting the antibiotic treatment for a patient suffering from pneumonia.

Particularly, the inventors of the present invention generated predictive signatures specific for *M. pneumoniae* using a lasso regression simulation approach and blood microarray data from 107 pneumonia children (including 30 *M. pneumoniae*)*.* 8 different signatures, ranging from 3 to 10 transcripts, were identified (see **Table 1**) that differentiate mycoplasma pneumonia from other bacterial and viral pneumonias with high accuracy (AUC: 0.84-0.95).

**Table 1. Model coefficients and weights (%) for the genes included in each of the signatures; with the genes (DIO3, GALM, and TXNDC 11) appearing in at least five transcript signatures bolded. n: number of transcripts in the optimal signature.**

| **Gene combinations** | **Gene Signatures (coefficients; weight in %)** |
|---|---|
| **3 genes** | *FKBP11* (1.246; 32.8%), ***TXNDC11*** (1.380; 36.3%), ***DIO3*** (-1.172, 30.9%). Intercept: -16.662 |
| **4 genes** | *GAS6* (0.387,11.76%), *HYOU1* (1.354, 41.13%), *CAV1* (0.623, 18.93%), ***DIO3*** (-0.928, 28.19%). Intercept: -10.676 |
| **5 genes** | *DNAI7* (-0.595; 13.54%), ***GALM*** (0.714; 16.25%), *GINS4* (0.419; 9.54%), ***TXNDC11*** (2.038; 46.39%), ***DIO3*** (-0.627; 14.27%). Intercept: -13.747 |
| **6 genes** | *PRR11* (0.905; 29.94%), *DNAI7* (-0.298; 9.87%), ***GALM*** (0.793; 26.25%), *GINS4* (0.260; 8.61%), ***TXNDC11*** (0.491; 16.26%), *CAV1* (0.274; 9.06%). Intercept: -14.197 |
| **7 genes** | *CCL23* (0.624; 14.16%), ***GALM*** (0.910; 20.65%), ***TXNDC11*** (1.400; 31.77%), *ELL2* (-0.009; 0.20%), ***DIO3*** (-0.839; 19.04%), *UAP1* (-0.211; 4.79%), *CDCA2* (0.414; 9.39%). Intercept: -13.914 |
| **8 genes** | *CCL23* (0.545; 10.06%), *PRR11* (0.974; 17.99%), *GAS6* (0.016; 0.29%), ***GALM*** (1.012; 18.69%), ***TXNDC11*** (0.980; 18.10%), *CAV1* (0.001; 0.02%), ***DIO3*** (-1.196; 22.09%), *CADM1* (0.691; 12.76%). Intercept: -0.187 |
| **9 genes** | *CCL23* (0.396; 8.25%), *PRR11* (0.926; 19.30%), *DNAI7* (-0.412; 8.59%), ***GALM*** (0.319; 6.65%), *GINS4* (0.315; 6.56%), ***TXNDC11*** (0.053; 1.10%), *CAV1*(0.952; 19.84%), ***DIO3*** (-1.163; 24.24%), *PTGDR2* (0.262; 5.46%). Intercept: -10.163 |
| **10 genes** | *CCL23* (0.374; 2.52%), *PRR11*(1.316; 8.86%), *DNAI7* (-1.146; 7.72%), ***GALM*** (2.091; 14.08%), *GINS4* (1.136; 7-65%), ***TXNDC11*** (2.901; 19.54%), *MAPRE3* (-0.823; 5.54%), ***DIO3*** (-3.411; 22.97%), *GPR107* (-0.876; 5.90%), *CADM1* (0.773; 5.21%). Intercept: -28.067 |

Additionally, the present invention demonstrates that existing signatures for broadly distinguishing viral/bacterial infections and viral/bacterial pneumonias were ineffective in distinguishing pneumonia caused by mycoplasma. The new mycoplasma signatures were successfully validated in an independent cohort of children with pneumonia, demonstrating their robustness. The present invention indicates that *M*. *pneumoniae* infection induces specific transcriptomic alterations, enabling the development of robust diagnostic signatures. These signatures offer promising potential for enhancing the diagnosis and management of *M. pneumoniae* pneumonia, particularly with the possibility of integration into point-of-care diagnostic tools, thereby improving treatment outcomes.

On the other hand, kindly note that the eight signatures of **Table 1** were considered as proof-of concept and that the present invention offers reliable data showing that the individual use of any of the following genes PRR11, FKBP11, TXNDC11, DIO3, GAS6, HYOU1, CAV1, GINS4, GALM, DNAI7, CCL23, ELL2, UAP1, CDCA2, PTGDR2, CADM1, GPR107 and/or MAPRE3, or any combination thereof comprising between 2 and 18 genes, can be used in the context of the present invention for the diagnosis of a *Mycoplasma pneumoniae* infection and/or for the differential diagnosis between *a Mycoplasma pneumoniae* pneumonia and other bacterial or viral pneumonias and/or for selecting the antibiotic treatment for a patient suffering from pneumonia. In this regard, please refer to **Table 2** below wherein the AUC value of each of the genes PRR11, FKBP11, TXNDC11, DIO3, GAS6, HYOU1, CAV1, GINS4, DNAI7, CCL23, ELL2, UAP1, CDCA2, GALM, PTGDR2, CADM1, GPR107 and/or MAPRE3; and several combinations between 2 and 10 genes are provided as proof of concept.

The "special technical feature" conferring unity of invention is precisely the use of host gene expression mechanisms specifically triggered by *M. pneumoniae,* particularly for the diagnosis of a *Mycoplasma pneumoniae* infection and/or for the differential diagnosis between a *Mycoplasma pneumoniae* pneumonia and other bacterial or viral pneumonias and/or for selecting the antibiotic treatment for a patient suffering from pneumonia. Since these genes have not been used before for this specific purpose, the use of alternative genes fulfils the requirements of unity of invention.

| **TABLE 2** | | |
|---|---|---|
| **Individual biomarkers** | | |
| FKBP11 | 0.8009 | 0.7043-0.8974 |
| TXNDC11 | 0.7983 | 0.6923-0.9042 |
| DIO3 | 0.731 | 0.6255-0.8364 |
| GAS6 | 0.7823 | 0.6854-0.8791 |
| HYOU1 | 0.8117 | 0.727-0.8964 |
| CAV1 | 0.7835 | 0.6763-0.8908 |
| GINS4 | 0.7909 | 0.6913-0.8758 |
| GALM | 0.7896 | 0.6935-0.8667 |
| DNAI7 | 0.7141 | 0.6019-0.8118 |
| PRR11 | 0.8139 | 0.7259-0.8896 |
| CCL23 | 0.7078 | 0.5853-0.8253 |
| ELL2 | 0.7714 | 0.6627-0.8749 |
| UAP1 | 0.7935 | 0.6996-0.8879 |
| CDCA2 | 0.7861 | 0.6952-0.8675 |
| PTGDR2 | 0.7143 | 0.6052-0.816 |
| CADM1 | 0.7905 | 0.6844-0.8766 |
| GPR107 | 0.6974 | 0.5885-0.8065 |
| MAPRE3 | 0.7411 | 0.6408-0.8275 |

| **Combinations of two biomarkers** | | |
|---|---|---|
| FKBP11_TXNDC11 | 0.8113 | 0.7121-0.9104 |
| FKBP11_DIO3 | 0.8203 | 0.7279-0.9127 |
| TXNDC11_DIO3 | 0.8199 | 0.7209-0.919 |
| GAS6_HYOU1 | 0.8286 | 0.7497-0.9075 |
| GAS6_CAV1 | 0.8095 | 0.7162-0.9029 |
| GAS6_DIO3 | 0.8095 | 0.7233-0.8958 |
| HYOU1_CAV1 | 0.8182 | 0.7287-0.9076 |
| HYOU1_DIO3 | 0.8476 | 0.7685-0.9267 |
| CAV1_DIO3 | 0.8152 | 0.7247-0.9056 |
| TXNDC11_DIO3 | 0.8403 | 0.7415-0.929 |
| GINS4_DIO3 | 0.8355 | 0.7537-0.9039 |
| GALM_DIO3 | 0.8372 | 0.7597-0.9104 |
| DNAI7_DIO3 | 0.7818 | 0.6852-0.868 |
| GINS4_TXNDC11 | 0.8242 | 0.7216-0.9095 |
| GALM_TXNDC11 | 0.858 | 0.7782-0.9307 |
| DNAI7_TXNDC11 | 0.8303 | 0.7402-0.9165 |
| GALM_GINS4 | 0.8333 | 0.7485-0.9122 |
| DNAI7_GINS4 | 0.7946 | 0.6978-0.8853 |
| DNAI7_GALM | 0.7827 | 0.6848-0.8632 |
| GALM_GINS4 | 0.8212 | 0.7403-0.8983 |
| DNAI7_GINS4 | 0.7981 | 0.7035-0.8829 |
| PRR11_GINS4 | 0.8468 | 0.7666-0.9139 |
| DNAI7_GALM | 0.7974 | 0.713-0.8753 |
| PRR11_GALM | 0.8524 | 0.7753-0.9208 |
| PRR11_DNAI7 | 0.8545 | 0.777-0.9217 |
| TXNDC11_CAV1 | 0.8074 | 0.7052-0.9044 |
| GINS4_CAV1 | 0.8342 | 0.7454-0.9087 |
| DNAI7_CAV1 | 0.8342 | 0.7549-0.913 |
| PRR11_CAV1 | 0.8593 | 0.7814-0.926 |
| GALM_CAV1 | 0.8403 | 0.7632-0.9069 |
| GINS4_TXNDC11 | 0.8329 | 0.735-0.9065 |
| GALM_TXNDC11 | 0.8511 | 0.7809-0.9143 |
| DNAI7_TXNDC11 | 0.8216 | 0.7177-0.9095 |
| PRR11_TXNDC11 | 0.8628 | 0.7883-0.9225 |
| UAP1_CDCA2 | 0.7056 | 0.6008-0.8 |
| DIO3_CDCA2 | 0.8381 | 0.7506-0.9221 |
| ELL2_CDCA2 | 0.7848 | 0.6895-0.8719 |
| TXNDC11_CDCA2 | 0.8268 | 0.7311-0.9087 |
| PRR11_CDCA2 | 0.8463 | 0.7718-0.913 |
| CCL23_CDCA2 | 0.8017 | 0.697-0.8918 |
| DIO3_UAP1 | 0.661 | 0.5571-0.7741 |
| ELL2_UAP1 | 0.7952 | 0.6892-0.8905 |
| TXNDC11_UAP1 | 0.7701 | 0.6593-0.8662 |
| PRR11_UAP1 | 0.729 | 0.6195-0.8204 |
| CCL23_UAP1 | 0.6615 | 0.5251-0.7827 |
| ELL2_DIO3 | 0.7251 | 0.6125-0.8234 |
| TXNDC11_DIO3 | 0.8277 | 0.7229-0.9278 |
| PRR11_DIO3 | 0.8368 | 0.7649-0.903 |
| CCL23_DIO3 | 0.7931 | 0.6895-0.8892 |
| TXNDC11_ELL2 | 0.7978 | 0.6926-0.8991 |
| PRR11_ELL2 | 0.7879 | 0.6926-0.8667 |
| CCL23_ELL2 | 0.7048 | 0.5731-0.8173 |
| PRR11_TXNDC11 | 0.8736 | 0.8052-0.9364 |
| CCL23_TXNDC11 | 0.8165 | 0.706-0.9044 |
| CCL23_PRR11 | 0.8385 | 0.7506-0.9104 |
| PRR11_GAS6 | 0.8165 | 0.7333-0.8922 |
| CCL23_GAS6 | 0.7143 | 0.5904-0.8195 |
| CCL23_PRR11 | 0.8264 | 0.7294-0.9078 |
| PRR11_GALM | 0.8424 | 0.7563-0.9065 |
| CCL23_GALM | 0.842 | 0.7593-0.916 |
| GAS6_GALM | 0.7918 | 0.7022-0.8719 |
| PRR11_TXNDC11 | 0.8645 | 0.7913-0.9294 |
| CCL23_TXNDC11 | 0.8069 | 0.7022-0.9035 |
| GAS6_TXNDC11 | 0.7996 | 0.6861-0.8978 |
| GALM_TXNDC11 | 0.8667 | 0.7983-0.9294 |
| PRR11_CAV1 | 0.8143 | 0.7285-0.8927 |
| CCL23_CAV1 | 0.7113 | 0.5856-0.8217 |
| GAS6_CAV1 | 0.7866 | 0.6909-0.8762 |
| GALM_CAV1 | 0.7896 | 0.6974-0.8706 |
| TXNDC11_CAV1 | 0.7983 | 0.684-0.8952 |
| PRR11_DIO3 | 0.8502 | 0.7644-0.9147 |
| CCL23_DIO3 | 0.7961 | 0.6939-0.884 |
| GAS6_DIO3 | 0.7351 | 0.6342-0.8303 |
| GALM_DIO3 | 0.8312 | 0.7463-0.9039 |
| TXNDC11_DIO3 | 0.8095 | 0.7004-0.9 |
| CAV1_DIO3 | 0.7338 | 0.6316-0.8346 |
| PRR11_CADM1 | 0.8489 | 0.768-0.9199 |
| CCL23_CADM1 | 0.8056 | 0.7013-0.9009 |
| GAS6_CADM1 | 0.7922 | 0.6965-0.8801 |
| GALM_CADM1 | 0.8481 | 0.7701-0.9139 |
| TXNDC11_CADM1 | 0.8251 | 0.7234-0.9173 |
| CAV1_CADM1 | 0.7913 | 0.6965-0.8797 |
| DIO3_CADM1 | 0.8251 | 0.739-0.9052 |
| DIO3_PTGDR2 | 0.7745 | 0.669-0.8756 |
| CAV1_PTGDR2 | 0.8091 | 0.7048-0.8979 |
| TXNDC11_PTGDR2 | 0.7368 | 0.6182-0.8355 |
| GINS4_PTGDR2 | 0.8113 | 0.7164-0.8996 |
| GALM_PTGDR2 | 0.8134 | 0.7242-0.8922 |
| DNAI7_PTGDR2 | 0.781 | 0.6757-0.8749 |
| PRR11_PTGDR2 | 0.8494 | 0.7705-0.9199 |
| CCL23_PTGDR2 | 0.7387 | 0.6207-0.8446 |
| CAV1_DIO3 | 0.8199 | 0.7251-0.9039 |
| TXNDC11_DIO3 | 0.7407 | 0.6251-0.8386 |
| GINS4_DIO3 | 0.8052 | 0.7212-0.8875 |
| GALM_DIO3 | 0.787 | 0.687-0.8741 |
| DNAI7_DIO3 | 0.7784 | 0.6813-0.8671 |
| PRR11_DIO3 | 0.8481 | 0.771-0.913 |
| CCL23_DIO3 | 0.79 | 0.6957-0.8741 |
| TXNDC11_CAV1 | 0.7857 | 0.6775-0.8862 |
| GINS4_CAV1 | 0.826 | 0.7346-0.9065 |
| GALM_CAV1 | 0.8411 | 0.745-0.9182 |
| DNAI7_CAV1 | 0.8463 | 0.7549-0.923 |
| PRR11_CAV1 | 0.8481 | 0.7684-0.9169 |
| CCL23_CAV1 | 0.8238 | 0.7268-0.9134 |
| GINS4_TXNDC11 | 0.8087 | 0.7008-0.8987 |
| GALM_TXNDC11 | 0.8152 | 0.7342-0.8927 |
| DNAI7_TXNDC11 | 0.7364 | 0.6247-0.8295 |
| PRR11_TXNDC11 | 0.826 | 0.745-0.8983 |
| CCL23_TXNDC11 | 0.7195 | 0.5952-0.8373 |
| GALM_GINS4 | 0.8364 | 0.7498-0.9078 |
| DNAI7_GINS4 | 0.7985 | 0.7013-0.881 |
| PRR11_GINS4 | 0.8502 | 0.7684-0.9247 |
| CCL23_GINS4 | 0.8056 | 0.6887-0.9026 |
| DNAI7_GALM | 0.7745 | 0.6723-0.8606 |
| PRR11_GALM | 0.8489 | 0.7758-0.9165 |
| CCL23_GALM | 0.8139 | 0.7182-0.8922 |
| PRR11_DNAI7 | 0.8502 | 0.7736-0.9169 |
| CCL23_DNAI7 | 0.8035 | 0.6991-0.897 |
| CCL23_PRR11 | 0.8424 | 0.7532-0.9195 |
| GPR107_CADM1 | 0.8013 | 0.7099-0.8914 |
| DIO3_CADM1 | 0.784 | 0.6801-0.8745 |
| MAPRE3_CADM1 | 0.8022 | 0.7125-0.8835 |
| TXNDC11_CADM1 | 0.8156 | 0.713-0.9061 |
| GINS4_CADM1 | 0.8294 | 0.7407-0.9048 |
| GALM_CADM1 | 0.8364 | 0.7589-0.9074 |
| DNAI7_CADM1 | 0.819 | 0.7272-0.8935 |
| PRR11_CADM1 | 0.8485 | 0.7714-0.9186 |
| CCL23_CADM1 | 0.8208 | 0.7225-0.9035 |
| DIO3_GPR107 | 0.7758 | 0.6757-0.8615 |
| MAPRE3_GPR107 | 0.7913 | 0.6857-0.8831 |
| TXNDC11_GPR107 | 0.8013 | 0.684-0.8953 |
| GINS4_GPR107 | 0.8266 | 0.74-0.9048 |
| GALM_GPR107 | 0.8294 | 0.748-0.903 |
| DNAI7_GPR107 | 0.7654 | 0.6547-0.8637 |
| PRR11_GPR107 | 0.8299 | 0.7402-0.9005 |
| CCL23_GPR107 | 0.7459 | 0.6307-0.8516 |
| MAPRE3_DIO3 | 0.7693 | 0.6667-0.8637 |
| TXNDC11_DIO3 | 0.8095 | 0.7125-0.8996 |
| GINS4_DIO3 | 0.8152 | 0.7273-0.8892 |
| GALM_DIO3 | 0.819 | 0.7294-0.9 |
| DNAI7_DIO3 | 0.7766 | 0.6706-0.8676 |
| PRR11_DIO3 | 0.816 | 0.7203-0.8901 |
| CCL23_DIO3 | 0.7554 | 0.6498-0.855 |
| TXNDC11_MAPRE3 | 0.8229 | 0.7307-0.9048 |
| GALM_MAPRE3 | 0.816 | 0.7325-0.8875 |
| CCL23_DNAI7_MAPRE3 | 0.8082 | 0.7203-0.8918 |
| DNAI7_MAPRE3 | 0.7727 | 0.6723-0.8572 |
| PRR11_MAPRE3 | 0.8398 | 0.7649-0.9078 |
| CCL23_MAPRE3 | 0.781 | 0.6757-0.8654 |
| GINS4_TXNDC11 | 0.8333 | 0.7403-0.9165 |
| GALM_TXNDC11 | 0.8745 | 0.8009-0.9381 |
| DNAI7_TXNDC11 | 0.8273 | 0.7359-0.9169 |
| PRR11_TXNDC11 | 0.842 | 0.7524-0.9191 |
| CCL23_TXNDC11 | 0.8156 | 0.7082-0.9104 |
| GALM_GINS4 | 0.8342 | 0.7545-0.9087 |
| DNAI7_GINS4 | 0.8019 | 0.7093-0.8855 |
| PRR11_GINS4 | 0.8459 | 0.7584-0.9147 |
| CCL23_GINS4 | 0.8359 | 0.7342-0.9199 |
| DNAI7_GALM | 0.7918 | 0.6991-0.8684 |
| PRR11_GALM | 0.8286 | 0.7394-0.8974 |
| CCL23_GALM | 0.8273 | 0.745-0.9 |
| PRR11_DNAI7 | 0.8316 | 0.7463-0.9052 |
| CCL23_DNAI7 | 0.7857 | 0.6857-0.8758 |
| CCL23_PRR11 | 0.8554 | 0.7714-0.9286 |

| **Combination of three biomarkers** | | |
|---|---|---|
| FKBP11_TXNDC11_DIO3 | 0.8437 | 0.7526-0.9349 |
| GAS6_HYOU1_CAV1 | 0.8303 | 0.7481-0.9125 |
| GAS6_HYOU1_DIO3 | 0.8684 | 0.7965-0.9403 |
| GAS6_CAV1_DIO3 | 0.8472 | 0.7656-0.9288 |
| HYOU1_CAV1_DIO3 | 0.861 | 0.7817-0.9404 |
| GINS4_TXNDC11_DIO3 | 0.8654 | 0.7697-0.9455 |
| GALM_TXNDC1_DIO3 | 0.8892 | 0.8078-0.9532 |
| DNAI7_TXNDC11_DIO3 | 0.8667 | 0.7723-0.9411 |
| GALM_GINS4_DIO3 | 0.8732 | 0.7982-0.9359 |
| DNAI7_GINS4_DIO3 | 0.8312 | 0.7468-0.9052 |
| DNAI7_GALM_DIO3 | 0.8307 | 0.7498-0.9017 |
| GALM_GINS4_TXNDC11 | 0.8727 | 0.7913-0.9364 |
| DNAI7_GINS4_TXNDC11 | 0.8429 | 0.7515-0.923 |
| DNAI7_GALM_TXNDC11 | 0.8649 | 0.7814-0.9351 |
| DNAI7_GALM_GINS4 | 0.8121 | 0.7212-0.8927 |
| GINS4_TXNDC11_CAV1 | 0.8407 | 0.7532-0.9182 |
| GALM_TXNDC11_CAV1 | 0.8779 | 0.8095-0.936 |
| DNAI7_TXNDC11_CAV1 | 0.8476 | 0.7593-0.9338 |
| PRR11_TXNDC11_CAV1 | 0.8667 | 0.7926-0.9303 |
| GALM_GINS4_CAV1 | 0.8693 | 0.8017-0.9338 |
| DNAI7_GINS4_CAV1 | 0.8554 | 0.7822-0.9212 |
| PRR11_GINS4_CAV1 | 0.8732 | 0.7926-0.9398 |
| DNAI7_GALM_CAV1 | 0.8394 | 0.7605-0.913 |
| PRR11_GALM_CAV1 | 0.8727 | 0.7952-0.9307 |
| PRR11_DNAI7_CAV1 | 0.8775 | 0.8108-0.9364 |
| GALM_GINS4_TXNDC11 | 0.8775 | 0.8069-0.9355 |
| DNAI7_GINS4_TXNDC11 | 0.8476 | 0.7628-0.9208 |
| PRR11_GINS4_TXNDC11 | 0.8784 | 0.8026-0.9386 |
| PRR11_DNAI7_GINS4 | 0.861 | 0.7857-0.9264 |
| PRR11_DNAI7_GALM | 0.8524 | 0.7697-0.9234 |
| DNAI7_GALM_GINS4 | 0.8199 | 0.732-0.8935 |
| DNAI7_GALM_TXNDC11 | 0.8433 | 0.7658-0.9083 |
| PRR11_GALM_TXNDC11 | 0.8887 | 0.8233-0.9476 |
| PRR11_DNAI7_TXNDC11 | 0.8835 | 0.8169-0.9455 |
| DIO3_UAP1_CDCA2 | 0.8152 | 0.7208-0.897 |
| ELL2_UAP1_CDCA2 | 0.7017 | 0.597-0.7953 |
| TXNDC11_UAP1_CDCA2 | 0.8156 | 0.7186-0.9035 |
| PRR11_UAP1_CDCA2 | 0.8281 | 0.7424-0.8974 |
| CCL23_UAP1_CDCA2 | 0.7771 | 0.6719-0.8771 |
| ELL2_DIO3_CDCA2 | 0.8364 | 0.7489-0.91 |
| TXNDC11_DIO3_CDCA2 | 0.8706 | 0.7814-0.9442 |
| PRR11_DIO3_CDCA2 | 0.8857 | 0.8229-0.9429 |
| CCL23_DIO3_CDCA2 | 0.8658 | 0.7753-0.9312 |
| TXNDC11_ELL2_CDCA2 | 0.8264 | 0.7346-0.91 |
| PRR11_ELL2_CDCA2 | 0.845 | 0.771-0.9152 |
| CCL23_ELL2_CDCA2 | 0.7987 | 0.6948-0.8944 |
| PRR11_TXNDC11_CDCA2 | 0.874 | 0.8056-0.9342 |
| CCL23_TXNDC11_CDCA2 | 0.8506 | 0.7545-0.9299 |
| CCL23_PRR11_CDCA2 | 0.8684 | 0.7974-0.9294 |
| ELL2_DIO3_UAP1 | 0.6584 | 0.5493-0.7702 |
| TXNDC11_DIO3_UAP1 | 0.8139 | 0.7182-0.9035 |
| PRR11_DIO3_UAP1 | 0.797 | 0.7099-0.8805 |
| CCL23_DIO3_UAP1 | 0.7554 | 0.6454-0.8481 |
| TXNDC11_ELL2_UAP1 | 0.7697 | 0.6528-0.8671 |
| PRR11_ELL2_UAP1 | 0.7268 | 0.6229-0.829 |
| CCL23_ELL2_UAP1 | 0.6584 | 0.5294-0.7779 |
| PRR11_TXNDC11_UAP1 | 0.8671 | 0.7922-0.9346 |
| CCL23_TXNDC11_UAP1 | 0.8013 | 0.6892-0.8974 |
| CCL23_PRR11_UAP1 | 0.8087 | 0.719-0.8935 |
| TXNDC11_ELL2_DIO3 | 0.8281 | 0.7099-0.9169 |
| PRR11_ELL2_DIO3 | 0.8364 | 0.7541-0.9039 |
| CCL23_ELL2_DIO3 | 0.7905 | 0.6874-0.8792 |
| PRR11_TXNDC11_DIO3 | 0.8948 | 0.8277-0.9463 |
| CCL23_TXNDC11_DIO3 | 0.8693 | 0.7706-0.9446 |
| CCL23_PRR11_DIO3 | 0.8658 | 0.7969-0.929 |
| PRR11_TXNDC11_ELL2 | 0.8732 | 0.803-0.9372 |
| CCL23_TXNDC11_ELL2 | 0.8165 | 0.7086-0.9095 |
| CCL23_PRR11_ELL2 | 0.8364 | 0.7558-0.9113 |
| CCL23_PRR11_TXNDC11 | 0.8835 | 0.8117-0.9459 |
| PRR11_GAS6_GALM | 0.845 | 0.7714-0.9169 |
| CCL23_GAS6_GALM | 0.842 | 0.7628-0.9156 |
| CCL23_PRR11_GALM | 0.8775 | 0.797-0.9372 |
| CCL23_PRR11_GAS6 | 0.8294 | 0.7398-0.9091 |
| PRR11_GAS6_TXNDC11 | 0.8641 | 0.7896-0.9234 |
| CCL23_GAS6_TXNDC11 | 0.8087 | 0.6991-0.9005 |
| CCL23_PRR11_TXNDC11 | 0.868 | 0.7931-0.932 |
| PRR11_GALM_TXNDC11 | 0.8952 | 0.829-0.9494 |
| CCL23_GALM_TXNDC11 | 0.8909 | 0.8186-0.9446 |
| GAS6_GALM_TXNDC11 | 0.8667 | 0.8013-0.9281 |
| PRR11_GAS6_CAV1 | 0.8169 | 0.7337-0.8888 |
| CCL23_GAS6_CAV1 | 0.7147 | 0.5935-0.829 |
| CCL23_PRR11_CAV1 | 0.8264 | 0.7342-0.9069 |
| PRR11_GALM_CAV1 | 0.842 | 0.7571-0.9108 |
| CCL23_GALM_CAV1 | 0.842 | 0.7623-0.9126 |
| GAS6_GALM_CAV1 | 0.7926 | 0.7034-0.8758 |
| PRR11_TXNDC11_CAV1 | 0.8645 | 0.7905-0.9264 |
| CCL23_TXNDC11_CAV1 | 0.8069 | 0.7-0.9035 |
| GAS6_TXNDC11_CAV1 | 0.7996 | 0.6957-0.8961 |
| GALM_TXNDC11_CAV1 | 0.8667 | 0.7952-0.9277 |
| PRR11_GAS6_DIO3 | 0.8502 | 0.7667-0.9221 |
| CCL23_GAS6_DIO3 | 0.797 | 0.6957-0.8827 |
| CCL23_PRR11_DIO3 | 0.8805 | 0.8113-0.9347 |
| PRR11_GALM_DIO3 | 0.8792 | 0.8064-0.9386 |
| CCL23_GALM_DIO3 | 0.8671 | 0.7883-0.9303 |
| GAS6_GALM_DIO3 | 0.8329 | 0.7489-0.9044 |
| PRR11_TXNDC11_DIO3 | 0.8805 | 0.803-0.9407 |
| CCL23_TXNDC11_DIO3 | 0.8571 | 0.7632-0.9338 |
| GAS6_TXNDC11_DIO3 | 0.81 | 0.7095-0.8996 |
| GALM_TXNDC11_DIO3 | 0.8762 | 0.8043-0.9355 |
| PRR11_CAV1_DIO3 | 0.8502 | 0.771-0.9191 |
| CCL23_CAV1_DIO3 | 0.7961 | 0.6978-0.8775 |
| GAS6_CAV1_DIO3 | 0.7351 | 0.628-0.8312 |
| GALM_CAV1_DIO3 | 0.8312 | 0.748-0.9022 |
| TXNDC11_CAV1_DIO3 | 0.8095 | 0.703-0.9017 |
| PRR11_GAS6_CADM1 | 0.8498 | 0.7727-0.9165 |
| CCL23_GAS6_CADM1 | 0.8082 | 0.706-0.9 |
| CCL23_PRR11_CADM1 | 0.8558 | 0.764-0.926 |
| CAV1_DIO3_CADM1 | 0.8251 | 0.7377-0.903 |
| TXNDC11_DIO3_CADM1 | 0.8576 | 0.7731-0.9325 |
| GALM_DIO3_CADM1 | 0.8788 | 0.8104-0.9329 |
| GAS6_DIO3_CADM1 | 0.8286 | 0.7394-0.9022 |
| PRR11_DIO3_CADM1 | 0.8853 | 0.8164-0.9398 |
| CCL23_DIO3_CADM1 | 0.8571 | 0.7636-0.9325 |
| TXNDC11_DIO3_CADM1 | 0.8576 | 0.7731-0.9325 |
| GAS6_CAV1_CADM1 | 0.7918 | 0.6892-0.881 |
| PRR11_CAV1_CADM1 | 0.8489 | 0.7671-0.9182 |
| CCL23_CAV1_CADM1 | 0.8056 | 0.703-0.9013 |
| GALM_TXNDC11_CADM1 | 0.8788 | 0.8134-0.9333 |
| GAS6_TXNDC11_CADM1 | 0.8251 | 0.7346-0.9078 |
| PRR11_TXNDC11_CADM1 | 0.8602 | 0.7796-0.9303 |
| CCL23_TXNDC11_CADM1 | 0.8381 | 0.7406-0.9234 |
| GAS6_GALM_CADM1 | 0.8481 | 0.7727-0.9152 |
| PRR11_GALM_CADM1 | 0.8758 | 0.8034-0.9394 |
| CCL23_GALM_CADM1 | 0.874 | 0.7961-0.9303 |
| TXNDC11_CAV1_CADM1 | 0.8251 | 0.7341-0.9117 |
| GALM_CAV1_CADM1 | 0.8481 | 0.7658-0.9148 |
| CAV1_DIO3_PTGDR2 | 0.8398 | 0.7476-0.9178 |
| TXNDC11_DIO3_PTGDR2 | 0.781 | 0.6899-0.8762 |
| GINS4-DIO3_PTGDR2 | 0.8307 | 0.7498-0.9091 |
| GALM_DIO3_PTGDR2 | 0.816 | 0.7251-0.8935 |
| DNAI7_DIO3_PTGDR2 | 0.8108 | 0.7073-0.8961 |
| PRR11_DIO3_PTGDR2 | 0.871 | 0.8008-0.9312 |
| CCL23_DIO3_PTGDR2 | 0.8052 | 0.7073-0.8927 |
| TXNDC11_CAV1_PTGDR2 | 0.81 | 0.7138-0.8966 |
| GINS4_CAV1_PTGDR2 | 0.8463 | 0.758-0.9212 |
| GALM_CAV1_PTGDR2 | 0.8576 | 0.7731-0.9268 |
| DNAI7_CAV1_PTGDR2 | 0.8524 | 0.7645-0.9303 |
| PRR11_CAV1_PTGDR2 | 0.8636 | 0.7874-0.9273 |
| GINS4_DIO3_PTGDR2 | 0.8307 | 0.7498-0.9091 |
| GALM_DIO3_PTGDR2 | 0.816 | 0.7251-0.8935 |
| DNAI7_DIO3_PTGDR2 | 0.8108 | 0.7073-0.8961 |
| PRR11_DIO3_PTGDR2 | 0.871 | 0.8008-0.9312 |
| CCL23_CAV1_PTGDR2 | 0.8225 | 0.7229-0.9052 |
| GINS4_TXNDC11_PTGDR2 | 0.8182 | 0.7238-0.9035 |
| GALM_TXNDC11_PTGDR2 | 0.8234 | 0.7328-0.9043 |
| DNAI7_TXNDC11_PTGDR2 | 0.7879 | 0.6753-0.8771 |
| PRR11_TXNDC11_PTGDR2 | 0.8563 | 0.7753-0.9199 |
| CCL23_TXNDC11_PTGDR2 | 0.742 | 0.626-0.8502 |
| GALM_GINS4_PTGDR2 | 0.8524 | 0.7775-0.9217 |
| DNAI7_GINS4_PTGDR2 | 0.8338 | 0.7437-0.9152 |
| PRR11_GINS4_PTGDR2 | 0.8723 | 0.797-0.9381 |
| CCL23_GINS4_PTGDR2 | 0.79 | 0.6848-0.8849 |
| DNAI7_GALM_PTGDR2 | 0.8342 | 0.7476-0.9108 |
| PRR11_GALM_PTGDR2 | 0.8719 | 0.7978-0.9346 |
| CCL23_GALM_PTGDR2 | 0.797 | 0.7008-0.8749 |
| PRR11_DNAI7_PTGDR2 | 0.8697 | 0.7957-0.9316 |
| CCL23_DNAI7_PTGDR2 | 0.7948 | 0.693-0.887 |
| CCL23_PRR11_PTGDR2 | 0.8234 | 0.7407-0.8944 |
| TXNDC11_CAV1_DIO3 | 0.8199 | 0.7164-0.9143 |
| GINS4_CAV1_DIO3 | 0.8537 | 0.7706-0.9256 |
| GALM_CAV1_DIO3 | 0.8524 | 0.7679-0.9238 |
| DNAI7_CAV1_DIO3 | 0.8545 | 0.7701-0.923 |
| PRR11_CAV1_DIO3 | 0.884 | 0.8125-0.9463 |
| CCL23_CAV1_DIO3 | 0.8619 | 0.7762-0.9347 |
| GINS4_TXNDC11_DIO3 | 0.8108 | 0.7199-0.8879 |
| GALM_TXNDC11_DIO3 | 0.7939 | 0.6943-0.8792 |
| DNAI7_TXNDC11_DIO3 | 0.7805 | 0.6814-0.8719 |
| PRR11_TXNDC11_DIO3 | 0.8515 | 0.7732-0.9212 |
| CCL23_TXNDC11_DIO3 | 0.7974 | 0.7065-0.8892 |
| GALM_GINS4_DIO3 | 0.8403 | 0.7554-0.91 |
| DNAI7_GINS4_DIO3 | 0.826 | 0.7407-0.9048 |
| PRR11_GINS4_DIO3 | 0.8792 | 0.8134-0.9372 |
| CCL23_GINS4_DIO3 | 0.8459 | 0.7649-0.9152 |
| DNAI7_GALM_DIO3 | 0.8065 | 0.7026-0.8892 |
| PRR11_GALM_DIO3 | 0.8675 | 0.7931-0.929 |
| CCL23_GALM_DIO3 | 0.8277 | 0.7441-0.9017 |
| PRR11_DNAI7_DIO3 | 0.8753 | 0.8013-0.9368 |
| CCL23_DNAI7_DIO3 | 0.8281 | 0.7285-0.9096 |
| CCL23_PRR11_DIO3 | 0.8805 | 0.8108-0.9346 |
| GINS4_TXNDC11_CAV1 | 0.8264 | 0.7394-0.9039 |
| GALM_TXNDC11_CAV1 | 0.8407 | 0.7506-0.9269 |
| DNAI7_TXNDC11_CAV1 | 0.8472 | 0.7519-0.9312 |
| PRR11_TXNDC11_CAV1 | 0.8502 | 0.7653-0.9191 |
| CCL23_TXNDC11_CAV1 | 0.8251 | 0.726-0.9147 |
| GALM_GINS4_CAV1 | 0.861 | 0.7805-0.9294 |
| DNAI7_GINS4_CAV1 | 0.8597 | 0.7779-0.9282 |
| PRR11_GINS4_CAV1 | 0.8675 | 0.7964-0.9342 |
| CCL23_GINS4_CAV1 | 0.8602 | 0.7671-0.9325 |
| DNAI7_GALM_CAV1 | 0.871 | 0.7913-0.9385 |
| PRR11_GALM_CAV1 | 0.8719 | 0.7991-0.9351 |
| CCL23_GALM_CAV1 | 0.8788 | 0.8069-0.9372 |
| PRR11_DNAI7_CAV1 | 0.8797 | 0.8009-0.9407 |
| CCL23_DNAI7_CAV1 | 0.8753 | 0.7835-0.9416 |
| CCL23_PRR11_CAV1 | 0.8866 | 0.8134-0.9433 |
| GALM_GINS4_TXNDC11 | 0.8481 | 0.7684-0.9156 |
| DNAI7_GINS4_TXNDC11 | 0.8074 | 0.7182-0.887 |
| PRR11_GINS4_TXNDC11 | 0.8567 | 0.7814-0.9243 |
| CCL23_GINS4_TXNDC11 | 0.81 | 0.7138-0.9048 |
| DNAI7_GALM_TXNDC11 | 0.7866 | 0.6848-0.8736 |
| PRR11_GALM_TXNDC11 | 0.8541 | 0.7814-0.9195 |
| CCL23_GALM_TXNDC11 | 0.8216 | 0.7337-0.9039 |
| PRR11_DNAI7_TXNDC11 | 0.8567 | 0.7792-0.9281 |
| CCL23_DNAI7_TXNDC11 | 0.8095 | 0.7017-0.897 |
| CCL23_PRR11_TXNDC11 | 0.8494 | 0.7658-0.9204 |
| DNAI7_GALM_GINS4 | 0.8095 | 0.722-0.8922 |
| PRR11_GALM_GINS4 | 0.8662 | 0.7974-0.9316 |
| CCL23_GALM_GINS4 | 0.8554 | 0.7774-0.9295 |
| PRR11_DNAI7_GINS4 | 0.8645 | 0.7908-0.9329 |
| CCL23_DNAI7_GINS4 | 0.8485 | 0.7476-0.9307 |
| CCL23_PRR11_GINS4 | 0.8732 | 0.7991-0.9403 |
| PRR11_DNAI7_GALM | 0.858 | 0.7857-0.9264 |
| CCL23_DNAI7_GALM | 0.845 | 0.7558-0.9199 |
| CCL23_PRR11_GALM | 0.8745 | 0.8013-0.9381 |
| CCL23_PRR11_DNAI7 | 0.8784 | 0.8069-0.9442 |
| DIO3_GPR107_CADM1 | 0.8195 | 0.7338-0.9004 |
| MAPRE3_GPR107_CADM1 | 0.8472 | 0.7584-0.92 |
| TXNDC11_GPR107_CADM1 | 0.8307 | 0.7299-0.9156 |
| GINS4_GPR107_CADM1 | 0.8468 | 0.7667-0.9173 |
| GALM_GPR107_CADM1 | 0.8628 | 0.7892-0.9234 |
| DNAI7_GPR107_CADM1 | 0.8329 | 0.7441-0.9082 |
| PRR11_GPR107_CADM1 | 0.8537 | 0.7692-0.9221 |
| CCL23_GPR107_CADM1 | 0.8143 | 0.7104-0.9082 |
| MAPRE3_DIO3_CADM1 | 0.8126 | 0.716-0.8935 |
| TXNDC11_DIO3_CADM1 | 0.8385 | 0.7519-0.9212 |
| GINS4_DIO3_CADM1 | 0.8433 | 0.7602-0.913 |
| GALM_DIO3_CADM1 | 0.8519 | 0.7766-0.9165 |
| DNAI7_DIO3_CADM1 | 0.8152 | 0.7272-0.8905 |
| PRR11_DIO3_CADM1 | 0.8446 | 0.7636-0.9126 |
| CCL23_DIO3_CADM1 | 0.8113 | 0.7182-0.8935 |
| TXNDC11_MAPRE3_CADM1 | 0.8385 | 0.7493-0.9182 |
| GINS4_MAPRE3_CADM1 | 0.8351 | 0.7485-0.9092 |
| GALM_MAPRE3_CADM1 | 0.8554 | 0.7753-0.923 |
| DNAI7_MAPRE3_CADM1 | 0.8212 | 0.7311-0.897 |
| MAPRE3_DIO3_CADM1 | 0.8126 | 0.716-0.8935 |
| PRR11_MAPRE3_CADM1 | 0.8532 | 0.7697-0.9229 |
| CCL23_MAPRE3_CADM1 | 0.8299 | 0.7315-0.9117 |
| GINS4_TXNDC11_CADM1 | 0.8433 | 0.7563-0.923 |
| GALM_TXNDC11_CADM1 | 0.881 | 0.8134-0.9407 |
| DNAI7_TXNDC11_CADM1 | 0.8498 | 0.7563-0.9294 |
| PRR11_TXNDC11_CADM1 | 0.8481 | 0.7601-0.9173 |
| CCL23_TXNDC11_CADM1 | 0.8342 | 0.7428-0.9195 |
| GALM_GINS4_CADM1 | 0.8645 | 0.7866-0.9199 |
| DNAI7_GINS4_CADM1 | 0.8429 | 0.7641-0.9191 |
| PRR11_GINS4_CADM1 | 0.8567 | 0.7848-0.9225 |
| CCL23_GINS4_CADM1 | 0.8563 | 0.7645-0.9277 |
| DNAI7_GALM_CADM1 | 0.8346 | 0.7511-0.9039 |
| PRR11_GALM_CADM1 | 0.8645 | 0.787-0.9269 |
| CCL23_GALM_CADM1 | 0.8615 | 0.7865-0.9273 |
| PRR11_DNAI7_CADM1 | 0.8649 | 0.7939-0.9295 |
| CCL23_DNAI7_CADM1 | 0.842 | 0.7515-0.9195 |
| CCL23_PRR11_CADM1 | 0.8593 | 0.7805-0.9294 |
| MAPRE3_DIO3_GPR107 | 0.8074 | 0.7073-0.8905 |
| TXNDC11_DIO3_GPR107 | 0.8333 | 0.7385-0.9104 |
| GINS4_DIO3_GPR107 | 0.8446 | 0.7687-0.9121 |
| GALM_DIO3_GPR107 | 0.839 | 0.7628-0.91 |
| DNAI7_DIO3_GPR107 | 0.81 | 0.7181-0.8827 |
| PRR11_DIO3_GPR107 | 0.8442 | 0.7658-0.9087 |
| CCL23_DIO3_GPR107 | 0.7965 | 0.7013-0.8818 |
| TXNDC11_MAPRE3_GPR107 | 0.8498 | 0.7528-0.9238 |
| GINS4_MAPRE3_GPR107 | 0.8472 | 0.7701-0.9139 |
| GALM_MAPRE3_GPR107 | 0.8528 | 0.7758-0.9195 |
| DNAI7_MAPRE3_GPR107 | 0.8134 | 0.7199-0.8935 |
| PRR11_MAPRE3_GPR107 | 0.8636 | 0.7883-0.9273 |
| CCL23_MAPRE3_GPR107 | 0.813 | 0.7051-0.9078 |
| GINS4_TXNDC11_GPR107 | 0.8502 | 0.7623-0.926 |
| GALM_TXNDC11_GPR107 | 0.8801 | 0.8112-0.939 |
| DNAI7_TXNDC11_GPR107 | 0.8368 | 0.7467-0.9212 |
| PRR11_TXNDC11_GPR107 | 0.8563 | 0.7783-0.9281 |
| CCL23_TXNDC11_GPR107 | 0.8255 | 0.7298-0.9165 |
| GALM_GINS4_GPR107 | 0.8602 | 0.7896-0.9273 |
| DNAI7_GINS4_GPR107 | 0.839 | 0.7571-0.9104 |
| PRR11_GINS4_GPR107 | 0.8619 | 0.7895-0.9264 |
| CCL23_GINS4_GPR107 | 0.8407 | 0.7614-0.9147 |
| DNAI7_GALM_GPR107 | 0.8212 | 0.7229-0.9 |
| PRR11_GALM_GPR107 | 0.8554 | 0.7835-0.926 |
| CCL23_GALM_GPR107 | 0.8494 | 0.7684-0.916 |
| PRR11_DNAI7_GPR107 | 0.8489 | 0.7675-0.9139 |
| CCL23_DNAI7_GPR107 | 0.8074 | 0.7086-0.8961 |
| CCL23_PRR11_GPR107 | 0.8403 | 0.7563-0.9122 |
| TXNDC11_MAPRE3_DIO3 | 0.8398 | 0.7446-0.9186 |
| GINS4_MAPRE3_DIO3 | 0.8364 | 0.7541-0.9108 |
| GALM_MAPRE3_DIO3 | 0.8364 | 0.7515-0.9078 |
| DNAI7_MAPRE3_DIO3 | 0.7991 | 0.6978-0.8853 |
| PRR11_MAPRE3_DIO3 | 0.8355 | 0.7498-0.9108 |
| CCL23_MAPRE3_DIO3 | 0.7957 | 0.6978-0.8901 |
| GINS4_TXNDC11_DIO3 | 0.8558 | 0.7714-0.9247 |
| GALM_TXNDC11_DIO3 | 0.8723 | 0.8065-0.9329 |
| PRR11_TXNDC11_DIO3 | 0.8584 | 0.7818-0.9286 |
| CCL23_TXNDC11_DIO3 | 0.8303 | 0.7264-0.9113 |
| GALM_GINS4_DIO3 | 0.8619 | 0.7905-0.9251 |
| DNAI7_GINS4_DIO3 | 0.8307 | 0.7441-0.9035 |
| PRR11_GINS4_DIO3 | 0.861 | 0.7827-0.9212 |
| CCL23_GINS4_DIO3 | 0.8372 | 0.7606-0.9121 |
| DNAI7_GALM_DIO3 | 0.8303 | 0.745-0.9022 |
| PRR11_GALM_DIO3 | 0.8541 | 0.7744-0.9178 |
| CCL23_GALM_DIO3 | 0.8394 | 0.758-0.9113 |
| PRR11_DNAI7_DIO3 | 0.8364 | 0.7489-0.9082 |
| CCL23_DNAI7_DIO3 | 0.7991 | 0.7035-0.8866 |
| CCL23_PRR11_DIO3 | 0.8377 | 0.7558-0.9061 |
| GINS4_TXNDC11_MAPRE3 | 0.8506 | 0.7701-0.9208 |
| GALM_TXNDC11_MAPRE3 | 0.8857 | 0.8264-0.9407 |
| DNAI7_TXNDC11_MAPRE3 | 0.8455 | 0.7632-0.9225 |
| PRR11_TXNDC11_MAPRE3 | 0.8658 | 0.7879-0.9312 |
| CCL23_TXNDC11_MAPRE3 | 0.8472 | 0.7571-0.9277 |
| DNAI7_GINS4_MAPRE3 | 0.8208 | 0.7346-0.9004 |
| PRR11_GINS4_MAPRE3 | 0.8537 | 0.7684-0.926 |
| GALM_MAPRE3_DIO3 | 0.8364 | 0.7515-0.9078 |
| DNAI7_MAPRE3_DIO3 | 0.7991 | 0.6978-0.8853 |
| PRR11_MAPRE3_DIO3 | 0.8355 | 0.7498-0.9108 |
| CCL23_MAPRE3_DIO3 | 0.7957 | 0.6978-0.8901 |
| GINS4_TXNDC11_DIO3 | 0.8558 | 0.7714-0.9247 |
| GALM_TXNDC11_DIO3 | 0.8723 | 0.8065-0.9329 |
| PRR11_TXNDC11_DIO3 | 0.8584 | 0.7818-0.9286 |
| CCL23_TXNDC11_DIO3 | 0.8303 | 0.7264-0.9113 |
| GALM_GINS4_DIO3 | 0.8619 | 0.7905-0.9251 |
| DNAI7_GINS4_DIO3 | 0.8307 | 0.7441-0.9035 |
| PRR11_GINS4_DIO3 | 0.861 | 0.7827-0.9212 |
| CCL23_GINS4_DIO3 | 0.8372 | 0.7606-0.9121 |
| DNAI7_GALM_DIO3 | 0.8303 | 0.745-0.9022 |
| PRR11_GALM_DIO3 | 0.8541 | 0.7744-0.9178 |
| CCL23_GALM_DIO3 | 0.8394 | 0.758-0.9113 |
| PRR11_DNAI7_DIO3 | 0.8364 | 0.7489-0.9082 |
| CCL23_DNAI7_DIO3 | 0.7991 | 0.7035-0.8866 |
| CCL23_PRR11_DIO3 | 0.8377 | 0.7558-0.9061 |
| GINS4_TXNDC11_MAPRE3 | 0.8506 | 0.7701-0.9208 |
| GALM_TXNDC11_MAPRE3 | 0.8857 | 0.8264-0.9407 |
| DNAI7_TXNDC11_MAPRE3 | 0.8455 | 0.7632-0.9225 |
| PRR11_TXNDC11_MAPRE3 | 0.8658 | 0.7879-0.9312 |
| CCL23_TXNDC11_MAPRE3 | 0.8472 | 0.7571-0.9277 |
| DNAI7_GINS4_MAPRE3 | 0.8208 | 0.7346-0.9004 |
| PRR11_GINS4_MAPRE3 | 0.8537 | 0.7684-0.926 |
| CCL23_PRR11_GALM | 0.8615 | 0.7891-0.9268 |
| CCL23_PRR11_DNAI7 | 0.8654 | 0.7909-0.9273 |
| GALM_GINS4_MAPRE3 | 0.8545 | 0.7818-0.9212 |

| **Combinations of four biomarkers** | | |
|---|---|---|
| GAS6_HYOU1_CAV1_DIO3 | 0.871 | 0.7976-0.9444 |
| GALM_GINS4_TXNDC11_DIO3 | 0.9022 | 0.838-0.958 |
| DNAI7_GINS4_TXNDC11_DIO3 | 0.8779 | 0.7857-0.9545 |
| DNAI7_GALM_TXNDC11_DIO3 | 0.8887 | 0.8147-0.9519 |
| DNAI7_GALM_GINS4_DIO3 | 0.8515 | 0.7727-0.9152 |
| DNAI7_GALM_GINS4_TXNDC11 | 0.8749 | 0.8013-0.9403 |
| GALM_GINS4_TXNDC11_CAV1 | 0.8887 | 0.8195-0.9463 |
| GALM_GINS4_TXNDC11_CAV1 | 0.8887 | 0.8195-0.9463 |
| DNAI7_GINS4_TXNDC11_CAV1 | 0.8597 | 0.771-0.9325 |
| PRR11_GINS4_CAV1_DIO3 | 0.8991 | 0.8364-0.9506 |
| PRR11_GINS4_TXNDC11_CAV1 | 0.8762 | 0.8051-0.9372 |
| DNAI7_GALM_TXNDC11_CAV1 | 0.8745 | 0.8004-0.9346 |
| PRR11_GALM_TXNDC11_CAV1 | 0.8913 | 0.8182-0.9485 |
| PRR11_DNAI7_TXNDC11_CAV1 | 0.8827 | 0.8117-0.9411 |
| DNAI7_GALM_GINS4_CAV1 | 0.8602 | 0.7856-0.9229 |
| PRR11_GALM_GINS4_CAV1 | 0.8844 | 0.8156-0.942 |
| PRR11_DNAI7_GINS4_CAV1 | 0.8887 | 0.8221-0.9476 |
| PRR11_DNAI7_GALM_CAV1 | 0.8758 | 0.8026-0.9346 |
| DNAI7_GALM_GINS4_TXNDC11 | 0.8645 | 0.7939-0.929 |
| PRR11_GALM_GINS4_TXNDC11 | 0.89 | 0.8203-0.945 |
| PRR11_DNAI7_GINS4_TXNDC11 | 0.8883 | 0.8151-0.9437 |
| PRR11_DNAI7_GALM_TXNDC11 | 0.8831 | 0.8151-0.9429 |
| PRR11_DNAI7_GALM_GINS4 | 0.8632 | 0.7879-0.929 |
| ELL2_DIO3_UAP1_CDCA2 | 0.813 | 0.7199-0.8926 |
| TXNDC11_DIO3_UAP1_CDCA2 | 0.8667 | 0.7771-0.9398 |
| PRR11_DIO3_UAP1_CDCA2 | 0.8727 | 0.7974-0.9321 |
| CCL23_DIO3_UAP1_CDCA2 | 0.8442 | 0.7532-0.9234 |
| TXNDC11_ELL2_UAP1_CDCA2 | 0.8152 | 0.7203-0.8978 |
| PRR11_ELL2_UAP1_CDCA2 | 0.8277 | 0.7459-0.9017 |
| CCL23_ELL2_UAP1_CDCA2 | 0.7749 | 0.6627-0.8723 |
| PRR11_TXNDC11_UAP1_CDCA2 | 0.8745 | 0.7983-0.9338 |
| CCL23_TXNDC11_UAP1_CDCA2 | 0.8346 | 0.7381-0.9182 |
| CCL23_PRR11_UAP1_CDCA2 | 0.8524 | 0.7753-0.9204 |
| TXNDC11_ELL2_DIO3_CDCA2 | 0.8706 | 0.7814-0.9433 |
| PRR11_ELL2_DIO3_CDCA2 | 0.8853 | 0.8069-0.9394 |
| CCL23_ELL2_DIO3_CDCA2 | 0.8654 | 0.7805-0.9373 |
| PRR11_TXNDC11_DIO3_CDCA2 | 0.9104 | 0.8554-0.9623 |
| CCL23_TXNDC11_DIO3_CDCA2 | 0.8931 | 0.8039-0.9593 |
| CCL23_PRR11_DIO3_CDCA2 | 0.9074 | 0.8537-0.955 |
| PRR11_TXNDC11_ELL2_CDCA2 | 0.8745 | 0.8008-0.9312 |
| CCL23_TXNDC11_ELL2_CDCA2 | 0.8502 | 0.7601-0.9273 |
| CCL23_PRR11_ELL2_CDCA2 | 0.868 | 0.7961-0.9342 |
| CCL23_PRR11_TXNDC11_CDCA2 | 0.8939 | 0.8268-0.9481 |
| TXNDC11_ELL2_DIO3_UAP1 | 0.8126 | 0.7022-0.9057 |
| PRR11_ELL2_DIO3_UAP1 | 0.7957 | 0.7056-0.8779 |
| CCL23_ELL2_DIO3_UAP1 | 0.7537 | 0.645-0.8442 |
| PRR11_TXNDC11_DIO3_UAP1 | 0.8857 | 0.8138-0.9424 |
| CCL23_TXNDC11_DIO3_UAP1 | 0.8515 | 0.7619-0.9281 |
| CCL23_PRR11_DIO3_UAP1 | 0.8433 | 0.7567-0.9126 |
| PRR11_TXNDC11_ELL2_UAP1 | 0.8658 | 0.793-0.9316 |
| CCL23_TXNDC11_ELL2_UAP1 | 0.7996 | 0.6857-0.8957 |
| CCL23_PRR11_ELL2_UAP1 | 0.8065 | 0.7116-0.8939 |
| CCL23_PRR11_TXNDC11_UAP1 | 0.8814 | 0.8043-0.9403 |
| PRR11_TXNDC11_ELL2_DIO3 | 0.8952 | 0.8242-0.9463 |
| CCL23_TXNDC11_ELL2_DIO3 | 0.8693 | 0.7762-0.9476 |
| CCL23_PRR11_ELL2_DIO3 | 0.8658 | 0.7887-0.9286 |
| CCL23_PRR11_TXNDC11_DIO3 | 0.9165 | 0.8511-0.9671 |
| CCL23_PRR11_TXNDC11_ELL2 | 0.8835 | 0.8069-0.9468 |
| CCL23_PRR11_GAS6_CADM1 | 0.8563 | 0.7753-0.9264 |
| CCL23_GAS6_GALM_TXNDC11 | 0.8913 | 0.8238-0.9468 |
| CCL23_PRR11_GALM_TXNDC11 | 0.9039 | 0.8463-0.9571 |
| CCL23_PRR11_GAS6_TXNDC11 | 0.8684 | 0.7922-0.9333 |
| CCL23_PRR11_GAS6_GALM | 0.8766 | 0.7991-0.939 |
| PRR11_GAS6_GALM_TXNDC11 | 0.8952 | 0.8255-0.9511 |
| PRR11_GAS6_GALM_CAV1 | 0.845 | 0.771-0.9152 |
| CCL23_GAS6_GALM_CAV1 | 0.8424 | 0.755-0.9087 |
| CCL23_PRR11_GALM_CAV1 | 0.8775 | 0.8039-0.936 |
| CCL23_PRR11_GAS6_CAV1 | 0.8294 | 0.7364-0.9069 |
| PRR11_GAS6_TXNDC11_CAV1 | 0.8641 | 0.7922-0.9255 |
| CCL23_GAS6_TXNDC11_CAV1 | 0.8082 | 0.6909-0.9083 |
| CCL23_PRR11_TXNDC11_CAV1 | 0.868 | 0.7905-0.9329 |
| PRR11_GALM_TXNDC11_CAV1 | 0.8952 | 0.8273-0.9463 |
| CCL23_GALM_TXNDC11_CAV1 | 0.8909 | 0.8207-0.9451 |
| GAS6_GALM_TXNDC11_CAV1 | 0.8667 | 0.7905-0.9303 |
| PRR11_GAS6_GALM_DIO3 | 0.881 | 0.8173-0.9364 |
| CCL23_GAS6_GALM_DIO3 | 0.8675 | 0.7939-0.9312 |
| CCL23_PRR11_GALM_DIO3 | 0.9074 | 0.8472-0.961 |
| CCL23_PRR11_GAS6_DIO3 | 0.881 | 0.8086-0.9351 |
| PRR11_GAS6_TXNDC11_DIO3 | 0.8823 | 0.8078-0.942 |
| CCL23_GAS6_TXNDC11_DIO3 | 0.8571 | 0.7679-0.9264 |
| CCL23_PRR11_TXNDC11_DIO3 | 0.9078 | 0.8385-0.9636 |
| PRR11_GALM_TXNDC11_DIO3 | 0.9143 | 0.8593-0.9619 |
| CCL23_GALM_TXNDC11_DIO3 | 0.9139 | 0.8524-0.9623 |
| GAS6_GALM_TXNDC11_DIO3 | 0.8775 | 0.8108-0.9368 |
| PRR11_GAS6_CAV1_DIO3 | 0.8502 | 0.7727-0.9169 |
| CCL23_GAS6_CAV1_DIO3 | 0.7974 | 0.6913-0.8875 |
| CCL23_PRR11_CAV1_DIO3 | 0.8805 | 0.813-0.9368 |
| PRR11_GALM_CAV1_DIO3 | 0.8792 | 0.8112-0.939 |
| CCL23_GALM_CAV1_DIO3 | 0.8671 | 0.7913-0.9273 |
| GAS6_GALM_CAV1_DIO3 | 0.8329 | 0.7515-0.9048 |
| PRR11_TXNDC11_CAV1_DIO3 | 0.8805 | 0.8069-0.942 |
| CCL23_TXNDC11_CAV1_DIO3 | 0.8571 | 0.7593-0.9338 |
| GAS6_TXNDC11_CAV1_DIO3 | 0.81 | 0.7112-0.9004 |
| GALM_TXNDC11_CAV1_DIO3 | 0.8762 | 0.8017-0.9312 |
| CCL23_PRR11_GALM_CADM1 | 0.8861 | 0.8151-0.9416 |
| PRR11_GAS6_GALM_CADM1 | 0.8762 | 0.8073-0.9351 |
| CCL23_GAS6_GALM_CADM1 | 0.874 | 0.8113-0.9351 |
| CCL23_PRR11_TXNDC11_CADM1 | 0.8688 | 0.7823-0.9355 |
| PRR11_GAS6_TXNDC11_CADM1 | 0.8602 | 0.7835-0.9281 |
| CCL23_GAS6_TXNDC11_CADM1 | 0.8394 | 0.7329-0.9195 |
| PRR11_GALM_TXNDC11_CADM1 | 0.8909 | 0.8329-0.9494 |
| CCL23_GALM_TXNDC11_CADM1 | 0.89 | 0.8182-0.9485 |
| GAS6_GALM_TXNDC11_CADM1 | 0.8775 | 0.8082-0.9372 |
| CCL23_PRR11_CAV1_CADM1 | | |
| PRR11_GAS6_CAV1_CADM1 | | |
| CCL23_GAS6_CAV1_CADM1 | 0.8087 | 0.703-0.8979 |
| PRR11_GALM_CAV1_CADM1 | 0.8758 | 0.8008-0.9321 |
| CCL23_GALM_CAV1_CADM1 | 0.874 | 0.8013-0.9338 |
| GAS6_GALM_CAV1_CADM1 | 0.8485 | 0.7697-0.9156 |
| PRR11_TXNDC11_CAV1_CADM1 | 0.8602 | 0.7805-0.9191 |
| CCL23_TXNDC11_CAV1_CADM1 | 0.8385 | 0.7312-0.9212 |
| GAS6_TXNDC11_CAV1_CADM1 | 0.8251 | 0.7273-0.9108 |
| GALM_TXNDC11_CAV1_CADM1 | 0.8788 | 0.8091-0.9355 |
| CCL23_PRR11_DIO3_CADM1 | 0.9048 | 0.8398-0.9589 |
| PRR11_GAS6_DIO3_CADM1 | 0.8857 | 0.813-0.9455 |
| CCL23_GAS6_DIO3_CADM1 | 0.8584 | 0.7602-0.9312 |
| PRR11_GALM_DIO3_CADM1 | 0.9156 | 0.8532-0.9619 |
| CCL23_GALM_DIO3_CADM1 | 0.9056 | 0.839-0.9545 |
| GAS6_GALM_DIO3_CADM1 | 0.881 | 0.816-0.9364 |
| PRR11_TXNDC11_DIO3_CADM1 | 0.8965 | 0.8277-0.958 |
| CCL23_TXNDC11_DIO3_CADM1 | 0.884 | 0.7965-0.9541 |
| GAS6_TXNDC11_DIO3_CADM1 | 0.858 | 0.7649-0.936 |
| GALM_TXNDC11_DIO3_CADM1 | 0.9017 | 0.8381-0.9572 |
| PRR11_CAV1_DIO3_CADM1 | 0.8853 | 0.8186-0.9433 |
| CCL23_CAV1_DIO3_CADM1 | 0.8571 | 0.764-0.9308 |
| GAS6_CAV1_DIO3_CADM1 | 0.8286 | 0.7433-0.9 |
| GALM_CAV1_DIO3_CADM1 | 0.8792 | 0.8117-0.9359 |
| TXNDC11_CAV1_DIO3_CADM1 | 0.8571 | 0.7632-0.9294 |
| TXNDC11_CAV1_DIO3_PTGDR2 | 0.8411 | 0.7459-0.9225 |
| GINS4_CAV1_DIO3_PTGDR2 | 0.8775 | 0.7931-0.9398 |
| GALM_CAV1_DIO3_PTGDR2 | 0.8758 | 0.7991-0.9416 |
| DNAI7_CAV1_DIO3_PTGDR2 | 0.8801 | 0.8087-0.9476 |
| PRR11_CAV1_DIO3_PTGDR2 | 0.8987 | 0.8294-0.9502 |
| CCL23_CAV1_DIO3_PTGDR2 | 0.8671 | 0.7818-0.9407 |
| GINS4_TXNDC11_DIO3_PTGDR2 | 0.8351 | 0.7455-0.9121 |
| GALM_TXNDC11_DIO3_PTGDR2 | 0.8229 | 0.729-0.8992 |
| DNAI7_TXNDC11_DIO3_PTGDR2 | 0.8147 | 0.7104-0.8996 |
| PRR11_TXNDC11_DIO3_PTGDR2 | 0.8758 | 0.8065-0.9364 |
| CCL23_TXNDC11_DIO3_PTGDR2 | 0.8113 | 0.7113-0.9004 |
| GALM_GINS4_DIO3_PTGDR2 | 0.8619 | 0.7883-0.9251 |
| DNAI7_GINS4_DIO3_PTGDR2 | 0.8554 | 0.7736-0.926 |
| PRR11_GINS4 _DIO3_PTGDR2 | 0.8996 | 0.839-0.9481 |
| CCL23_GINS4_DIO3_PTGDR2 | 0.8489 | 0.7653-0.923 |
| DNAI7_GALM_DIO3_PTGDR2 | 0.839 | 0.7467-0.9156 |
| PRR11_GALM_DIO3_PTGDR2 | 0.8961 | 0.8346-0.9455 |
| CCL23_GALM_DIO3_PTGDR2 | 0.829 | 0.7333-0.9078 |
| PRR11_DNAI7_DIO3_PTGDR2 | 0.8944 | 0.8281-0.9489 |
| CCL23_DNAI7_DIO3_PTGDR2 | 0.8377 | 0.7484-0.9212 |
| CCL23_PRR11_DIO3_PTGDR2 | 0.8727 | 0.7939-0.9347 |
| GINS4_TXNDC11_CAV1_PTGDR2 | 0.8481 | 0.7645-0.9195 |
| GALM_TXNDC11_CAV1_PTGDR2 | 0.8576 | 0.771-0.9247 |
| DNAI7_TXNDC11_CAV1_PTGDR2 | 0.8532 | 0.7636-0.9277 |
| PRR11_TXNDC11_CAV1_PTGDR2 | 0.8645 | 0.79-0.9273 |
| CCL23_TXNDC11_CAV1_PTGDR2 | 0.8225 | 0.7264-0.9048 |
| GALM_GINS4_CAV1_PTGDR2 | 0.8723 | 0.7983-0.9342 |
| DNAI7_GINS4_CAV1_PTGDR2 | 0.8745 | 0.7978-0.9403 |
| PRR11_GINS4_CAV1_PTGDR2 | 0.8784 | 0.8147-0.9368 |
| CCL23_GINS4_CAV1_PTGDR2 | 0.8489 | 0.7623-0.9229 |
| DNAI7_GALM_CAV1_PTGDR2 | 0.8779 | 0.8082-0.9442 |
| PRR11_GALM_CAV1_PTGDR2 | 0.8771 | 0.8065-0.9355 |
| CCL23_GALM_CAV1_PTGDR2 | 0.8602 | 0.7814-0.9243 |
| PRR11_DNAI7_CAV1_PTGDR2 | 0.8805 | 0.8134-0.9455 |
| CCL23_DNAI7_CAV1_PTGDR2 | 0.8615 | 0.774-0.9329 |
| CCL23_PRR11_CAV1_PTGDR2 | 0.874 | 0.8009-0.9342 |
| GALM_GINS4_TXNDC11_PTGDR2 | 0.8571 | 0.7788-0.9208 |
| DNAI7_GINS4_TXNDC11_PTGDR2 | 0.8394 | 0.7454-0.9091 |
| PRR11_GINS4_TXNDC11_PTGDR2 | 0.8732 | 0.803-0.9403 |
| CCL23_GINS4_TXNDC11_PTGDR2 | 0.7944 | 0.6918-0.8862 |
| DNAI7_GALM_TXNDC11_PTGDR2 | 0.8368 | 0.7459-0.9117 |
| PRR11_GALM_TXNDC11_PTGDR2 | 0.8771 | 0.8082-0.9355 |
| CCL23_GALM_TXNDC11_PTGDR2 | 0.8017 | 0.7121-0.8883 |
| PRR11_DNAI7_TXNDC11_PTGDR2 | 0.8745 | 0.7991-0.9334 |
| CCL23_DNAI7_TXNDC11_PTGDR2 | 0.7991 | 0.7013-0.8991 |
| CCL23_PRR11_TXNDC11_PTGDR2 | 0.8277 | 0.7454-0.9035 |
| DNAI7_GALM_GINS4_PTGDR2 | 0.8545 | 0.7727-0.9234 |
| PRR11_GALM_GINS4_PTGDR2 | 0.8879 | 0.8216-0.9472 |
| CCL23_GALM_GINS4_PTGDR2 | 0.8351 | 0.7472-0.9074 |
| PRR11_DNAI7_GINS4_PTGDR2 | 0.8814 | 0.8125-0.9429 |
| CCL23_DNAI7_GINS4_PTGDR2 | 0.8247 | 0.7299-0.9148 |
| CCL23_PRR11_GINS4_PTGDR2 | 0.8532 | 0.7736-0.9234 |
| PRR11_DNAI7_GALM_PTGDR2 | 0.8758 | 0.8004-0.9346 |
| CCL23_DNAI7_GALM_PTGDR2 | 0.8281 | 0.7359-0.9052 |
| CCL23_PRR11_GALM_PTGDR2 | 0.8563 | 0.7731-0.9204 |
| CCL23_PRR11_DNAI7_PTGDR2 | 0.8619 | 0.787-0.9234 |
| GINS4_TXNDC11_CAV1_DIO3 | 0.8558 | 0.7775-0.9312 |
| GALM_TXNDC11_CAV1_DIO3 | 0.855 | 0.7679-0.9251 |
| DNAI7_TXNDC11_CAV1_DIO3 | 0.8571 | 0.7766-0.9299 |
| PRR11_TXNDC11_CAV1_DIO3 | 0.8835 | 0.8121-0.9446 |
| CCL23_TXNDC11_CAV1_DIO3 | 0.8615 | 0.7736-0.9355 |
| GALM_GINS4_CAV1_DIO3 | 0.8866 | 0.8164-0.942 |
| DNAI7_GINS4_CAV1_DIO3 | 0.8883 | 0.8138-0.9498 |
| CCL23_GINS4_CAV1_DIO3 | 0.8948 | 0.8182-0.9545 |
| DNAI7_GALM_CAV1_DIO3 | 0.8758 | 0.7991-0.9334 |
| PRR11_GALM_CAV1_DIO3 | 0.9017 | 0.8376-0.9541 |
| CCL23_GALM_CAV1_DIO3 | 0.9004 | 0.8389-0.9537 |
| PRR11_DNAI7_CAV1_DIO3 | 0.9048 | 0.8415-0.9533 |
| CCL23_DNAI7_CAV1_DIO3 | 0.897 | 0.8181-0.9545 |
| CCL23_PRR11_CAV1_DIO3 | 0.9143 | 0.8602-0.9628 |
| GALM_GINS4_TXNDC11_DIO3 | 0.8446 | 0.7654-0.9147 |
| DNAI7_GINS4_TXNDC11_DIO3 | 0.8303 | 0.7463-0.9052 |
| PRR11_GINS4_TXNDC11_DIO3 | 0.8818 | 0.8078-0.9381 |
| CCL23_GINS4_TXNDC11_DIO3 | 0.8515 | 0.7706-0.919 |
| DNAI7_GALM_TXNDC11_DIO3 | 0.8147 | 0.7229-0.8879 |
| PRR11_GALM_TXNDC11_DIO3 | 0.8701 | 0.8013-0.9338 |
| CCL23_GALM_TXNDC11_DIO3 | 0.8333 | 0.7433-0.9056 |
| PRR11_DNAI7_TXNDC11_DIO3 | 0.8784 | 0.8056-0.9424 |
| CCL23_DNAI7_TXNDC11_DIO3 | 0.8333 | 0.7433-0.9134 |
| CCL23_PRR11_TXNDC11_DIO3 | 0.8835 | 0.8125-0.9429 |
| DNAI7_GALM_GINS4_DIO3 | 0.8429 | 0.7602-0.9091 |
| PRR11_GALM_GINS4_DIO3 | 0.8944 | 0.8281-0.9494 |
| CCL23_GALM_GINS4_DIO3 | 0.8788 | 0.8099-0.9351 |
| PRR11_DNAI7_GINS4_DIO3 | 0.8944 | 0.8329-0.9468 |
| CCL23_DNAI7_GINS4_DIO3 | 0.8736 | 0.7987-0.9364 |
| CCL23_PRR11_GINS4_DIO3 | 0.9061 | 0.8472-0.9545 |
| PRR11_DNAI7_GALM_DIO3 | 0.887 | 0.8251-0.9429 |
| CCL23_DNAI7_GALM_DIO3 | 0.8537 | 0.7736-0.9199 |
| CCL23_PRR11_GALM_DIO3 | 0.9026 | 0.839-0.9515 |
| CCL23_PRR11_DNAI7_DIO3 | 0.9039 | 0.8429-0.9559 |
| GALM_GINS4_TXNDC11_CAV1 | 0.8619 | 0.7822-0.9312 |
| DNAI7_GINS4_TXNDC11_CAV1 | 0.8602 | 0.7727-0.929 |
| PRR11_GINS4_TXNDC11_CAV1 | 0.8667 | 0.7961-0.9264 |
| CCL23_GINS4_TXNDC11_CAV1 | 0.8597 | 0.7753-0.9307 |
| DNAI7_GALM_TXNDC11_CAV1 | 0.8701 | 0.7857-0.9411 |
| PRR11_GALM_TXNDC11_CAV1 | 0.871 | 0.7939-0.9316 |
| CCL23_GALM_TXNDC11_CAV1 | 0.8788 | 0.8064-0.9394 |
| PRR11_DNAI7_TXNDC11_CAV1 | 0.8797 | 0.803-0.9411 |
| CCL23_DNAI7 _TXNDC11_CAV1 | 0.8758 | 0.7991-0.9425 |
| CCL23_PRR11_TXNDC11_CAV1 | 0.887 | 0.8169-0.9463 |
| PRR11_GALM_GINS4_CAV1 | 0.8814 | 0.8156-0.9407 |
| CCL23_GALM_GINS4_CAV1 | 0.8939 | 0.8325-0.9476 |
| PRR11_DNAI7_GINS4_CAV1 | 0.8866 | 0.8138-0.9429 |
| CCL23_DNAI7_GINS4_CAV1 | 0.8948 | 0.8238-0.9485 |
| CCL23_PRR11_GINS4_CAV1 | 0.8926 | 0.8277-0.9468 |
| PRR11_DNAI7_GALM_CAV1 | 0.8896 | 0.8186-0.9489 |
| CCL23_DNAI7_GALM_CAV1 | 0.9043 | 0.8436-0.9576 |
| CCL23_PRR11_GALM_CAV1 | 0.9009 | 0.8372-0.9485 |
| CCL23_PRR11_DNAI7_CAV1 | 0.9078 | 0.845-0.9567 |
| DNAI7_GALM_GINS4_TXNDC11 | 0.819 | 0.7294-0.8961 |
| PRR11_GALM_GINS4_TXNDC11 | 0.871 | 0.7952-0.9303 |
| CCL23_GALM_GINS4_TXNDC11 | 0.8597 | 0.7848-0.9303 |
| PRR11_DNAI7_GINS4_TXNDC11 | 0.8654 | 0.7939-0.932 |
| CCL23_DNAI7_GINS4_TXNDC11 | 0.8502 | 0.7515-0.9308 |
| CCL23_PRR11_GINS4_TXNDC11 | 0.8727 | 0.7948-0.9398 |
| PRR11_DNAI7_GALM_TXNDC11 | 0.8619 | 0.7853-0.9199 |
| CCL23_DNAI7_GALM_TXNDC11 | 0.8472 | 0.7636-0.9169 |
| CCL23_PRR11_GALM_TXNDC11 | 0.8775 | 0.81-0.9368 |
| CCL23_PRR11_DNAI7_TXNDC11 | 0.8827 | 0.8082-0.9411 |
| PRR11_DNAI7_GALM_GINS4 | 0.8714 | 0.7948-0.9329 |
| CCL23_DNAI7_GALM_GINS4 | 0.8745 | 0.7983-0.9424 |
| CCL23_PRR11_GALM_GINS4 | 0.89 | 0.8186-0.9489 |
| CCL23_PRR11_DNAI7_GINS4 | 0.8883 | 0.8164-0.9519 |
| CCL23_PRR11_DNAI7_GALM | 0.8883 | 0.8216-0.9442 |
| MAPRE3_DIO3_GPR107_CADM1 | 0.8411 | 0.748-0.9156 |
| TXNDC11_DIO3_GPR107_CADM1 | 0.8554 | 0.7658-0.9334 |
| GINS4-DIO3_GPR107_CADM1 | 0.868 | 0.7892-0.9281 |
| GALM_DIO3_GPR107_CADM1 | 0.8667 | 0.7965-0.9247 |
| DNAI7_DIO3_GPR107_CADM1 | 0.8411 | 0.7606-0.9108 |
| PRR11_DIO3_GPR107_CADM1 | 0.8649 | 0.8009-0.9277 |
| CCL23_DIO3_GPR107_CADM1 | 0.8312 | 0.7467-0.9134 |
| XNDC11_MAPRE3_GPR107_CADM1 | 0.8502 | 0.7511-0.9242 |
| GINS4_MAPRE3_GPR107_CADM1 | 0.861 | 0.7818-0.9264 |
| GALM_MAPRE3_GPR107_CADM1 | 0.8688 | 0.7987-0.929 |
| DNAI7_MAPRE3_GPR107_CADM1 | 0.8468 | 0.7571-0.9169 |
| PRR11_MAPRE3_GPR107_CADM1 | 0.8714 | 0.7879-0.9342 |
| CCL23_MAPRE3_GPR107_CADM1 | 0.8476 | 0.7489-0.9273 |
| GINS4_TXNDC11_GPR107_CADM1 | 0.858 | 0.7632-0.9307 |
| GALM_TXNDC11_GPR107_CADM1 | 0.8853 | 0.8121-0.9416 |
| DNAI7_TXNDC11_GPR107_CADM1 | 0.8567 | 0.7675-0.9351 |
| PRR11_TXNDC11_GPR107_CADM1 | 0.8641 | 0.7861-0.9307 |
| CCL23_TXNDC11_GPR107_CADM1 | 0.8429 | 0.7389-0.9321 |
| GALM_GINS4_GPR107_CADM1 | 0.8805 | 0.8074-0.9359 |
| DNAI7_GINS4_GPR107_CADM1 | 0.8675 | 0.79-0.9295 |
| PRR11_GINS4_GPR107_CADM1 | 0.8714 | 0.8022-0.9294 |
| CCL23_GINS4_GPR107_CADM1 | 0.8628 | 0.777-0.9303 |
| DNAI7_GALM_GPR107_CADM1 | 0.8515 | 0.7706-0.919 |
| PRR11_GALM_GPR107_CADM1 | 0.8823 | 0.8103-0.9429 |
| CCL23_GALM_GPR107_CADM1 | 0.8749 | 0.8-0.9338 |
| PRR11_DNAI7_GPR107_CADM1 | 0.8701 | 0.7991-0.9303 |
| CCL23_DNAI7_GPR107_CADM1 | 0.8537 | 0.7606-0.9277 |
| CCL23_PRR11_GPR107_CADM1 | 0.8619 | 0.78-0.9303 |
| TXNDC11_MAPRE3_DIO3_CADM1 | 0.8649 | 0.7835-0.9377 |
| GINS4_MAPRE3_DIO3_CADM1 | 0.8567 | 0.777-0.9173 |
| GALM_MAPRE3_DIO3_CADM1 | 0.858 | 0.7878-0.9221 |
| DNAI7_MAPRE3_DIO3_CADM1 | 0.8342 | 0.7537-0.9052 |
| PRR11_MAPRE3_DIO3_CADM1 | 0.8628 | 0.7814-0.9234 |
| CCL23_MAPRE3_DIO3_CADM1 | 0.8281 | 0.7329-0.9061 |
| GINS4_TXNDC11_DIO3_CADM1 | 0.8732 | 0.7939-0.9381 |
| GALM_TXNDC11_DIO3_CADM1 | 0.8861 | 0.8207-0.939 |
| DNAI7_TXNDC11_DIO3_CADM1 | 0.8623 | 0.7853-0.9351 |
| PRR11_TXNDC11_DIO3_CADM1 | 0.8706 | 0.7922-0.9381 |
| CCL23_TXNDC11_DIO3_CADM1 | 0.8537 | 0.7697-0.9251 |
| DNAI7_GALM_GINS4_DIO3_CADM1 | 0.8818 | 0.8125-0.9372 |
| GALM_GINS4_DIO3_CADM1 | 0.8831 | 0.8203-0.9398 |
| DNAI7_GINS4_DIO3_CADM1 | 0.8571 | 0.7835-0.9238 |
| CCL23_PRR11 GINS4_DIO3_CADM1 | 0.8948 | 0.8264-0.9481 |
| PRR11_GINS4_DIO3_CADM1 | 0.8784 | 0.8103-0.9333 |
| CCL23_GINS4_DIO3_CADM1 | 0.8675 | 0.7883-0.9312 |
| DNAI7_GALM_DIO3_CADM1 | 0.8576 | 0.784-0.9191 |
| PRR11_GALM_DIO3_CADM1 | 0.8775 | 0.8017-0.9377 |
| CCL23_GALM_DIO3_CADM1 | 0.8667 | 0.7883-0.9277 |
| PRR11_DNAI7_DIO3_CADM1 | 0.8658 | 0.7948-0.9307 |
| CCL23_DNAI7_DIO3_CADM1 | 0.8446 | 0.7584-0.9143 |
| CCL23_PRR11_DIO3_CADM1 | 0.8662 | 0.7926-0.9286 |
| GINS4_TXNDC11_MAPRE3_CADM1 | 0.8571 | 0.7723-0.9273 |
| GALM_TXNDC11_MAPRE3_CADM1 | 0.8866 | 0.8225-0.9394 |
| DNAI7_TXNDC11_MAPRE3_CADM1 | 0.8563 | 0.7697-0.9329 |
| PRR11_TXNDC11_MAPRE3_CADM1 | 0.8619 | 0.7857-0.9312 |
| CCL23_TXNDC11_MAPRE3_CADM1 | 0.8463 | 0.7467-0.9251 |
| GALM_GINS4_MAPRE3_CADM1 | 0.874 | 0.8034-0.9321 |
| DNAI7_GINS4_MAPRE3_CADM1 | 0.8489 | 0.7632-0.9134 |
| PRR11_GINS4-MAPRE3_CADM1 | 0.8606 | 0.7792-0.9234 |
| CCL23_GINS4_MAPRE3_CADM1 | 0.8563 | 0.7667-0.926 |
| DNAI7_GALM_MAPRE3_CADM1 | 0.8437 | 0.7584-0.9108 |
| PRR11_GALM_MAPRE3_CADM1 | 0.8688 | 0.8009-0.9277 |
| CCL23_GALM_MAPRE3_CADM1 | 0.8736 | 0.8048-0.9304 |
| PRR11_DNAI7_MAPRE3_CADM1 | 0.8623 | 0.7861-0.9251 |
| CCL23_DNAI7_MAPRE3_CADM1 | 0.8385 | 0.7545-0.9121 |
| CCL23_PRR11_MAPRE3_CADM1 | 0.8675 | 0.7926-0.9312 |
| GALM_GINS4_TXNDC11_CADM1 | 0.8892 | 0.8216-0.9481 |
| DNAI7_GINS4_TXNDC11_CADM1 | 0.8623 | 0.7788-0.932 |
| PRR11_GINS4_TXNDC11_CADM1 | 0.8658 | 0.7922-0.9277 |
| CCL23_GINS4_TXNDC11_CADM1 | 0.8597 | 0.774-0.9312 |
| DNAI7_GALM_TXNDC11_CADM1 | 0.8775 | 0.8073-0.9377 |
| PRR11_GALM_TXNDC11_CADM1 | 0.8909 | 0.8268-0.9424 |
| CCL23_GALM_TXNDC11_CADM1 | 0.8926 | 0.8268-0.9442 |
| CCL23_PRR11_DNAI7_TXNDC11_CADM1 | 0.8779 | 0.8022-0.939 |
| PRR11_DNAI7_TXNDC11_CADM1 | 0.8662 | 0.7905-0.9381 |
| CCL23_DNAI7_TXNDC11_CADM1 | 0.8623 | 0.7762-0.9377 |
| CCL23_PRR11_TXNDC11_CADM1 | 0.8688 | 0.7861-0.9351 |
| DNAI7_GALM_GINS4_CADM1 | 0.8502 | 0.7732-0.9143 |
| PRR11_GALM_GINS4_CADM1 | 0.8823 | 0.8091-0.939 |
| CCL23_GALM_GINS4_CADM1 | 0.887 | 0.8225-0.9437 |
| PRR11_DNAI7_GINS4_CADM1 | 0.8706 | 0.7965-0.9355 |
| CCL23_DNAI7_GINS4_CADM1 | 0.8662 | 0.7931-0.9333 |
| CCL23_PRR11_GINS4_CADM1 | 0.8719 | 0.8008-0.9359 |
| PRR11_DNAI7_GALM_CADM1 | 0.8597 | 0.7814-0.9204 |
| CCL23_DNAI7_GALM_CADM1 | 0.8593 | 0.7883-0.916 |
| CCL23_PRR11_GALM_CADM1 | 0.8827 | 0.8147-0.9429 |
| CCL23_PRR11_DNAI7_CADM1 | 0.8814 | 0.8121-0.939 |
| TXNDC11_MAPRE3_DIO3_GPR107 | 0.8632 | 0.7775-0.9325 |
| GINS4_MAPRE3_DIO3_GPR107 | 0.8628 | 0.7844-0.9281 |
| GALM_MAPRE3_DIO3_GPR107 | 0.8567 | 0.7861-0.9243 |
| DNAI7_MAPRE3_DIO3_GPR107 | 0.8273 | 0.7385-0.903 |
| PRR11_MAPRE3_DIO3_GPR107 | 0.8602 | 0.7818-0.926 |
| CCL23_MAPRE3_DIO3_GPR107 | 0.8307 | 0.7428-0.91 |
| GINS4_TXNDC11_DIO3_GPR107 | 0.871 | 0.7909-0.9368 |
| GALM_TXNDC11_DIO3_GPR107 | 0.8848 | 0.819-0.9416 |
| DNAI7_TXNDC11_DIO3_GPR107 | 0.8641 | 0.7844-0.932 |
| PRR11_TXNDC11_DIO3_GPR107 | 0.8727 | 0.8043-0.9416 |
| CCL23_TXNDC11_DIO3_GPR107 | 0.8485 | 0.7623-0.9234 |
| GALM_GINS4_DIO3_GPR107 | 0.8814 | 0.806-0.9385 |
| DNAI7_GINS4_DIO3_GPR107 | 0.8563 | 0.7745-0.9195 |
| PRR11_GINS4_DIO3_GPR107 | 0.8792 | 0.8143-0.9351 |
| CCL23_GINS4_DIO3_GPR107 | 0.8576 | 0.7866-0.9221 |
| DNAI7_GALM_DIO3_GPR107 | 0.8502 | 0.7783-0.9165 |
| PRR11_GALM_DIO3_GPR10 | 0.8706 | 0.7987-0.9286 |
| CCL23_GALM_DIO3_GPR107 | 0.8593 | 0.7926-0.923 |
| PRR11_DNAI7_DIO3_GPR107 | 0.8554 | 0.7818-0.923 |
| CCL23_DNAI7_DIO3_GPR107 | 0.8294 | 0.7437-0.9005 |
| CCL23_PRR11_DIO3_GPR107 | 0.8563 | 0.7762-0.9238 |
| GINS4_TXNDC11_MAPRE3_GPR107 | 0.871 | 0.8-0.9364 |
| GALM_TXNDC11_MAPRE3_GPR107 | 0.9022 | 0.8398-0.9537 |
| DNAI7_TXNDC11_MAPRE3_GPR107 | 0.858 | 0.777-0.9307 |
| PRR11_TXNDC11_MAPRE3_GPR107 | 0.8844 | 0.8087-0.9459 |
| CCL23_TXNDC11_MAPRE3_GPR107 | 0.8645 | 0.7701-0.9442 |
| GALM_GINS4_MAPRE3_GPR107 | 0.8714 | 0.7991-0.9338 |
| DNAI7_GINS4_MAPRE3_GPR107 | 0.8537 | 0.7761-0.9217 |
| PRR11_GINS4_MAPRE3_GPR107 | 0.8766 | 0.8143-0.9338 |
| CCL23_GINS4_MAPRE3_GPR107 | 0.8706 | 0.8-0.9359 |
| DNAI7_GALM_MAPRE3_GPR107 | 0.839 | 0.761-0.913 |
| PRR11_GALM_MAPRE3_GPR107 | 0.8701 | 0.7991-0.9303 |
| CCL23_GALM_MAPRE3_GPR107 | 0.8727 | 0.8004-0.9338 |
| PRR11_DNAI7_MAPRE3_GPR107 | 0.8662 | 0.7892-0.9299 |
| CCL23_DNAI7_MAPRE3_GPR107 | 0.8394 | 0.7519-0.9156 |
| CCL23_PRR11_MAPRE3_GPR107 | 0.8684 | 0.797-0.9347 |
| GALM_GINS4_TXNDC11_GPR107 | 0.8961 | 0.832-0.9502 |
| DNAI7_GINS4_TXNDC11_GPR107 | 0.8567 | 0.7714-0.9316 |
| PRR11_GINS4_TXNDC11_GPR107 | 0.8805 | 0.8104-0.9437 |
| CCL23_GINS4_TXNDC11_GPR107 | 0.8641 | 0.7762-0.9329 |
| DNAI7_GALM_TXNDC11_GPR107 | 0.8771 | 0.8021-0.9329 |
| PRR11_GALM_TXNDC11_GPR107 | 0.8909 | 0.8238-0.9481 |
| CCL23_GALM_TXNDC11_GPR107 | 0.8987 | 0.835-0.9485 |
| PRR11_DNAI7_TXNDC11_GPR107 | 0.8714 | 0.7947-0.9372 |
| CCL23_DNAI7_TXNDC11_GPR107 | 0.8524 | 0.7554-0.9325 |
| CCL23_PRR11_TXNDC11_GPR107 | 0.8706 | 0.7926-0.9403 |
| DNAI7_GALM_GINS4_GPR107 | 0.8515 | 0.774-0.9199 |
| PRR11_GALM_GINS4_GPR107 | 0.8797 | 0.8112-0.9372 |
| CCL23_GALM_GINS4_GPR107 | 0.8749 | 0.8074-0.9351 |
| PRR11_DNAI7_GINS4_GPR107 | 0.8684 | 0.7904-0.9338 |
| CCL23_DNAI7_GINS4_GPR107 | 0.8632 | 0.7792-0.9368 |
| CCL23_PRR11_GINS4_GPR107 | 0.8758 | 0.8121-0.9307 |
| PRR11_DNAI7_GALM_GPR107 | 0.8506 | 0.7697-0.9152 |
| CCL23_DNAI7_GALM_GPR107 | 0.8463 | 0.7705-0.9186 |
| CCL23_PRR11_GALM_GPR107 | 0.8771 | 0.8048-0.939 |
| CCL23_PRR11_DNAI7_GPR107 | 0.8697 | 0.797-0.9346 |
| GINS4_TXNDC11_MAPRE3_DIO3 | 0.8784 | 0.8047-0.9416 |
| GALM_TXNDC11_MAPRE3_DIO3 | 0.8831 | 0.8151-0.9377 |
| DNAI7_TXNDC11_MAPRE3_DIO3 | 0.8615 | 0.7818-0.9303 |
| PRR11_TXNDC11_MAPRE3_DIO3 | 0.8775 | 0.7987-0.9416 |
| CCL23_TXNDC11_MAPRE3_DIO3 | 0.8615 | 0.7654-0.9342 |
| GALM_GINS4_MAPRE3_DIO3 | 0.8693 | 0.7961-0.9333 |
| DNAI7_GINS4_MAPRE3 _DIO3 | 0.8446 | 0.7706-0.9143 |
| PRR11_GINS4_MAPRE3_DIO3 | 0.8727 | 0.7982-0.9351 |
| CCL23_GINS4_MAPRE3_DIO3 | 0.8558 | 0.7723-0.9238 |
| DNAI7_GALM_MAPRE3_DIO3 | 0.8433 | 0.7615-0.9143 |
| PRR11_GALM_MAPRE3_DIO3 | 0.8671 | 0.7952-0.9273 |
| CCL23_GALM_MAPRE3_DIO3 | 0.8506 | 0.7766-0.9165 |
| PRR11_DNAI7_MAPRE3_DIO3 | 0.8472 | 0.7693-0.9169 |
| CCL23_DNAI7_MAPRE3_DIO3 | 0.8203 | 0.729-0.9004 |
| CCL23_PRR11_MAPRE3_DIO3 | 0.8584 | 0.7848-0.9217 |
| GALM_GINS4_TXNDC11_DIO3 | 0.8991 | 0.8337-0.9494 |
| PRR11_GINS4_TXNDC11_DIO3 | 0.887 | 0.8229-0.9433 |
| CCL23_GINS4_TXNDC11_DIO3 | 0.8766 | 0.8008-0.9394 |
| DNAI7_GALM_TXNDC11_DIO3 | 0.8844 | 0.8112-0.9403 |
| PRR11_GALM_TXNDC11_DIO3 | 0.8978 | 0.839-0.9502 |
| CCL23_GALM_TXNDC11_DIO3 | 0.8913 | 0.8259-0.945 |
| PRR11_DNAI7_TXNDC11_DIO3 | 0.8766 | 0.7974-0.9411 |
| CCL23_DNAI7_TXNDC11_DIO3 | 0.8619 | 0.7749-0.9316 |
| DNAI7_TXNDC11_DIO3 | 0.8403 | 0.7415-0.919 |
| CCL23_PRR11_TXNDC11_DIO3 | 0.8693 | 0.7835-0.939 |
| DNAI7_GALM_GINS4_DIO3 | 0.8615 | 0.7814-0.926 |
| PRR11_GALM_GINS4_DIO3 | 0.8848 | 0.8212-0.942 |
| CCL23_GALM_GINS4_DIO3 | 0.8831 | 0.813-0.9398 |
| PRR11_DNAI7_GINS4_DIO3 | 0.8762 | 0.8013-0.9377 |
| CCL23_PRR11_GINS4_DIO3 | 0.8805 | 0.8134-0.9377 |
| PRR11_DNAI7_GALM_DIO3 | 0.8641 | 0.7905-0.9277 |
| CCL23_DNAI7_GALM_DIO3 | 0.8502 | 0.7753-0.9178 |
| CCL23_PRR11_GALM_DIO3 | 0.8732 | 0.8026-0.932 |
| CCL23_PRR11_DNAI7_DIO3 | 0.8576 | 0.7775-0.9273 |
| GALM_GINS4_TXNDC11_MAPRE3 | 0.8961 | 0.8312-0.9476 |
| DNAI7_GINS4_TXNDC11_MAPRE3 | 0.8641 | 0.7844-0.9338 |
| PRR11_GINS4_TXNDC11_MAPRE3 | 0.8753 | 0.8013-0.9372 |
| CCL23_GINS4_TXNDC11_MAPRE3 | 0.8623 | 0.7762-0.932 |
| DNAI7_GALM_TXNDC11_MAPRE3 | 0.8801 | 0.8134-0.9329 |
| PRR11_GALM_TXNDC11_MAPRE3 | 0.9 | 0.8407-0.9511 |
| CCL23_GALM_TXNDC11_MAPRE3 | 0.9052 | 0.8415-0.955 |
| PRR11_DNAI7_TXNDC11_MAPRE3 | 0.8688 | 0.7948-0.9372 |
| CCL23_DNAI7_TXNDC11_MAPRE3 | 0.8641 | 0.7827-0.9351 |
| CCL23_PRR11_TXNDC11_MAPRE3 | 0.8753 | 0.7956-0.9364 |
| CCL23_GALM_GINS4_MAPRE3 | 0.8749 | 0.8056-0.936 |
| PRR11_DNAI7_GINS4_MAPRE3 | 0.8645 | 0.7861-0.9277 |
| CCL23_DNAI7_GINS4_MAPRE3 | 0.8532 | 0.7688-0.92 |
| CCL23_PRR11_GINS4_MAPRE3 | 0.8753 | 0.7961-0.9394 |
| PRR11_DNAI7_GALM_MAPRE3 | 0.8463 | 0.7679-0.9143 |
| CCL23_DNAI7_GALM_MAPRE3 | 0.842 | 0.7584-0.916 |
| CCL23_PRR11_GALM_MAPRE3 | 0.8745 | 0.8047-0.9364 |
| CCL23_PRR11_DNAI7_MAPRE3 | 0.8697 | 0.7939-0.9351 |
| DNAI7-GALM_GINS4_TXNDC11 | 0.8805 | 0.8069-0.9407 |
| PRR11_GALM_GINS4_TXNDC11 | 0.8987 | 0.8346-0.9481 |
| CCL23_GALM_GINS4_TXNDC11 | 0.8957 | 0.832-0.9515 |
| PRR11_DNAI7_GINS4_TXNDC11 | 0.8727 | 0.7974-0.9368 |
| CCL23_DNAI7_GINS4_TXNDC11 | 0.8632 | 0.7775-0.9398 |
| CCL23_PRR11_GINS4_TXNDC11 | 0.8775 | 0.8052-0.9433 |
| PRR11_DNAI7_GALM_TXNDC11 | 0.8874 | 0.8199-0.9424 |
| CCL23_DNAI7_GALM_TXNDC11 | 0.8814 | 0.8126-0.9381 |
| CCL23_PRR11_GALM_TXNDC11 | 0.9013 | 0.835-0.9506 |
| CCL23_PRR11_DNAI7_TXNDC11 | 0.8714 | 0.7818-0.939 |
| PRR11_DNAI7_GALM_GINS4 | 0.8519 | 0.774-0.9217 |
| CCL23_DNAI7_GALM_GINS4 | 0.8511 | 0.7701-0.9195 |
| CCL23_PRR11_GALM_GINS4 | 0.8797 | 0.8117-0.9416 |
| CCL23_PRR11_DNAI7_GINS4 | 0.8779 | 0.7957-0.9416 |
| CCL23_PRR11_DNAI7_GALM | 0.8589 | 0.774-0.9208 |
| DNAI7_GALM_GINS4_MAPRE3 | 0.839 | 0.761-0.907 |
| CCL23_DNAI7_GINS4_DIO3 | 0.8567 | 0.7719-0.9208 |
| DNAI7_GALM_GINS4_CAV1 | 0.8814 | 0.8134-0.9416 |

| **Combinations of five biomarkers** | | |
|---|---|---|
| DNAI7_GALM_GINS4_TXNDC11_DIO3 | 0.9022 | 0.8377-0.955 |
| CCL23_PRR11_DNAI7_TXNDC11_GPR107 | 0.8814 | 0.8017-0.9455 |
| CCL23_PRR11_GALM_TXNDC11_GPR107 | 0.9082 | 0.8502-0.9567 |
| CCL23_PRR11_DNAI7_GALM_GPR107 | 0.8784 | 0.8087-0.9347 |
| DNAI7_GALM_GINS4_TXNDC11_CAV1 | 0.887 | 0.8229-0.9459 |
| CCL23_GALM_GINS4_MAPRE3_DIO3 | 0.8848 | 0.819-0.9399 |
| CCL23_DNAI7_GINS4_TXNDC11_MAPRE3 | 0.8745 | 0.7905-0.9407 |
| PRR11_GALM_GINS4_TXNDC11_CAV1 | 0.8974 | 0.8376-0.9532 |
| PRR11_DNAI7_GINS4_TXNDC11_CAV1 | 0.8905 | 0.8307-0.9437 |
| PRR11_DNAI7_GALM_TXNDC11_CAV1 | 0.8957 | 0.8312-0.9537 |
| PRR11_DNAI7_GALM_GINS4_CAV1 | 0.8848 | 0.8156-0.9403 |
| PRR11_DNAI7_GINS4_TXNDC11_CAV1 | 0.8905 | 0.8307-0.9437 |
| PRR11_DNAI7_GALM_GINS4_TXNDC11 | 0.8905 | 0.8203-0.9455 |
| TXNDC11_ELL2_DIO3_UAP1_CDCA2 | 0.8662 | 0.777-0.9416 |
| PRR11_ELL2_DIO3_UAP1_CDCA2 | 0.8714 | 0.8013-0.9303 |
| CCL23_ELL2_DIO3_UAP1_CDCA2 | 0.8424 | 0.7446-0.9191 |
| PRR11_TXNDC11_DIO3_UAP1_CDCA2 | 0.9065 | 0.845-0.9571 |
| CCL23_TXNDC11_DIO3_UAP1_CDCA2 | 0.8926 | 0.8091-0.9585 |
| CCL23_PRR11_DIO3_UAP1_CDCA2 | 0.8939 | 0.8298-0.9468 |
| PRR11_TXNDC11_ELL2_UAP1_CDCA2 | 0.8749 | 0.8039-0.9355 |
| CCL23_TXNDC11_ELL2_UAP1_CDCA2 | 0.8342 | 0.7402-0.9204 |
| CCL23_PRR11_ELL2_UAP1_CDCA2 | 0.8515 | 0.7766-0.9208 |
| CCL23_PRR11_TXNDC11_UAP1_CDCA2 | 0.8948 | 0.826-0.9485 |
| PRR11_TXNDC11_ELL2_DIO3_CDCA2 | 0.91 | 0.8537-0.9615 |
| CCL23_TXNDC11_ELL2_DIO3_CDCA2 | 0.8931 | 0.8026-0.9576 |
| CCL23_PRR11_ELL2_DIO3_CDCA2 | 0.9061 | 0.8428-0.9572 |
| CCL23_PRR11_TXNDC11_DIO3_CDCA2 | 0.9247 | 0.8666-0.9732 |
| CCL23_PRR11_TXNDC11_ELL2 CDCA2 | 0.8939 | 0.8273-0.9537 |
| PRR11_TXNDC11_ELL2_DIO3_UAP1 | 0.8857 | 0.8238-0.9429 |
| CCL23_TXNDC11_ELL2_DIO3_UAP1 | 0.8511 | 0.7502-0.9295 |
| CCL23_PRR11_TXNDC11_DIO3_UAP1 | 0.9065 | 0.8455-0.958 |
| CCL23_PRR11_TXNDC11 ELL2 UAP1 | 0.8818 | 0.8081-0.9381 |
| CCL23_PRR11_TXNDC11_ELL2_DIO3 | 0.9156 | 0.858-0.9667 |
| CCL23_PRR11_ELL2_DIO3_UAP1 | 0.8429 | 0.7602-0.9048 |
| CCL23_PRR11_GAS6_GALM_DIO3 | 0.9078 | 0.8467-0.9628 |
| GAS6_GALM_TXNDC11_DIO3_CADM1 | 0.9026 | 0.8355-0.952 |
| PRR11_GALM_TXNDC11_DIO3_CADM1 | 0.9255 | 0.8662-0.9684 |
| CCL23_GALM_TXNDC11_DIO3_CADM1 | 0.9273 | 0.8667-0.9779 |
| PRR11_GAS6_TXNDC11_DIO3_CADM1 | 0.8965 | 0.832-0.9519 |
| CCL23_GAS6_TXNDC11_DIO3_CADM1 | 0.8835 | 0.8022-0.9498 |
| PRR11_GAS6_GALM_DIO3_CADM1 | 0.916 | 0.8593-0.9636 |
| CCL23_GAS6_GALM_DIO3_CADM1 | 0.9065 | 0.8411-0.9554 |
| CCL23_PRR11_GALM_DIO3_CADM1 | 0.9333 | 0.8796-0.9753 |
| CCL23_PRR11_GAS6_DIO3_CADM1 | 0.9052 | 0.8411-0.9593 |
| PRR11_GALM_TXNDC11_CAV1_CADM1 | 0.8909 | 0.8238-0.9485 |
| CCL23_GALM_TXNDC11_CAV1_CADM1 | 0.8905 | 0.8212-0.952 |
| PRR11_GAS6_TXNDC11_CAV1_CADM1 | 0.8602 | 0.7818-0.9277 |
| CCL23_GAS6_TXNDC11_CAV1_CADM1 | 0.8394 | 0.7359-0.9204 |
| CCL23_PRR11_TXNDC11_CAV1_CADM1 | 0.8688 | 0.7835-0.9407 |
| PRR11_GAS6_GALM_CAV1_CADM1 | 0.8762 | 0.8086-0.9381 |
| CCL23_GAS6_GALM_CAV1_CADM1 | 0.874 | 0.8017-0.9346 |
| CCL23_PRR11_GALM_CAV1_CADM1 | 0.8861 | 0.8138-0.9446 |
| PRR11_GAS6_GALM_TXNDC11_CADM1 | 0.89 | 0.8268-0.945 |
| CCL23_GAS6_GALM_TXNDC11_CADM1 | 0.8909 | 0.8117-0.952 |
| CCL23_PRR11_GALM_TXNDC11_CADM1 | 0.9026 | 0.8459-0.9541 |
| CCL23_PRR11_GAS6_TXNDC11_CADM1 | 0.8697 | 0.7835-0.9372 |
| CCL23_PRR11_GAS6_GALM_CADM1 | 0.8857 | 0.816-0.9403 |
| GAS6_GALM_TXNDC11_CAV1_DIO3 | 0.8775 | 0.81-0.9342 |
| PRR11_GALM_TXNDC11_CAV1_DIO3 | 0.9143 | 0.8532-0.9623 |
| CCL23_GALM_TXNDC11_CAV1_DIO3 | 0.9139 | 0.8593-0.9649 |
| PRR11_GAS6_TXNDC11_CAV1_DIO3 | 0.8823 | 0.81-0.9424 |
| CCL23_GAS6_TXNDC11_CAV1_DIO3 | 0.8571 | 0.761-0.9364 |
| CCL23_PRR11_TXNDC11_CAV1_DIO3 | 0.9078 | 0.8394-0.9615 |
| PRR11_GAS6_GALM_CAV1_DIO3 | 0.881 | 0.8086-0.9385 |
| CCL23_GAS6_GALM_CAV1_DIO3 | 0.868 | 0.7939-0.929 |
| CCL23_PRR11_GALM_CAV1_DIO3 | 0.9074 | 0.8463-0.9567 |
| CCL23_PRR11_GAS6_CAV1_DIO3 | 0.8814 | 0.8086-0.9416 |
| PRR11_GAS6_GALM_TXNDC11_DIO3 | 0.9147 | 0.8602-0.9623 |
| CCL23_GAS6_GALM_TXNDC11_DIO3 | 0.9134 | 0.8545-0.9593 |
| CCL23_PRR11_GALM_TXNDC11_DIO3 | 0.9351 | 0.884-0.9749 |
| CCL23_PRR11_GAS6_TXNDC11_DIO3 | 0.9087 | 0.8424-0.9632 |
| PRR11_GAS6_GALM_TXNDC11_CAV1 | 0.8952 | 0.8355-0.9502 |
| CCL23_GAS6_GALM_TXNDC11_CAV1 | 0.8918 | 0.8286-0.9494 |
| CCL23_PRR11_GALM_TXNDC11_CAV1 | 0.9039 | 0.8437-0.9559 |
| CCL23_PRR11_GAS6_TXNDC11_CAV1 | 0.8688 | 0.7943-0.9312 |
| CCL23_PRR11_GAS6_GALM_CAV1 | 0.8766 | 0.8082-0.9407 |
| CCL23_PRR11_GAS6_GALM_TXNDC11 | 0.9039 | 0.8489-0.9559 |
| GAS6_TXNDC11_CAV1_DIO3_CADM1 | 0.858 | 0.7631-0.9355 |
| PRR11_TXNDC11_CAV1_DIO3_CADM1 | 0.8965 | 0.8264-0.9511 |
| CCL23_TXNDC11_CAV1_DIO3_CADM1 | 0.884 | 0.7957-0.9546 |
| GAS6_GALM_CAV1_DIO3_CADM1 | 0.881 | 0.8082-0.9355 |
| PRR11_GALM_CAV1_DIO3_CADM1 | 0.9156 | 0.8589-0.9645 |
| CCL23_GALM_CAV1_DIO3_CADM1 | 0.9061 | 0.8402-0.9563 |
| PRR11_GAS6_CAV1_DIO3_CADM1 | 0.8857 | 0.8164-0.9433 |
| CCL23_GAS6_CAV1_DIO3_CADM1 | 0.8584 | 0.7648-0.9329 |
| CCL23_PRR11_CAV1_DIO3_CADM1 | 0.9048 | 0.8333-0.9589 |
| GALM_TXNDC11_CAV1_DIO3_CADM1 | 0.9017 | 0.8398-0.9528 |
| GAS6_GALM_TXNDC11_CAV1_CADM1 | 0.8775 | 0.809-0.9347 |
| CCL23_PRR11_GAS6_CAV1_CADM1 | 0.8563 | 0.7796-0.929 |
| GINS4_TXNDC11_CAV1_DIO3_PTGDR2 | 0.8762 | 0.7965-0.9416 |
| GALM_TXNDC11_CAV1_DIO3_PTGDR2 | 0.8758 | 0.8052-0.9368 |
| DNAI7_TXNDC11_CAV1_DIO3_PTGDR2 | 0.8801 | 0.8039-0.9476 |
| PRR11_TXNDC11_CAV1_DIO3_PTGDR2 | 0.8996 | 0.8303-0.9489 |
| CCL23_TXNDC11_CAV1_DIO3_PTGDR2 | 0.8675 | 0.7788-0.9429 |
| GALM_GINS4_CAV1_DIO3_PTGDR2 | 0.9004 | 0.8385-0.9533 |
| DNAI7_GINS4_CAV1_DIO3_PTGDR2 | 0.9004 | 0.8355-0.9559 |
| PRR11_GINS4_CAV1_DIO3_PTGDR2 | 0.9165 | 0.8566-0.9632 |
| CCL23_GINS4_CAV1_DIO3_PTGDR2 | 0.8926 | 0.8121-0.9585 |
| DNAI7_GALM_CAV1_DIO3_PTGDR2 | 0.8983 | 0.8312-0.9494 |
| PRR11_GALM_CAV1_DIO3_PTGDR2 | 0.9139 | 0.855-0.9602 |
| CCL23_GALM_CAV1_DIO3_PTGDR2 | 0.8944 | 0.8203-0.9537 |
| PRR11_DNAI7_CAV1_DIO3_PTGDR2 | 0.9186 | 0.8593-0.9623 |
| CCL23_DNAI7_CAV1_DIO3_PTGDR2 | 0.8926 | 0.8073-0.9563 |
| CCL23_PRR11_CAV1_DIO3_PTGDR2 | 0.9126 | 0.8515-0.9636 |
| GALM_GINS4_TXNDC11_DIO3_PTGDR2 | 0.8654 | 0.7909-0.9251 |
| DNAI7_GINS4_TXNDC11_DIO3_PTGDR2 | 0.8558 | 0.7662-0.9294 |
| PRR11_GINS4_TXNDC11_DIO3_PTGDR2 | 0.903 | 0.8441-0.9524 |
| CCL23_GINS4_TXNDC11_DIO3_PTGDR2 | 0.8524 | 0.7615-0.9221 |
| DNAI7_GALM_TXNDC11_DIO3_PTGDR2 | 0.8455 | 0.755-0.9169 |
| PRR11_GALM_TXNDC11_DIO3_PTGDR2 | 0.8957 | 0.8329-0.9506 |
| CCL23_GALM_TXNDC11_DIO3_PTGDR2 | 0.8342 | 0.7416-0.91 |
| PRR11_DNAI7_TXNDC11_DIO3_PTGDR2 | 0.8948 | 0.829-0.9533 |
| CCL23_DNAI7_TXNDC11_DIO3_PTGDR2 | 0.842 | 0.7472-0.9216 |
| CCL23_PRR11_TXNDC11_DIO3_PTGDR2 | 0.8749 | 0.7974-0.9355 |
| DNAI7_GALM_GINS4_DIO3_PTGDR2 | 0.8736 | 0.7991-0.9377 |
| PRR11_GALM_GINS4_DIO3_PTGDR2 | 0.9134 | 0.8567-0.9576 |
| CCL23_GALM_GINS4_DIO3_PTGDR2 | 0.8675 | 0.7839-0.9333 |
| PRR11_DNAI7_GINS4_DIO3_PTGDR2 | 0.9143 | 0.8554-0.9606 |
| CCL23_DNAI7_GINS4_DIO3_PTGDR2 | 0.871 | 0.7887-0.9399 |
| CCL23_PRR11_GINS4_DIO3_PTGDR2 | 0.9022 | 0.8368-0.9502 |
| PRR11_DNAI7_GALM_DIO3_PTGDR2 | 0.9069 | 0.8537-0.9545 |
| CCL23_DNAI7_GALM_DIO3_PTGDR2 | 0.8584 | 0.7749-0.9303 |
| CCL23_PRR11_GALM_DIO3_PTGDR2 | 0.8987 | 0.8372-0.9498 |
| CCL23_PRR11_DNAI7_DIO3_PTGDR2 | 0.9017 | 0.8355-0.9584 |
| GALM_GINS4_TXNDC11_CAV1_PTGDR2 | 0.8745 | 0.7918-0.9351 |
| DNAI7_GINS4_TXNDC11_CAV1_PTGDR2 | 0.874 | 0.797-0.9385 |
| PRR11_GINS4_TXNDC11_CAV1_PTGDR2 | 0.8801 | 0.8082-0.9381 |
| CCL23_GINS4_TXNDC11_CAV1_PTGDR2 | 0.8494 | 0.7697-0.923 |
| DNAI7_GALM_TXNDC11_CAV1_PTGDR2 | 0.8797 | 0.8078-0.9424 |
| PRR11_GALM_TXNDC11_CAV1_PTGDR2 | 0.8762 | 0.8082-0.9372 |
| CCL23_GALM_TXNDC11_CAV1_PTGDR2 | 0.8602 | 0.7801-0.9256 |
| PRR11_DNAI7_TXNDC11_CAV1_PTGDR2 | 0.881 | 0.813-0.9424 |
| CCL23_DNAI7_TXNDC11_CAV1_PTGDR2 | 0.8597 | 0.7714-0.9334 |
| CCL23_PRR11_TXNDC11_CAV1_PTGDR2 | 0.8732 | 0.8013-0.9355 |
| DNAI7_GALM_GINS4_CAV1_PTGDR2 | 0.8887 | 0.8156-0.9429 |
| PRR11_GALM_GINS4_CAV1_PTGDR2 | 0.8896 | 0.829-0.9437 |
| CCL23_GALM_GINS4_CAV1_PTGDR2 | 0.8775 | 0.8051-0.939 |
| PRR11_DNAI7_GINS4_CAV1_PTGDR2 | 0.8913 | 0.8259-0.9494 |
| CCL23_DNAI7_GINS4_CAV1_PTGDR2 | 0.8788 | 0.7974-0.9481 |
| CCL23_PRR11_GINS4_CAV1_PTGDR2 | 0.8866 | 0.8203-0.9429 |
| PRR11_DNAI7_GALM_CAV1_PTGDR2 | 0.8961 | 0.8338-0.9481 |
| CCL23_DNAI7_GALM_CAV1_PTGDR2 | 0.8831 | 0.8182-0.9385 |
| CCL23_PRR11_GALM_CAV1_PTGDR2 | 0.8926 | 0.826-0.9459 |
| CCL23_PRR11_DNAI7_CAV1_PTGDR2 | 0.8926 | 0.822-0.9476 |
| DNAI7_GALM_GINS4_TXNDC11_PTGDR2 | 0.8567 | 0.7779-0.9282 |
| PRR11_GALM_GINS4_TXNDC11_PTGDR2 | 0.8913 | 0.8247-0.9507 |
| CCL23_GALM_GINS4_TXNDC11_PTGDR2 | 0.8385 | 0.7433-0.916 |
| PRR11_DNAI7_GINS4_TXNDC11_PTGDR2 | 0.8844 | 0.8112-0.9429 |
| CCL23_DNAI7_GINS4_TXNDC11_PTGDR2 | 0.8277 | 0.7351-0.9173 |
| CCL23_PRR11_GINS4_TXNDC11_PTGDR2 | 0.8563 | 0.7817-0.9269 |
| CCL23_PRR11_TXNDC11_DIO3_PTGDR2 | 0.8749 | 0.7974-0.9355 |
| PRR11_DNAI7_GALM_TXNDC11_PTGDR2 | 0.8775 | 0.813-0.9368 |
| CCL23_DNAI7_GALM_TXNDC11_PTGDR2 | 0.8286 | 0.7415-0.9061 |
| CCL23_PRR11_GALM_TXNDC11_PTGDR2 | 0.8597 | 0.7823-0.9225 |
| CCL23_PRR11_DNAI7_TXNDC11_PTGDR2 | 0.8632 | 0.7801-0.926 |
| PRR11_DNAI7_GALM_GINS4_PTGDR2 | 0.8857 | 0.8199-0.9442 |
| CCL23_DNAI7_GALM_GINS4_PTGDR2 | 0.8571 | 0.7757-0.9251 |
| CCL23_PRR11_GALM_GINS4_PTGDR2 | 0.874 | 0.7965-0.9364 |
| CCL23_PRR11_DNAI7_GINS4_PTGDR2 | 0.8745 | 0.7996-0.9381 |
| CCL23_PRR11_DNAI7_GALM_PTGDR2 | 0.8758 | 0.8025-0.9329 |
| GALM_GINS4_TXNDC11_CAV1_DIO3 | 0.8861 | 0.8225-0.9468 |
| DNAI7_GINS4_TXNDC11_CAV1_DIO3 | 0.8879 | 0.8225-0.9481 |
| PRR11_GINS4_TXNDC11_CAV1_DIO3 | 0.9004 | 0.8337-0.9533 |
| CCL23_GINS4_TXNDC11_CAV1_DIO3 | 0.8948 | 0.8182-0.9537 |
| DNAI7_GALM_TXNDC11_CAV1 DIO | 0.8775 | 0.8039-0.9411 |
| PRR11_GALM_TXNDC11_CAV1_DIO3 | 0.9017 | 0.8394-0.955 |
| CCL23_GALM_TXNDC11_CAV1_DIO3 | 0.9022 | 0.8377-0.9541 |
| PRR11_DNAI7_TXNDC11_CAV1_DIO3 | 0.9039 | 0.839-0.9567 |
| CCL23_DNAI7_TXNDC11_CAV1_DIO3 | 0.8965 | 0.8238-0.955 |
| CCL23_PRR11_TXNDC11_CAV1_DIO3 | 0.9143 | 0.8589-0.9597 |
| DNAI7_GALM_GINS4_CAV1_DIO3 | 0.9013 | 0.8355-0.9554 |
| PRR11_GALM_GINS4_CAV1_DIO3 | 0.9165 | 0.8649-0.9632 |
| CCL23_GALM_GINS4_CAV1_DIO3 | 0.9208 | 0.8636-0.9697 |
| PRR11_DNAI7_GINS4_CAV1_DIO3 | 0.916 | 0.8589-0.9597 |
| CCL23_DNAI7_GINS4_CAV1_DIO3 | 0.9177 | 0.8532-0.9672 |
| CCL23_PRR11_GINS4_CAV1_DIO3 | 0.9242 | 0.871-0.9667 |
| PRR11_DNAI7_GALM_CAV1_DIO3 | 0.9152 | 0.8631-0.9593 |
| CCL23-DNAI7-GALM_CAV1_DIO3 | 0.9134 | 0.8554-0.9623 |
| CCL23_PRR11_GALM_CAV1_DIO | 0.9303 | 0.8761-0.9693 |
| CCL23_PRR11_DNAI7_CAV1_DIO3 | 0.9333 | 0.884-0.9723 |
| DNAI7_GALM_GINS4_TXNDC11_DIO3 | 0.8481 | 0.7697-0.9126 |
| PRR11_GALM_GINS4_TXNDC11_DIO3 | 0.897 | 0.8346-0.9498 |
| CCL23_GALM_GINS4_TXNDC11_DIO3 | 0.8831 | 0.8164-0.9403 |
| PRR11_DNAI7_GINS4_TXNDC11_DIO3 | 0.8991 | 0.8255-0.9528 |
| CCL23_DNAI7_GINS4_TXNDC11_DIO3 | 0.8762 | 0.7948-0.9359 |
| CCL23_PRR11_GINS4_TXNDC11_DIO3 | 0.9091 | 0.8463-0.9589 |
| PRR11_DNAI7_GALM_TXNDC11_DIO3 | 0.8879 | 0.8182-0.9424 |
| CCL23_DNAI7_GALM_TXNDC11_DIO3 | 0.8584 | 0.7809-0.9238 |
| CCL23_PRR11_GALM_TXNDC11_DIO3 | 0.9056 | 0.845-0.9576 |
| CCL23_PRR11_DNAI7_TXNDC11_DIO3 | 0.9056 | 0.8485-0.9558 |
| PRR11_DNAI7_GALM_GINS4_DIO3 | 0.9035 | 0.8398-0.9554 |
| CCL23_DNAI7_GALM_GINS4_DIO3 | 0.8892 | 0.826-0.9459 |
| CCL23_PRR11_GALM_GINS4_DIO3 | 0.9182 | 0.8649-0.9615 |
| CCL23_PRR11_DNAI7_GINS4_DIO3 | 0.9238 | 0.8706-0.9667 |
| CCL23_PRR11_DNAI7_GALM_DIO3 | 0.9143 | 0.8606-0.958 |
| DNAI7_GALM_GINS4_TXNDC11_CAV1 | 0.8818 | 0.8121-0.945 |
| PRR11_GALM_GINS4_TXNDC11_CAV1 | 0.8823 | 0.8121-0.9351 |
| CCL23_GALM_GINS4_TXNDC11_CAV1 | 0.8952 | 0.8368-0.9506 |
| PRR11_DNAI7_GINS4_TXNDC11_CAV1 | 0.8853 | 0.8203-0.9446 |
| CCL23_DNAI7_GINS4_TXNDC11_CAV1 | 0.8939 | 0.8173-0.9546 |
| CCL23_PRR11_GINS4_TXNDC11_CAV1 | 0.8922 | 0.8173-0.9485 |
| PRR11_DNAI7_GALM_TXNDC11_CAV1 | 0.8905 | 0.8147-0.9472 |
| CCL23_DNAI7_GALM_TXNDC11_CAV1 | 0.9035 | 0.8433-0.9554 |
| CCL23_PRR11_GALM_TXNDC11_CAV1 | 0.9026 | 0.8429-0.9533 |
| CCL23_PRR11_DNAI7_TXNDC11_CAV1 | 0.9056 | 0.8481-0.9567 |
| PRR11_DNAI7_GALM_GINS4_CAV1 | 0.8948 | 0.829-0.952 |
| CCL23_DNAI7_GALM_GINS4CAV1 | 0.913 | 0.8588-0.9598 |
| CCL23_PRR11_GALM_GINS4_CAV1 | 0.9048 | 0.8441-0.9533 |
| CCL23_PRR11_DNAI7_GINS4_CAV1 | 0.9095 | 0.8571-0.9602 |
| CCL23_PRR11_DNAI7_GALM_CAV1 | 0.9156 | 0.8589-0.9576 |
| PRR11_DNAI7_GALM_GINS4_TXNDC11 | 0.8753 | 0.8035-0.9342 |
| CCL23_DNAI7_GALM_GINS4_TXNDC11 | 0.8749 | 0.7904-0.942 |
| CCL23_PRR11_GALM_GINS4_TXNDC11 | 0.8918 | 0.826-0.9485 |
| CCL23_PRR11_DNAI7_GINS4_TXNDC11 | 0.89 | 0.8177-0.9476 |
| CCL23_PRR11_DNAI7_GALM_TXNDC11 | 0.8909 | 0.8212-0.9455 |
| CCL23_PRR11_DNAI7_GALM_GINS4 | 0.8974 | 0.8333-0.9485 |
| TXNDC11_MAPRE3_DIO3_GPR107_CADM1 | 0.8775 | 0.7913-0.9468 |
| GINS4_MAPRE3_DIO3_GPR107_CADM1 | 0.8797 | 0.8056-0.9403 |
| GALM_MAPRE3_DIO3_GPR107_CADM1 | 0.8775 | 0.8082-0.9338 |
| DNAI7_MAPRE3_DIO3_GPR107_CADM1 | 0.855 | 0.7753-0.9216 |
| PRR11_MAPRE3_DIO3_GPR107_CADM1 | 0.8853 | 0.81-0.9411 |
| CCL23_MAPRE3_DIO3_GPR107_CADM1 | 0.8606 | 0.7805-0.9329 |
| GINS4_TXNDC11_DIO3_GPR107_CADM1 | 0.8835 | 0.7996-0.9472 |
| GALM_TXNDC11_DIO3_GPR107_CADM1 | 0.8983 | 0.8406-0.9524 |
| DNAI7_TXNDC11_DIO3_GPR107_CADM1 | 0.8805 | 0.7987-0.9416 |
| PRR11_TXNDC11_DIO3_GPR107_CADM1 | 0.8848 | 0.8086-0.9442 |
| CCL23_TXNDC11_DIO3_GPR107_CADM1 | 0.8701 | 0.7835-0.9381 |
| GALM_GINS4_DIO3_GPR107_CADM1 | 0.8974 | 0.8415-0.9481 |
| DNAI7_GINS4_DIO3_GPR107_CADM1 | 0.8805 | 0.8099-0.9368 |
| PRR11_GINS4_DIO3_GPR107_CADM1 | 0.8931 | 0.8329-0.945 |
| CCL23_GINS4_DIO3_GPR107_CADM1 | 0.8823 | 0.8117-0.9416 |
| DNAI7_GALM_DIO3_GPR107_CADM1 | 0.8719 | 0.8056-0.9299 |
| PRR11_GALM_DIO3_GPR107_CADM1 | 0.8918 | 0.8281-0.9438 |
| CCL23_GALM_DIO3_GPR107_CADM1 | 0.8818 | 0.8151-0.9394 |
| PRR11_DNAI7_DIO3_GPR107_CADM1 | 0.8818 | 0.8177-0.9381 |
| CCL23_DNAI7_DIO3_GPR107_CADM1 | 0.8619 | 0.7809-0.9277 |
| CCL23_PRR11_DIO3_GPR107_CADM1 | 0.8749 | 0.8022-0.9368 |
| GINS4_TXNDC11_MAPRE3_GPR107_CADM1 | 0.8688 | 0.7827-0.9342 |
| GALM_TXNDC11_MAPRE3_GPR107_CADM1 | 0.9004 | 0.8303-0.9541 |
| DNAI7_TXNDC11_MAPRE3_GPR107_CADM1 | 0.8662 | 0.7792-0.9364 |
| PRR11_TXNDC11_MAPRE3_GPR107_CADM1 | 0.8792 | 0.7983-0.945 |
| CCL23_TXNDC11_MAPRE3_GPR107_CADM1 | 0.8667 | 0.7706-0.9463 |
| GALM_GINS4_MAPRE3_GPR107_CADM1 | 0.8874 | 0.8177-0.942 |
| DNAI7_GINS4_MAPRE3_GPR107_CADM1 | 0.8693 | 0.7965-0.9299 |
| PRR11_GINS4_MAPRE3_GPR107_CADM1 | 0.8805 | 0.8091-0.9377 |
| CCL23_GINS4_MAPRE3_GPR107_CADM1 | 0.8745 | 0.7922-0.9364 |
| DNAI7_GALM_MAPRE3_GPR107_CADM1 | 0.8571 | 0.7861-0.9191 |
| PRR11_GALM_MAPRE3_GPR107_CADM1 | 0.8866 | 0.8247-0.9437 |
| CCL23_GALM_MAPRE3_GPR107_CADM1 | 0.8866 | 0.822-0.9416 |
| PRR11_DNAI7_MAPRE3_GPR107_CADM1 | 0.8766 | 0.8074-0.9372 |
| CCL23_DNAI7_MAPRE3_GPR107_CADM1 | 0.8584 | 0.768-0.9264 |
| CCL23_PRR11_MAPRE3_GPR107_CADM1 | 0.8784 | 0.7956-0.9446 |
| GALM_GINS4_TXNDC11_GPR107_CADM1 | 0.8952 | 0.8281-0.9476 |
| DNAI7_GINS4_TXNDC11_GPR107_CADM1 | 0.8688 | 0.79-0.9407 |
| PRR11_GINS4_TXNDC11_GPR107_CADM1 | 0.8818 | 0.8099-0.9403 |
| CCL23_GINS4_TXNDC11_GPR107_CADM1 | 0.8654 | 0.7788-0.9377 |
| DNAI7_GALM_TXNDC11_GPR107_CADM1 | 0.8823 | 0.8129-0.9403 |
| PRR11_GALM_TXNDC11_GPR107_CADM1 | 0.8944 | 0.8299-0.9507 |
| CCL23_GALM_TXNDC11_GPR107_CADM1 | 0.8978 | 0.835-0.9485 |
| PRR11_DNAI7_TXNDC11_GPR107_CADM1 | 0.8762 | 0.7965-0.942 |
| CCL23_DNAI7_TXNDC11_GPR107_CADM1 | 0.8684 | 0.7762-0.9485 |
| CCL23_PRR11_TXNDC11_GPR107_CADM1 | 0.8723 | 0.7939-0.939 |
| DNAI7_GALM_GINS4_GPR107_CADM1 | 0.8714 | 0.8013-0.9303 |
| PRR11_GALM_GINS4_GPR107_CADM1 | 0.887 | 0.8212-0.9446 |
| CCL23_GALM_GINS4_GPR107_CADM1 | 0.8957 | 0.8316-0.9489 |
| PRR11_DNAI7_GINS4_GPR107_CADM1 | 0.8792 | 0.816-0.9356 |
| CCL23_DNAI7_GINS4_GPR107_CADM1 | 0.8801 | 0.8035-0.9433 |
| CCL23_PRR11_GINS4_GPR107_CADM1 | 0.8853 | 0.8155-0.942 |
| PRR11_DNAI7_GALM_GPR107_CADM1 | 0.8771 | 0.8069-0.9355 |
| CCL23_DNAI7_GALM_GPR107_CADM1 | 0.874 | 0.8026-0.9281 |
| CCL23_PRR11_GALM_GPR107_CADM1 | 0.8926 | 0.8312-0.9485 |
| CCL23_PRR11_DNAI7_GPR107_CADM1 | 0.8866 | 0.8173-0.9463 |
| GINS4_TXNDC11_MAPRE3_DIO3_CADM1 | 0.89 | 0.813-0.9498 |
| GALM_TXNDC11_MAPRE3_DIO3_CADM1 | 0.8957 | 0.8346-0.9494 |
| DNAI7_TXNDC11_MAPRE3_DIO3_CADM1 | 0.8784 | 0.8004-0.9481 |
| PRR11_TXNDC11_MAPRE3_DIO3 CADM1 | 0.89 | 0.813-0.9476 |
| CCL23_TXNDC11_MAPRE3_DIO3_CADM1 | 0.8766 | 0.787-0.9411 |
| GALM_GINS4_MAPRE3_DIO3_CADM1 | 0.8874 | 0.8216-0.9403 |
| DNAI7_GINS4_MAPRE3_DIO3_CADM1 | 0.8745 | 0.7983-0.9325 |
| PRR11_GINS4_MAPRE3_DIO3_CADM1 | 0.8926 | 0.8303-0.9472 |
| CCL23_GINS4_MAPRE3_DIO3_CADM1 | 0.8762 | 0.8035-0.9351 |
| DNAI7_GALM_MAPRE3_DIO3_CADM1 | 0.8658 | 0.7909-0.9273 |
| PRR11_GALM_MAPRE3_DIO3_CADM1 | 0.8874 | 0.8182-0.9481 |
| CCL23_GALM_MAPRE3_DIO3_CADM1 | 0.8753 | 0.8056-0.9303 |
| PRR11_DNAI7_MAPRE3_DIO3_CADM1 | 0.8745 | 0.8013-0.9347 |
| CCL23_DNAI7_MAPRE3_DIO3_CADM1 | 0.855 | 0.7701-0.9212 |
| CCL23_PRR11_MAPRE3_DIO3_CADM1 | 0.8784 | 0.8034-0.9372 |
| GALM_GINS4_TXNDC11_DIO3_CADM1 | 0.9065 | 0.8454-0.9554 |
| DNAI7_GINS4_TXNDC11_DIO3_CADM1 | 0.8909 | 0.8069-0.9502 |
| PRR11_GINS4_TXNDC11_DIO3_CADM1 | 0.8952 | 0.8242-0.952 |
| CCL23_GINS4_TXNDC11_DIO3_CADM1 | 0.8892 | 0.8082-0.9529 |
| DNAI7_GALM_TXNDC11_DIO3_CADM1 | 0.8974 | 0.8372-0.9506 |
| PRR11_GALM_TXNDC11_DIO3_CADM1 | 0.9069 | 0.8455-0.958 |
| CCL23_GALM_TXNDC11_DIO3_CADM1 | 0.8996 | 0.8342-0.9537 |
| PRR11_DNAI7_TXNDC11_DIO3_CADM1 | 0.8931 | 0.8207-0.9533 |
| CCL23_DNAI7_TXNDC11_DIO3_CADM1 | 0.8835 | 0.8099-0.9494 |
| CCL23_PRR11_TXNDC11_DIO3_CADM1 | 0.8848 | 0.8151-0.9476 |
| PRR11_GALM_GINS4_DIO3_CADM1 | 0.9022 | 0.8355-0.9511 |
| CCL23_GALM_GINS4_DIO3_CADM1 | 0.8965 | 0.8394-0.9489 |
| PRR11_DNAI7_GINS4_DIO3_CADM1 | 0.8926 | 0.8251-0.9463 |
| CCL23_DNAI7_GINS4_DIO3_CADM1 | 0.8792 | 0.8056-0.939 |
| PRR11_DNAI7_GALM_DIO3_CADM1 | 0.8823 | 0.8138-0.939 |
| CCL23_DNAI7_GALM_DIO3_CADM1 | 0.8701 | 0.7995-0.9281 |
| CCL23_PRR11_GALM_DIO3_CADM1 | 0.8905 | 0.8229-0.9468 |
| CCL23_PRR11_DNAI7_DIO3_CADM1 | 0.8827 | 0.8121-0.9411 |
| GALM_GINS4_TXNDC11_MAPRE3-CADM1 | 0.8961 | 0.8316-0.9476 |
| DNAI7_GINS4_TXNDC11_MAPRE3_CADM1 | 0.8675 | 0.7853-0.9303 |
| CCL23_GINS4_TXNDC11_MAPRE3_CADM1 | 0.8675 | 0.7861-0.9377 |
| DNAI7_GALM_TXNDC11_MAPRE3_CADM1 | 0.8874 | 0.8212-0.9442 |
| PRR11_GALM_TXNDC11_MAPRE3_CADM1 | 0.8961 | 0.8351-0.9489 |
| CCL23_GALM_TXNDC11_MAPRE3_CADM1 | 0.903 | 0.8385-0.9537 |
| PRR11_DNAI7_TXNDC11_MAPRE3_CADM1 | 0.8753 | 0.8056-0.942 |
| CCL23_DNAI7_TXNDC11_MAPRE3_CADM1 | 0.8654 | 0.7749-0.9385 |
| CCL23_PRR11_TXNDC11_MAPRE3_CADM1 | 0.8753 | 0.7887-0.9394 |
| DNAI7_GALM_GINS4_MAPRE3_CADM1 | 0.8619 | 0.7891-0.9225 |
| PRR11_GALM_GINS4_MAPRE3_CADM1 | 0.8861 | 0.8152-0.9416 |
| CCL23_GALM_GINS4_MAPRE3_CADM1 | 0.887 | 0.8199-0.9424 |
| PRR11_DNAI7_GINS4_MAPRE3_CADM1 | 0.8723 | 0.8013-0.932 |
| CCL23_DNAI7_GINS4_MAPRE3_CADM1 | 0.8667 | 0.7853-0.9277 |
| CCL23_PRR11_GINS4_MAPRE3_CADM1 | 0.874 | 0.8009-0.9403 |
| PRR11_DNAI7_GALM_MAPRE3_CADM1 | 0.8632 | 0.79-0.9273 |
| CCL23_DNAI7_GALM_MAPRE3_CADM1 | 0.8649 | 0.7943-0.9273 |
| CCL23_PRR11_GALM_MAPRE3_CADM1 | 0.887 | 0.8233-0.9446 |
| CCL23_PRR11_DNAI7_MAPRE3_CADM1 | 0.8801 | 0.8125-0.9385 |
| DNAI7_GALM_GINS4_TXNDC11_CADM1 | 0.8887 | 0.8208-0.942 |
| PRR11_GALM_GINS4_TXNDC11_CADM1 | 0.8996 | 0.8415-0.9507 |
| CCL23_GALM_GINS4_TXNDC11_CADM1 | 0.9043 | 0.8415-0.9572 |
| PRR11_DNAI7_GINS4_TXNDC11_CADM1 | 0.8771 | 0.8013-0.9416 |
| CCL23_DNAI7_GINS4_TXNDC11_CADM1 | 0.8714 | 0.787-0.9416 |
| CCL23_PRR11_GINS4_TXNDC11_CADM1 | 0.8723 | 0.7943-0.9364 |
| PRR11_DNAI7_GALM_TXNDC11_CADM1 | 0.8905 | 0.8264-0.9433 |
| CCL23_DNAI7_GALM_TXNDC11_CADM1 | 0.8918 | 0.8329-0.9455 |
| CCL23_PRR11_GALM_TXNDC11_CADM1 | 0.9043 | 0.8398-0.9524 |
| PRR11_DNAI7_GALM_GINS4_CADM1 | 0.8727 | 0.7996-0.9325 |
| CCL23_DNAI7_GALM_GINS4_CADM1 | 0.8745 | 0.8008-0.9338 |
| CCL23_PRR11_GALM_GINS4_CADM1 | 0.8909 | 0.829-0.9498 |
| CCL23_PRR11_DNAI7_GINS4_CADM1 | 0.8866 | 0.8199-0.9429 |
| CCL23_PRR11_DNAI7_GALM_CADM1 | 0.8771 | 0.8095-0.9359 |
| GINS4_TXNDC11_MAPRE3_DIO3_GPR107 | 0.89 | 0.8203-0.9455 |
| GALM_TXNDC11_MAPRE3_DIO3_GPR107 | 0.9048 | 0.8381-0.9528 |
| DNAI7_TXNDC11_MAPRE3_DIO3_GPR107 | 0.881 | 0.8017-0.9489 |
| PRR11_TXNDC11_MAPRE3_DIO3_GPR107 | 0.8935 | 0.8277-0.9476 |
| CCL23_TXNDC11_MAPRE3_DIO3_GPR107 | 0.8749 | 0.7982-0.9425 |
| GALM_GINS4_MAPRE3_DIO3_GPR107 | 0.8861 | 0.8182-0.9433 |
| DNAI7_GINS4_MAPRE3_DIO3_GPR107 | 0.8671 | 0.7896-0.9286 |
| PRR11_GINS4_MAPRE3_DIO3_GPR107 | 0.8931 | 0.832-0.9446 |
| CCL23_GINS4_MAPRE3_DIO3_GPR107 | 0.8814 | 0.816-0.9394 |
| DNAI7_GALM_MAPRE3_DIO3_GPR107 | 0.8641 | 0.7922-0.923 |
| PRR11_GALM _MAPRE3_DIO3_GPR107 | 0.8831 | 0.8156-0.9429 |
| CCL23_GALM_MAPRE3_DIO3_GPR107 | 0.8693 | 0.7974-0.932 |
| PRR11_DNAI7_MAPRE3_DIO3_GPR107 | 0.8762 | 0.8138-0.9325 |
| CCL23_DNAI7_MAPRE3_DIO3_GPR107 | 0.8472 | 0.7528-0.9177 |
| CCL23_PRR11_MAPRE3_DIO3_GPR107 | 0.8771 | 0.8056-0.9338 |
| GALM_GINS4_TXNDC11_DIO3_GPR107 | 0.9134 | 0.8576-0.961 |
| DNAI7_GINS4_TXNDC11_DIO3_GPR107 | 0.887 | 0.8117-0.9476 |
| PRR11_GINS4_TXNDC11_DIO3_GPR107 | 0.9 | 0.832-0.9533 |
| CCL23_GINS4_TXNDC11_DIO3_GPR107 | 0.8818 | 0.803-0.9442 |
| DNAI7_GALM_TXNDC11_DIO3_GPR107 | 0.8948 | 0.8303-0.9494 |
| PRR11_GALM_TXNDC11_DIO3_GPR107 | 0.9052 | 0.8433-0.9533 |
| CCL23_GALM_TXNDC11_DIO3_GPR107 | 0.9017 | 0.8402-0.9528 |
| PRR11_DNAI7_TXNDC11_DIO3_GPR107 | 0.8918 | 0.8225-0.9498 |
| CCL23_DNAI7_TXNDC11_DIO3_GPR107 | 0.8775 | 0.8-0.9463 |
| CCL23_PRR11_TXNDC11_DIO3_GPR107 | 0.8866 | 0.7995-0.9537 |
| DNAI7_GALM_GINS4_DIO3_GPR107 | 0.8835 | 0.8108-0.939 |
| PRR11_GALM_GINS4_DIO3_GPR107 | 0.8987 | 0.8368-0.9472 |
| CCL23_GALM_GINS4_DIO3_GPR107 | 0.8991 | 0.8441-0.9511 |
| PRR11_DNAI7_GINS4_DIO3_GPR107 | 0.8861 | 0.816-0.945 |
| CCL23_DNAI7_GINS4_DIO3_GPR107 | 0.8766 | 0.8061-0.9381 |
| CCL23_PRR11_GINS4_DIO3_GPR107 | 0.8909 | 0.8307-0.9424 |
| PRR11_DNAI7_GALM_DIO3_GPR107 | 0.8771 | 0.8061-0.9346 |
| CCL23_DNAI7_GALM_DIO3_GPR107 | 0.8675 | 0.8004-0.9269 |
| CCL23_PRR11_GALM_DIO3_GPR107 | 0.8879 | 0.8199-0.9394 |
| CCL23_PRR11_DNAI7_DIO3_GPR107 | 0.8762 | 0.8056-0.9303 |
| GALM_GINS4_TXNDC11_MAPRE3_GPR107 | 0.9078 | 0.8459-0.9558 |
| DNAI7_GINS4_TXNDC11_MAPRE3_GPR107 | 0.8736 | 0.7926-0.9372 |
| PRR11_GINS4_TXNDC11_MAPRE3_GPR107 | 0.8905 | 0.829-0.9502 |
| CCL23_GINS4_TXNDC11_MAPRE3_GPR107 | 0.881 | 0.803-0.9468 |
| DNAI7_GALM_TXNDC11_MAPRE3_GPR107 | 0.8879 | 0.8264-0.9424 |
| PRR11_GALM_TXNDC11_MAPRE3_GPR107 | 0.9165 | 0.8545-0.9662 |
| CCL23_GALM_TXNDC11_MAPRE3_GPR107 | 0.9104 | 0.851-0.9606 |
| PRR11_DNAI7_TXNDC11_MAPRE3_GPR107 | 0.8835 | 0.8182-0.942 |
| CCL23_DNAI7_TXNDC11_MAPRE3_GPR107 | 0.8719 | 0.7879-0.9463 |
| CCL23_PRR11_TXNDC11_MAPRE3_GPR107 | 0.8905 | 0.8151-0.9507 |
| DNAI7_GALM_GINS4_MAPRE3_GPR107 | 0.8602 | 0.7865-0.923 |
| PRR11_GALM_GINS4_MAPRE3_GPR107 | 0.8887 | 0.8212-0.9407 |
| CCL23_GALM_GINS4_MAPRE3_GPR107 | 0.8905 | 0.8247-0.9429 |
| PRR11_DNAI7_GINS4_MAPRE3_GPR107 | 0.8797 | 0.8134-0.9368 |
| CCL23_DNAI7_GINS4_MAPRE3_GPR107 | 0.8732 | 0.7982-0.9385 |
| CCL23_PRR11_GINS4_MAPRE3_GPR107 | 0.8896 | 0.8143-0.9468 |
| PRR11_DNAI7_GALM_MAPRE3_GPR107 | 0.861 | 0.7892-0.926 |
| CCL23_DNAI7_GALM_MAPRE3_GPR107 | 0.8641 | 0.787-0.9299 |
| CCL23_PRR11_GALM_MAPRE3_GPR107 | 0.8913 | 0.8251-0.9481 |
| CCL23_PRR11_DNAI7_MAPRE3_GPR107 | 0.884 | 0.8126-0.9407 |
| DNAI7_GALM_GINS4_TXNDC11_GPR107 | 0.89 | 0.8216-0.9489 |
| PRR11_GALM_GINS4_TXNDC11_GPR107 | 0.9026 | 0.8415-0.955 |
| CCL23_GALM_GINS4_TXNDC11_GPR107 | 0.9043 | 0.842-0.9554 |
| PRR11_DNAI7_GINS4_TXNDC11_GPR107 | 0.8779 | 0.7996-0.939 |
| CCL23_DNAI7_GINS4_TXNDC11_GPR107 | 0.8719 | 0.784-0.9459 |
| CCL23_PRR11_GINS4_TXNDC11_GPR107 | 0.89 | 0.8169-0.9446 |
| PRR11_DNAI7_GALM_TXNDC11_GPR107 | 0.8905 | 0.826-0.9446 |
| CCL23_DNAI7_GALM_TXNDC11_GPR107 | 0.8918 | 0.8234-0.9481 |
| CCL23_PRR11_DNAI7_GALM_GINS4_GPR107 | 0.8853 | 0.826-0.9437 |
| PRR11_DNAI7_GALM_GINS4_GPR107 | 0.8675 | 0.7922-0.9333 |
| CCL23_DNAI7_GALM_GINS4_GPR107 | 0.8667 | 0.7852-0.932 |
| CCL23_PRR11_GALM_GINS4_GPR107 | 0.8896 | 0.8285-0.9459 |
| CCL23_PRR11_DNAI7_GINS4_GPR107 | 0.8835 | 0.8099-0.9446 |
| GALM_GINS4_TXNDC11_MAPRE3_DIO3 | 0.9095 | 0.8523-0.9589 |
| DNAI7_GINS4_TXNDC11_MAPRE3_DIO3 | 0.8879 | 0.8186-0.9459 |
| PRR11_GINS4_TXNDC11_MAPRE3_DIO3 | 0.9035 | 0.8346-0.955 |
| CCL23_GINS4_TXNDC11_MAPRE3_DIO3 | 0.8939 | 0.8169-0.9563 |
| DNAI7_GALM_TXNDC11_MAPRE3_DIO3 | 0.8944 | 0.8273-0.9494 |
| PRR11_GALM_TXNDC11_MAPRE3_DIO3 | 0.9078 | 0.8502-0.9528 |
| CCL23_GALM_TXNDC11_MAPRE3_DIO3 | 0.8996 | 0.8403-0.9468 |
| PRR11_DNAI7_TXNDC11_MAPRE3_DIO3 | 0.8935 | 0.8264-0.9472 |
| CCL23_DNAI7_TXNDC11_MAPRE3_DIO3 | 0.8814 | 0.8052-0.945 |
| CCL23_PRR11_TXNDC11_MAPRE3_DIO3 | 0.8922 | 0.8195-0.9532 |
| DNAI7_GALM_GINS4_MAPRE3_DIO3 | 0.8701 | 0.7982-0.9308 |
| PRR11_GALM_GINS4_MAPRE3_DIO3 | 0.8935 | 0.8225-0.9481 |
| CCL23_PRR11_DNAI7_GINS4_MAPRE3_DIO3 | 0.897 | 0.8277-0.9528 |
| PRR11_DNAI7_GINS4_MAPRE3_DIO3 | 0.8831 | 0.8043-0.9403 |
| CCL23_DNAI7_GINS4_MAPRE3_DIO3 | 0.8697 | 0.7939-0.9346 |
| CCL23_PRR11_GINS4_MAPRE3_DIO3 | 0.8922 | 0.8316-0.9485 |
| CCL23_PRR11_DNAI7_GALM_MAPRE3_DIO3 | 0.887 | 0.8169-0.945 |
| PRR11_DNAI7_GALM_MAPRE3_DIO3 | 0.8749 | 0.8034-0.9347 |
| CCL23_DNAI7_GALM_MAPRE3_DIO3 | 0.8645 | 0.7896-0.926 |
| CCL23_PRR11_GALM_MAPRE3_DIO3 | 0.881 | 0.8199-0.9377 |
| CCL23_PRR11_DNAI7_MAPRE3_DIO3 | 0.8697 | 0.7948-0.9325 |
| DNAI7_GALM_GINS4_TXNDC11_DIO3 | 0.9043 | 0.8493-0.9537 |
| PRR11_GALM_GINS4_TXNDC11_DIO3 | 0.9147 | 0.8589-0.9606 |
| CCL23_GALM_GINS4_TXNDC11_DIO3 | 0.9134 | 0.855-0.9606 |
| PRR11_DNAI7_GINS4_TXNDC11_DIO3 | 0.8978 | 0.8303-0.9507 |
| CCL23_DNAI7_GINS4_TXNDC11_DIO3 | 0.8913 | 0.8173-0.9515 |
| DNAI7_GINS4_TXNDC11_DIO3 | 0.8723 | 0.7896-0.9372 |
| CCL23_PRR11_GINS4_TXNDC11_DIO3 | 0.8978 | 0.8268-0.9546 |
| PRR11_DNAI7_GALM_TXNDC11_DIO3 | 0.9035 | 0.8381-0.9533 |
| CCL23_DNAI7_GALM_TXNDC11_DIO3 | 0.8952 | 0.8368-0.9455 |
| CCL23_PRR11_GALM_TXNDC11_DIO3 | 0.9087 | 0.8524-0.9602 |
| CCL23_PRR11_DNAI7_TXNDC11_DIO3 | 0.8939 | 0.8237-0.9537 |
| PRR11_DNAI7_GALM_GINS4_DIO3 | 0.8857 | 0.81-0.9472 |
| CCL23_DNAI7_GALM_GINS4_DIO3 | 0.8775 | 0.8091-0.9316 |
| CCL23_PRR11_GALM_GINS4_DIO3 | 0.9022 | 0.8402-0.9528 |
| CCL23_PRR11_DNAI7_GINS4_DIO3 | 0.8909 | 0.8242-0.9476 |
| CCL23_PRR11_DNAI7_GALM_DIO3_ | 0.8797 | 0.8065-0.9398 |
| DNAI7_GALM_GINS4_TXNDC11_MAPRE3 | 0.8879 | 0.8186-0.9481 |
| PRR11_GALM_GINS4_TXNDC11_MAPRE3 | 0.9039 | 0.8437-0.955 |
| CCL23_GALM_GINS4_TXNDC11_MAPRE3 | 0.9061 | 0.8428-0.9584 |
| PRR11_DNAI7_GINS4_TXNDC11_MAPRE3 | 0.8805 | 0.8073-0.942 |
| CCL23_PRR11_GINS4_TXNDC11_MAPRE3 | 0.881 | 0.8073-0.9442 |
| PRR11_DNAI7_GALM_TXNDC11_MAPRE3 | 0.8957 | 0.8325-0.9532 |
| CCL23_DNAI7-GALM_TXNDC11_MAPRE3 | 0.8926 | 0.8286-0.9481 |
| CCL23_PRR11_GALM_TXNDC11_MAPRE3 | 0.9126 | 0.8524-0.9584 |
| CCL23_PRR11_DNAI7_TXNDC11_MAPRE3 | 0.884 | 0.8095-0.9476 |
| PRR11_DNAI7_GALM_GINS4_MAPRE3 | 0.8658 | 0.7939-0.926 |
| CCL23_DNAI7_GALM_GINS4_MAPRE3 | 0.8636 | 0.7831-0.9268 |
| DNAI7_GALM_GINS4_MAPRE3 | 0.839 | 0.761-0.907 |
| CCL23_PRR11_GALM_GINS4_MAPRE3 | 0.8926 | 0.8272-0.9485 |
| CCL23_PRR11_DNAI7_GINS4_MAPRE3 | 0.8762 | 0.8022-0.9394 |
| CCL23_PRR11_DNAI7_GALM_MAPRE3 | 0.8654 | 0.7969-0.9277 |
| PRR11_DNAI7_GALM_GINS4_TXNDC11 | 0.8887 | 0.8251-0.9472 |
| CCL23_DNAI7_GALM_GINS4_TXNDC11 | 0.8926 | 0.8342-0.9511 |
| CCL23_PRR11_GALM_GINS4_TXNDC11 | 0.9087 | 0.8476-0.9593 |
| CCL23_PRR11_DNAI7_GINS4_TXNDC11 | 0.8814 | 0.8142-0.942 |
| CCL23_PRR11_DNAI7_GALM_TXNDC11 | 0.8939 | 0.8303-0.9455 |
| CCL23_PRR11_DNAI7_GALM_GINS4 | 0.8762 | 0.8025-0.9368 |

| **Combinations of six biomarkers** | | |
|---|---|---|
| PRR11_DNAI7_GALM_GINS4_TXNDC11_CAV1 | 0.9022 | 0.8433-0.9554 |
| PRR11_TXNDC11_ELL2_DIO3_UAP1_CDCA2 | 0.9065 | 0.8454-0.9559 |
| CCL23_PRR11_DNAI7_GALM_DIO3_CADM1 | 0.8974 | 0.8346-0.9502 |
| CCL23_TXNDC11_ELL2_DIO3_UAP1_CDCA2 | 0.8931 | 0.8108-0.9593 |
| CCL23_GALM_GINS4_TXNDC11_DIO3_CADM1 | 0.9169 | 0.8571-0.9628 |
| CCL23_PRR11_ELL2_DIO3_UAP1_CDCA2 | 0.8939 | 0.832-0.9489 |
| CCL23_PRR11_DNAI7_TXNDC11_MAPRE3_CADM1 | 0.8827 | 0.8091-0.9424 |
| CCL23_PRR11_TXNDC11_DIO3_UAP1_CDCA2 | 0.9273 | 0.8675-0.9749 |
| CCL23_DNAI7_GALM_GINS4_TXNDC11_CADM1 | 0.8996 | 0.8316-0.9515 |
| CCL23_PRR11_TXNDC11_ELL2_UAP1_CDCA2 | 0.8944 | 0.829-0.9529 |
| CCL23_PRR11_DNAI7_GALM_TXNDC11_CADM1 | 0.8991 | 0.8394-0.9498 |
| CCL23_PRR11_TXNDC11_ELL2_DIO3_CDCA2 | 0.9247 | 0.8615-0.9736 |
| CCL23_PRR11_DNAI7_GALM_GINS4_CADM1 | 0.89 | 0.819-0.9433 |
| CCL23_PRR11_TXNDC11_ELL2_DIO3_UAP1 | 0.9061 | 0.845-0.9589 |
| GAS6_GALM_TXNDC11_CAV1_DIO3_CADM1 | 0.9026 | 0.8385-0.9541 |
| PRR11_GALM_TXNDC11_CAV1_DIO3_CADM1 | 0.9255 | 0.8692-0.9697 |
| CCL23_GALM_TXNDC11_CAV1_DIO3_CADM1 | 0.9273 | 0.868-0.974 |
| PRR11_GAS6_TXNDC11_CAV1_DIO3_CADM1 | 0.8965 | 0.8307-0.9528 |
| CCL23_GAS6_TXNDC11_CAV1_DIO3_CADM1 | 0.8835 | 0.7983-0.9511 |
| CCL23_PRR11_TXNDC11_CAV1_DIO3_CADM1 | 0.9113 | 0.8446-0.9645 |
| PRR11_GAS6_GALM_CAV1_DIO3_CADM1 | 0.916 | 0.8576-0.9615 |
| CCL23_GAS6_GALM_CAV1_DIO3_CADM1 | 0.9065 | 0.842-0.9558 |
| CCL23_PRR11_GALM_CAV1_DIO3_CADM1 | 0.9333 | 0.8783-0.9766 |
| CCL23_PRR11_GAS6_CAV1_DIO3_CADM1 | 0.9052 | 0.8324-0.9619 |
| PRR11_GAS6_GALM_TXNDC11_DIO3_CADM1 | 0.926 | 0.8675-0.9693 |
| CCL23_GAS6_GALM_TXNDC11 _DIO3_CADM1 | 0.9277 | 0.8645-0.9753 |
| CCL23_PRR11_GALM_TXNDC11_DIO3_CADM1 | 0.939 | 0.8866-0.981 |
| CCL23_PRR11_GAS6_TXNDC11_DIO3_CADM1 | 0.9113 | 0.8433-0.9632 |
| CCL23_PRR11_GAS6_GALM_DIO3_CADM1 | 0.9338 | 0.8852-0.9758 |
| PRR11_GAS6-GALM_TXNDC11_CAV1_CADM1 | 0.89 | 0.8234-0.9437 |
| CCL23_GAS6_GALM_TXNDC11_CAV1_CADM1 | 0.8909 | 0.8242-0.9472 |
| CCL23_PRR11_GAS6_TXNDC11_CAV1_CADM1 | 0.8697 | 0.7926-0.9381 |
| CCL23_PRR11_GAS6_GALM_TXNDC11_CADM1 | 0.9026 | 0.8381-0.9537 |
| PRR11_GAS6_GALM_TXNDC11_CAV1_DIO3 | 0.9152 | 0.8628-0.9602 |
| CCL23_GAS6_GALM_TXNDC11_CAV1_DIO3 | 0.9134 | 0.8528-0.9602 |
| CCL23_PRR11_GALM_TXNDC11_CAV1_DIO3 | 0.9351 | 0.8831-0.9753 |
| CCL23_PRR11_GAS6_GALM_TXNDC11_DIO3 | 0.9351 | 0.884-0.9758 |
| CCL23_PRR11_GAS6_GALM_TXNDC11_CAV1 | 0.9039 | 0.8437-0.9567 |
| CCL23_PRR11_GAS6_TXNDC11_CAV1_DIO3 | 0.9087 | 0.8346-0.9623 |
| CCL23_PRR11_GAS6_GALM_CAV1_DIO3 | 0.9078 | 0.8467-0.9602 |
| CCL23_PRR11_GALM_TXNDC11_CAV1_CADM1 | 0.9026 | 0.8389-0.9546 |
| CCL23_PRR11_GAS6_GALM_CAV1_CADM1 | 0.8866 | 0.8125-0.9433 |
| GALM_GINS4_TXNDC11_CAV1_DIO3_PTGDR2 | 0.9009 | 0.8324-0.9502 |
| DNAI7_GINS4_TXNDC11_CAV1_DIO3_PTGDR2 | 0.8991 | 0.8285-0.9559 |
| PRR11_GINS4_TXNDC11_CAV1_DIO3_PTGDR2 | 0.9165 | 0.8597-0.9654 |
| CCL23_GINS4_TXNDC11_CAV1_DIO3_PTGDR2 | 0.8926 | 0.8173-0.955 |
| DNAI7_GALM_TXNDC11_CAV1_DIO3_PTGDR2 | 0.8983 | 0.832-0.9515 |
| PRR11_GALM_TXNDC11_CAV1_DIO3_PTGDR2 | 0.913 | 0.8541-0.9597 |
| CCL23_GALM_TXNDC11_CAV1_DIO3_PTGDR2 | 0.8957 | 0.8251-0.9559 |
| PRR11_DNAI7_TXNDC11_CAV1_DIO3_PTGDR2 | 0.9212 | 0.8636-0.9641 |
| CCL23_DNAI7_TXNDC11_CAV1_DIO3_PTGDR2 | 0.8957 | 0.8203-0.9593 |
| CCL23_PRR11_TXNDC11_CAV1_DIO3_PTGDR2 | 0.9147 | 0.8498-0.9619 |
| DNAI7_GALM_GINS4_CAV1_DIO3_PTGDR2 | 0.916 | 0.8545-0.9628 |
| PRR11_GALM_GINS4_CAV1_DIO3_PTGDR2 | 0.9251 | 0.8706-0.9671 |
| CCL23_GALM_GINS4_CAV1_DIO3_PTGDR2 | 0.9121 | 0.8485-0.9662 |
| PRR11_DNAI7_GINS4_CAV1_DIO3_PTGDR2 | 0.9307 | 0.8758-0.9688 |
| CCL23_DNAI7_GINS4_CAV1_DIO3_PTGDR2 | 0.9195 | 0.8493-0.9736 |
| CCL23_PRR11_GINS4_CAV1_DIO3_PTGDR2 | 0.9268 | 0.8671-0.9732 |
| PRR11_DNAI7_GALM_CAV1_DIO3_PTGDR2 | 0.926 | 0.8736-0.968 |
| CCL23_DNAI7_GALM_CAV1_DIO3_PTGDR2 | 0.9095 | 0.8442-0.9597 |
| CCL23_PRR11_GALM_CAV1_DIO3_PTGDR2 | 0.9281 | 0.8701-0.9675 |
| CCL23_PRR11_DNAI7_CAV1_DIO3_PTGDR2 | 0.9338 | 0.8779-0.9736 |
| DNAI7_GALM _GINS4_TXNDC11_DIO3_PTGDR2 | 0.8771 | 0.8091-0.9372 |
| PRR11_GALM_GINS4_TXNDC11_DIO3_PTGDR2 | 0.9169 | 0.8593-0.9624 |
| CCL23_GALM_GINS4_TXNDC11_DIO3_PTGDR2 | 0.8723 | 0.7995-0.9359 |
| PRR11_DNAI7_GINS4_TXNDC11_DIO3_PTGDR2 | 0.9169 | 0.8571-0.9628 |
| CCL23_DNAI7_GINS4_TXNDC11_DIO3_PTGDR2 | 0.8736 | 0.7887-0.9446 |
| CCL23_PRR11_GINS4_TXNDC11_DIO3_PTGDR2 | 0.9074 | 0.845-0.9593 |
| PRR11_DNAI7_GALM_TXNDC11_DIO3_PTGDR2 | 0.9108 | 0.8519-0.9593 |
| CCL23_DNAI7_GALM_TXNDC11_DIO3_PTGDR2 | 0.8623 | 0.7766-0.9277 |
| CCL23_PRR11_GALM_TXNDC11_DIO3_PTGDR2 | 0.9035 | 0.8351-0.9567 |
| CCL23_PRR11_DNAI7_TXNDC11_DIO3_PTGDR2 | 0.9043 | 0.8411-0.9563 |
| PRR11_DNAI7_GALM_GINS4_DIO3_PTGDR2 | 0.9199 | 0.8649-0.9649 |
| CCL23_DNAI7_GALM_GINS4_DIO3_PTGDR2 | 0.8831 | 0.8013-0.9455 |
| CCL23_PRR11_GALM_GINS4_DIO3_PTGDR2 | 0.9169 | 0.8584-0.9628 |
| CCL23_PRR11_DNAI7_GINS4_DIO3_PTGDR2 | 0.9203 | 0.8649-0.968 |
| CCL23_PRR11_DNAI7_GALM_DIO3_PTGDR2 | 0.9104 | 0.8498-0.9563 |
| DNAI7_GALM_GINS4_TXNDC11_CAV1_PTGDR2 | 0.89 | 0.8229-0.9494 |
| PRR11_GALM_GINS4_TXNDC11_CAV1_PTGDR2 | 0.8892 | 0.8242-0.9424 |
| CCL23_GALM_GINS4_TXNDC11_CAV1_PTGDR2 | 0.8771 | 0.8051-0.9368 |
| PRR11_DNAI7_GINS4_TXNDC11_CAV1_PTGDR2 | 0.8909 | 0.819-0.9433 |
| CCL23_DNAI7_GINS4_TXNDC11_CAV1_PTGDR2 | 0.8818 | 0.7957-0.945 |
| CCL23_PRR11_GINS4_TXNDC11_CAV1_PTGDR2 | 0.887 | 0.8169-0.9446 |
| PRR11_DNAI7_GALM_TXNDC11_CAV1_PTGDR2 | 0.8935 | 0.8298-0.9459 |
| CCL23_DNAI7_GALM_TXNDC11_CAV1_PTGDR2 | 0.8853 | 0.8173-0.9468 |
| CCL23_PRR11_GALM_TXNDC11_CAV1_PTGDR2 | 0.8935 | 0.8307-0.9455 |
| CCL23_PRR11_DNAI7_TXNDC11_CAV1_PTGDR2 | 0.8909 | 0.8303-0.9446 |
| PRR11_DNAI7_GALM_GINS4_CAV1_PTGDR2 | 0.8991 | 0.8355-0.9541 |
| CCL23_DNAI7_GALM_GINS4_CAV1_PTGDR2 | 0.8987 | 0.8359-0.9502 |
| CCL23_PRR11_GALM_GINS4_CAV1_PTGDR2 | 0.9017 | 0.8389-0.9489 |
| CCL23_PRR11_DNAI7_GINS4_CAV1_PTGDR2 | 0.8983 | 0.8324-0.9528 |
| CCL23_PRR11_DNAI7_GALM_CAV1_PTGDR2 | 0.9052 | 0.8441-0.9567 |
| PRR11_DNAI7_GALM_GINS4_TXNDC11_PTGDR2 | 0.8861 | 0.81-0.945 |
| CCL23_DNAI7_GALM_GINS4_TXNDC11_PTGDR2 | 0.8593 | 0.7714-0.9303 |
| CCL23_PRR11_GALM_GINS4_TXNDC11_PTGDR2 | 0.8758 | 0.7991-0.9364 |
| CCL23_PRR11_DNAI7_GINS4_TXNDC11_PTGDR2 | 0.8753 | 0.8026-0.9377 |
| CCL23_PRR11_DNAI7_GALM_TXNDC11_PTGDR2 | 0.8779 | 0.8108-0.9368 |
| CCL23_PRR11_DNAI7_GALM_GINS4_PTGDR2 | 0.8831 | 0.8108-0.9476 |
| DNAI7_GALM_GINS4_TXNDC11_CAV1_DIO3 | 0.9017 | 0.8351-0.9537 |
| PRR11_GALM_GINS4_TXNDC11_CAV1_DIO3 | 0.9177 | 0.8602-0.9619 |
| CCL23_GALM_GINS4_TXNDC11_CAV1_DIO3 | 0.9212 | 0.8623-0.9697 |
| PRR11_DNAI7_GINS4_TXNDC11_CAV1_DIO3 | 0.9165 | 0.8615-0.9619 |
| CCL23_DNAI7_GINS4_TXNDC11_CAV1_DIO3 | 0.9186 | 0.8602-0.9697 |
| CCL23_PRR11_GINS4_TXNDC11_CAV1_DIO3 | 0.926 | 0.8714-0.9701 |
| PRR11_DNAI7_GALM_TXNDC11_CAV1_DIO3 | 0.9156 | 0.855-0.9598 |
| CCL23_DNAI7_GALM_TXNDC11_CAV1_DIO3 | 0.9139 | 0.8589-0.9589 |
| CCL23_PRR11_GALM_TXNDC11_CAV1_DIO3 | 0.9307 | 0.8745-0.9701 |
| CCL23_PRR11_DNAI7_TXNDC11_CAV1_DIO3 | 0.9329 | 0.8801-0.971 |
| PRR11_DNAI7_GALM_GINS4_CAV1_DIO3 | 0.9238 | 0.8693-0.9675 |
| CCL23_DNAI7_GALM_GINS4_CAV1_DIO3 | 0.9299 | 0.8731-0.9714 |
| CCL23_PRR11_GALM_GINS4_CAV1_DIO3 | 0.9377 | 0.8909-0.9745 |
| CCL23_PRR11_DNAI7_GINS4_CAV1_DIO3 | 0.9385 | 0.8883-0.974 |
| CCL23_PRR11_DNAI7_GALM_CAV1_DIO3 | 0.9394 | 0.8943-0.9753 |
| PRR11_DNAI7_GALM_GINS4_TXNDC11_DIO3 | 0.9052 | 0.8403-0.9545 |
| CCL23_DNAI7_GALM_GINS4_TXNDC11_DIO3 | 0.8931 | 0.8247-0.9459 |
| CCL23_PRR11_GALM_GINS4_TXNDC11_DIO3 | 0.9203 | 0.8645-0.9632 |
| CCL23_PRR11_DNAI7_GINS4_TXNDC11_DIO3 | 0.9242 | 0.8688-0.9645 |
| CCL23_PRR11_DNAI7_GALM_TXNDC11_DIO3 | 0.9169 | 0.8597-0.9598 |
| CCL23_PRR11_DNAI7_GALM_GINS4_DIO3 | 0.9286 | 0.8796-0.9684 |
| PRR11_DNAI7_GALM_GINS4_TXNDC11_CAV1 | 0.8957 | 0.8307-0.9515 |
| CCL23_DNAI7_GALM_GINS4_TXNDC11_CAV1 | 0.9095 | 0.8476-0.9563 |
| CCL23_PRR11_GALM_GINS4_TXNDC11_CAV1 | 0.9061 | 0.8476-0.9546 |
| CCL23_PRR11_DNAI7_GINS4_TXNDC11_CAV1 | 0.9078 | 0.8446-0.955 |
| CCL23_PRR11_DNAI7_GALM_TXNDC11_CAV1 | 0.9147 | 0.8519-0.9589 |
| CCL23_PRR11_DNAI7_GALM_GINS4_CAV1 | 0.9156 | 0.8619-0.9606 |
| CCL23_PRR11_DNAI7_GALM_GINS4_TXNDC11 | 0.8983 | 0.8281-0.955 |
| GINS4_TXNDC11_MAPRE3_DIO3_GPR107_CADM1 | 0.9022 | 0.8251-0.9602 |
| GALM_TXNDC11_MAPRE3_DIO3_GPR107_CADM1 | 0.9139 | 0.8576-0.9623 |
| DNAI7_TXNDC11_MAPRE3_DIO3_GPR107_CADM1 | 0.8961 | 0.8125-0.9541 |
| PRR11_TXNDC11_MAPRE3_DIO3_GPR107_CADM1 | 0.9026 | 0.8337-0.9589 |
| CCL23_TXNDC11_MAPRE3_DIO3_GPR107_CADM1 | 0.8861 | 0.8052-0.9532 |
| GALM_GINS4_MAPRE3_DIO3_GPR107_CADM1 | 0.9048 | 0.8407-0.9511 |
| DNAI7_GINS4_MAPRE3_DIO3_GPR107_CADM1 | 0.8948 | 0.8298-0.9498 |
| PRR11_GINS4_MAPRE3_DIO3_GPR107_CADM1 | 0.9061 | 0.8519-0.9554 |
| CCL23_GINS4_MAPRE3_DIO3_GPR107_CADM1 | 0.8948 | 0.8203-0.952 |
| DNAI7_GALM_MAPRE3_DIO3_GPR107_CADM1 | 0.8823 | 0.8086-0.9407 |
| PRR11_GALM_MAPRE3_DIO3_GPR107_CADM1 | 0.9013 | 0.8398-0.952 |
| CCL23_GALM_MAPRE3_DIO3_GPR107_CADM1 | 0.8905 | 0.8251-0.9437 |
| PRR11_DNAI7_MAPRE3_DIO3_GPR107_CADM1 | 0.8926 | 0.8281-0.9472 |
| CCL23_DNAI7_MAPRE3_DIO3_GPR107_CADM1 | 0.8749 | 0.7961-0.9372 |
| CCL23_PRR11_MAPRE3_DIO3_GPR107_CADM1 | 0.8918 | 0.8195-0.9498 |
| GALM_GINS4_TXNDC11_DIO3_GPR107_CADM1 | 0.9173 | 0.8541-0.9615 |
| DNAI7_GINS4_TXNDC11_DIO3_GPR107CADM1 | 0.903 | 0.8286-0.961 |
| PRR11_GINS4_TXNDC11_DIO3_GPR107_CADM1 | 0.9061 | 0.8429-0.9602 |
| CCL23_GINS4_TXNDC11_DIO3_GPR107_CADM1 | 0.8961 | 0.8156-0.9563 |
| DNAI7_GALM_TXNDC11_DIO3_GPR107_CADM1 | 0.9065 | 0.8424-0.9567 |
| PRR11_GALM_TXNDC11_DIO3_GPR107_CADM1 | 0.9134 | 0.8584-0.9641 |
| CCL23_GALM_TXNDC11_DIO3_GPR107_CADM1 | 0.9074 | 0.8459-0.958 |
| PRR11_DNAI7_TXNDC11_DIO3_GPR107_CADM1 | 0.9017 | 0.8346-0.9602 |
| CCL23_DNAI7_TXNDC11_DIO3_GPR107_CADM1 | 0.8909 | 0.8086-0.955 |
| CCL23_PRR11_TXNDC11_DIO3_GPR107_CADM1 | 0.8944 | 0.8169-0.9554 |
| DNAI7_GALM_GINS4_DIO3_GPR107_CADM1 | 0.8983 | 0.8394-0.9485 |
| PRR11_GALM_GINS4_DIO3_GPR107_CADM1 | 0.9117 | 0.8597-0.9567 |
| CCL23_GALM_GINS4_DIO3_GPR107_CADM1 | 0.9087 | 0.8485-0.9576 |
| PRR11_DNAI7_GINS4_DIO3_GPR107_CADM1 | 0.9069 | 0.8489-0.9546 |
| CCL23_DNAI7_GINS4_DIO3_GPR107_CADM1 | 0.8957 | 0.8272-0.9485 |
| CCL23_PRR11_GINS4_DIO3_GPR107_CADM1 | 0.9035 | 0.8485-0.9524 |
| PRR11_DNAI7_GALM_DIO3_GPR107_CADM1 | 0.8978 | 0.8315-0.9502 |
| CCL23_DNAI7_GALM_DIO3_GPR107_CADM1 | 0.8892 | 0.822-0.9398 |
| CCL23_PRR11_GALM_DIO3_GPR107_CADM1 | 0.9035 | 0.8437-0.9519 |
| CCL23_PRR11_DNAI7_DIO3_GPR107_CADM1 | 0.8965 | 0.8307-0.9494 |
| GALM_GINS4_TXNDC11_MAPRE3_GPR107_CADM1 | 0.9048 | 0.8463-0.9563 |
| DNAI7_GINS4_TXNDC11_MAPRE3_GPR107_CADM1 | 0.8827 | 0.8052-0.9476 |
| PRR11_GINS4_TXNDC11_MAPRE3_GPR107 _CADM1 | 0.8892 | 0.8173-0.9481 |
| CCL23_GINS4_TXNDC11_MAPRE3_GPR107_CADM1 | 0.8771 | 0.8017-0.9502 |
| DNAI7_GALM_TXNDC11_MAPRE3_GPR107_CADM1 | 0.8939 | 0.826-0.945 |
| PRR11_GALM_TXNDC11_MAPRE3_GPR107_CADM1 | 0.9065 | 0.845-0.9593 |
| CCL23_GALM_TXNDC11_MAPRE3_GPR107_CADM1 | 0.9074 | 0.8428-0.9576 |
| PRR11_DNAI7_TXNDC11_MAPRE3_GPR107_CADM1 | 0.884 | 0.8151-0.9433 |
| CCL23_DNAI7_TXNDC11_MAPRE3_GPR107_CADM1 | 0.8762 | 0.7918-0.9494 |
| CCL23_PRR11_TXNDC11_MAPRE3_GPR107_CADM1 | 0.884 | 0.803-0.9459 |
| DNAI7_GALM_GINS4_MAPRE3_GPR107_CADM1 | 0.8749 | 0.8065-0.9312 |
| PRR11_GALM_GINS4_MAPRE3_GPR107_CADM1 | 0.8939 | 0.8355-0.9468 |
| CCL23_GALM_GINS4_MAPRE3_GPR107_CADM1 | 0.8996 | 0.8359-0.9515 |
| PRR11_DNAI7_GINS4_MAPRE3_GPR107_CADM1 | 0.8844 | 0.8182-0.945 |
| CCL23_DNAI7_GINS4_MAPRE3_GPR107_CADM1 | 0.8788 | 0.8078-0.939 |
| CCL23_PRR11_GINS4_MAPRE3_GPR107_CADM1 | 0.8887 | 0.8177-0.945 |
| PRR11_DNAI7_GALM_MAPRE3_GPR107_CADM1 | 0.8797 | 0.813-0.9364 |
| CCL23_DNAI7_GALM_MAPRE3_GPR107_CADM1 | 0.8792 | 0.8104-0.9359 |
| CCL23_PRR11_GALM_MAPRE3_GPR107_CADM1 | 0.8996 | 0.8428-0.952 |
| CCL23_PRR11_DNAI7_MAPRE3_GPR107_CADM1 | 0.8861 | 0.8151-0.9455 |
| DNAI7_GALM_GINS4_TXNDC11_GPR107_CADM1 | 0.8909 | 0.8268-0.9476 |
| PRR11_GALM_GINS4_TXNDC11_GPR107_CADM1 | 0.9022 | 0.8467-0.9584 |
| CCL23_GALM_GINS4_TXNDC11_GPR107_CADM1 | 0.9056 | 0.8428-0.9541 |
| PRR11_DNAI7_GINS4_TXNDC11_GPR107_CADM1 | 0.8831 | 0.8112-0.945 |
| CCL23_DNAI7_GINS4_TXNDC11_GPR107_CADM1 | 0.8823 | 0.806-0.9481 |
| CCL23_PRR11_GINS4_TXNDC11_GPR107_CADM1 | 0.8866 | 0.8151-0.9481 |
| PRR11_DNAI7_GALM_TXNDC11_GPR107_CADM1 | 0.8931 | 0.8299-0.9468 |
| CCL23_DNAI7_GALM_TXNDC11_GPR107_CADM1 | 0.8978 | 0.8363-0.9494 |
| CCL23_PRR11_GALM_TXNDC11_GPR107_CADM1 | 0.9052 | 0.8481-0.9558 |
| CCL23_PRR11_DNAI7_TXNDC11_GPR107_CADM1 | 0.8861 | 0.8061-0.9485 |
| PRR11_DNAI7_GALM_GINS4_GPR107_CADM1 | 0.8831 | 0.8152-0.9411 |
| CCL23_DNAI7_GALM_GINS4_GPR107_CADM1 | 0.8861 | 0.8195-0.9429 |
| CCL23_PRR11_GALM_GINS4_GPR107_CADM1 | 0.8996 | 0.839-0.952 |
| CCL23_PRR11_DNAI7_GINS4_GPR107_CADM1 | 0.8931 | 0.8221-0.9468 |
| CCL23_PRR11_DNAI7_GALM_GPR107_CADM1 | 0.887 | 0.8203-0.9394 |
| GALM_GINS4 _TXNDC11_MAPRE3_DIO3_CADM1 | 0.9186 | 0.8631-0.9641 |
| DNAI7_GINS4_TXNDC11_MAPRE3_DIO3_CADM1 | 0.8987 | 0.8285-0.955 |
| PRR11_GINS4_TXNDC11_MAPRE3 DIO3 CADM1 | 0.9095 | 0.848-0.9598 |
| CCL23_GINS4_TXNDC11_MAPRE3_DIO3_CADM1 | 0.9009 | 0.8329-0.9593 |
| DNAI7_GALM_TXNDC11_MAPRE3_DIO3_CADM1 | 0.9074 | 0.8502-0.9563 |
| PRR11_GALM_TXNDC11_MAPRE3_DIO3_CADM1 | 0.9169 | 0.8593-0.9615 |
| CCL23_GALM_TXNDC11_MAPRE3_DIO3_CADM1 | 0.9126 | 0.8532-0.9619 |
| PRR11_DNAI7_TXNDC11_MAPRE3_DIO3_CADM1 | 0.9022 | 0.8333-0.9571 |
| CCL23_DNAI7_TXNDC11_MAPRE3_DIO3_CADM1 | 0.8948 | 0.8156-0.9602 |
| CCL23_PRR11_TXNDC11_MAPRE3_DIO3_CADM1 | 0.8983 | 0.829-0.9559 |
| DNAI7_GALM_GINS4_MAPRE3_DIO3_CADM1 | 0.8874 | 0.8212-0.9429 |
| PRR11_GALM_GINS4_MAPRE3_DIO3_CADM1 | 0.9091 | 0.8459-0.9563 |
| CCL23_GALM_GINS4_MAPRE3_DIO3_CADM1 | 0.9009 | 0.839-0.952 |
| PRR11_DNAI7_GINS4_MAPRE3_DIO3_CADM1 | 0.9004 | 0.8346-0.9546 |
| CCL23_DNAI7_GINS4_MAPRE3_DIO3_CADM1 | 0.8879 | 0.8207-0.945 |
| CCL23_PRR11_GINS4_MAPRE3_DIO3_CADM1 | 0.903 | 0.845-0.9524 |
| PRR11_DNAI7_GALM_MAPRE3_DIO3_CADM1 | 0.89 | 0.8272-0.9472 |
| CCL23_DNAI7_GALM_MAPRE3_DIO3_CADM1 | 0.884 | 0.8186-0.9381 |
| CCL23_PRR11_GALM_MAPRE3_DIO3_CADM1 | 0.8952 | 0.8354-0.9472 |
| CCL23_PRR11_DNAI7_MAPRE3 _DIO3_CADM1 | 0.8896 | 0.8255-0.9424 |
| DNAI7_GALM_GINS4_TXNDC11_DIO3_CADM1 | 0.9069 | 0.8472-0.9589 |
| PRR11_GALM_GINS4_TXNDC11_DIO3_CADM1 | 0.9208 | 0.8649-0.9667 |
| PRR11_DNAI7_GINS4_TXNDC11_DIO3_CADM1 | 0.9104 | 0.8403-0.9606 |
| CCL23_DNAI7_GINS4_TXNDC11_DIO3_CADM1 | 0.9009 | 0.822-0.9576 |
| CCL23_PRR11_GINS4_TXNDC11_DIO3_CADM1 | 0.9069 | 0.8376-0.9641 |
| PRR11_DNAI7_GALM_TXNDC11_DIO3_CADM1 | 0.9169 | 0.8601-0.9645 |
| CCL23_DNAI7_GALM_TXNDC11_DIO3_CADM1 | 0.913 | 0.8589-0.961 |
| CCL23_PRR11_GALM_TXNDC11_DIO3_CADM1 | 0.9139 | 0.8506-0.958 |
| CCL23_PRR11_DNAI7_TXNDC11_DIO3_CADM1 | 0.9043 | 0.8311-0.9593 |
| PRR11_DNAI7_GALM_GINS4_DIO3_CADM1 | 0.9039 | 0.8411-0.9533 |
| CCL23_DNAI7_GALM_GINS4_DIO3_CADM1 | 0.8978 | 0.8325-0.9502 |
| CCL23_PRR11 GALM_GINS4_DIO3_CADM1 | 0.9143 | 0.8567-0.9632 |
| CCL23_PRR11_DNAI7_GINS4_DIO3_CADM1 | 0.9087 | 0.8528-0.9585 |
| DNAI7_GALM_GINS4_TXNDC11_MAPRE3_CADM1 | 0.8935 | 0.8312-0.9515 |
| PRR11_GALM_GINS4_TXNDC11_MAPRE3_CADM1 | 0.903 | 0.845-0.952 |
| CCL23_GALM_GINS4_TXNDC11_MAPRE3_CADM1 | 0.9022 | 0.8415-0.9507 |
| PRR11_DNAI7_GINS4_TXNDC11_MAPRE3_CADM1 | 0.8835 | 0.8095-0.9429 |
| CCL23_DNAI7_GINS4_TXNDC11_MAPRE3_CADM1 | 0.8736 | 0.7965-0.942 |
| CCL23_PRR11_GINS4_TXNDC11_MAPRE3_CADM1 | 0.8805 | 0.8121-0.9411 |
| PRR11_GINS4_TXNDC11_MAPRE3_CADM1 | 0.8723 | 0.8048-0.9281 |
| PRR11_DNAI7_GALM_TXNDC11_MAPRE3_CADM1 | 0.8952 | 0.829-0.9485 |
| CCL23_DNAI7_GALM_TXNDC11_MAPRE3_CADM1 | 0.8991 | 0.8312-0.9507 |
| CCL23_PRR11_GALM_TXNDC11_MAPRE3_CADM1 | 0.9087 | 0.8494-0.958 |
| PRR11_DNAI7_GALM_GINS4_MAPRE3_CADM1 | 0.8749 | 0.8095-0.9333 |
| CCL23_DNAI7_GALM_GINS4_MAPRE3_CADM1 | 0.8753 | 0.8069-0.9338 |
| CCL23_PRR11_GALM_GINS4_MAPRE3_CADM1 | 0.8961 | 0.826-0.9498 |
| CCL23_PRR11_DNAI7_GINS4_MAPRE3_CADM1 | 0.8835 | 0.8086-0.9429 |
| CCL23_PRR11_DNAI7_GALM_MAPRE3_CADM1 | 0.8749 | 0.8022-0.9316 |
| CCL23_PRR11_DNAI7_GALM_GINS4_TXNDC11_CADM1 | 0.9061 | 0.8485-0.9576 |
| PRR11_DNAI7_GALM_GINS4_TXNDC11_CADM1 | 0.8974 | 0.835-0.9498 |
| CCL23_PRR11_GALM_GINS4_TXNDC11_CADM1 | 0.9035 | 0.8476-0.9558 |
| CCL23_PRR11_DNAI7_GINS4_TXNDC11_CADM1 | 0.8853 | 0.822-0.9507 |
| GALM_GINS4_TXNDC11_MAPRE3_DIO3_GPR107 | 0.9173 | 0.8645-0.9619 |
| DNAI7_GINS4_TXNDC11_MAPRE3_DIO3_GPR107 | 0.903 | 0.8429-0.9589 |
| PRR11_GINS4_TXNDC11_MAPRE3_DIO3_GPR107 | 0.9126 | 0.8506-0.9645 |
| CCL23_GINS4_TXNDC11_MAPRE3_DIO3_GPR107 | 0.9017 | 0.8303-0.9585 |
| DNAI7_GALM_TXNDC11_MAPRE3_DIO3_GPR107 | 0.9043 | 0.8415-0.9537 |
| PRR11_GALM_TXNDC11_MAPRE3_DIO3_GPR107 | 0.9182 | 0.8636-0.9615 |
| CCL23_GALM_TXNDC11_MAPRE3_DIO3_GPR107 | 0.9134 | 0.8558-0.9623 |
| PRR11_DNAI7_TXNDC11_MAPRE3_DIO3_GPR107 | 0.9061 | 0.8407-0.9615 |
| CCL23_DNAI7_TXNDC11_MAPRE3_DIO3_GPR107 | 0.8948 | 0.8143-0.9567 |
| CCL23_PRR11_TXNDC11_MAPRE3_DIO3_GPR107 | 0.903 | 0.8381-0.9598 |
| DNAI7_GALM_GINS4_MAPRE3_DIO3_GPR107 | 0.8896 | 0.8242-0.9429 |
| PRR11_GALM_GINS4 _MAPRE3_DIO3_GPR107 | 0.9074 | 0.8419-0.9559 |
| CCL23_GALM_GINS4_MAPRE3_DIO3_GPR107 | 0.8996 | 0.8381-0.9502 |
| PRR11_DNAI7_GINS4_MAPRE3_DIO3_GPR107 | 0.8978 | 0.8359-0.9498 |
| CCL23_DNAI7_GINS4_MAPRE3_DIO3_GPR107 | 0.8883 | 0.8203-0.9437 |
| CCL23_PRR11_GINS4_MAPRE3_DIO3_GPR107 | 0.9039 | 0.8467-0.9554 |
| PRR11_DNAI7_GALM_MAPRE3_DIO3_GPR107 | 0.8861 | 0.8212-0.9411 |
| CCL23_DNAI7_GALM_MAPRE3_DIO3_GPR107 | 0.8762 | 0.8091-0.9316 |
| CCL23_PRR11_GALM_MAPRE3_DIO3_GPR107 | 0.8952 | 0.8355-0.9476 |
| CCL23_PRR11_DNAI7 MAPRE3_DIO3_GPR107 | 0.8879 | 0.8216-0.9424 |
| DNAI7_GALM_GINS4_TXNDC11_DIO3_GPR107 | 0.9143 | 0.8589-0.9615 |
| CCL23_PRR11_GALM_GINS4_TXNDC11_DIO3_GPR107 | 0.929 | 0.8788-0.9688 |
| PRR11_GALM_GINS4_TXNDC11_DIO3_GPR107 | 0.9229 | 0.8658-0.9671 |
| CCL23_GALM_GINS4_TXNDC11_DIO3_GPR107 | 0.9199 | 0.8606-0.9658 |
| PRR11_DNAI7_GINS4_TXNDC11_DIO3_GPR107 | 0.9091 | 0.8407-0.9571 |
| CCL23_DNAI7_GINS4_TXNDC11_DIO3_GPR107 | 0.9039 | 0.8285-0.958 |
| CCL23_PRR11_GINS4_TXNDC11_DIO3_GPR107 | 0.9078 | 0.8428-0.9641 |
| PRR11_DNAI7_GALM_TXNDC11_DIO3_GPR107 | 0.9074 | 0.8502-0.9528 |
| CCL23_DNAI7_GALM_TXNDC11_DIO3_GPR107 | 0.9048 | 0.8441-0.9502 |
| CCL23_PRR11_GALM_TXNDC11_DIO3_GPR107 | 0.9182 | 0.8688-0.9632 |
| CCL23_PRR11_DNAI7_TXNDC11_DIO3_GPR107 | 0.9035 | 0.8325-0.9563 |
| PRR11_DNAI7_GALM_GINS4_DIO3_GPR107 | 0.9022 | 0.8381-0.9524 |
| CCL23_DNAI7_GALM_GINS4_DIO3_GPR107 | 0.8935 | 0.8338-0.9485 |
| CCL23_PRR11_GALM_GINS4_DIO3_GPR107 | 0.9104 | 0.8524-0.9584 |
| CCL23_PRR11_DNAI7_GINS4_DIO3_GPR107 | 0.9043 | 0.8428-0.9541 |
| CCL23_PRR11_DNAI7_GALM_DIO3_GPR107 | 0.8948 | 0.8333-0.945 |
| DNAI7_GALM_GINS4_TXNDC11_MAPRE3_GPR107 | 0.8996 | 0.8368-0.952 |
| PRR11_GALM_GINS4_TXNDC11_MAPRE3_GPR107 | 0.913 | 0.8532-0.9606 |
| CCL23_GALM_GINS4_TXNDC11_MAPRE3_GPR107 | 0.913 | 0.8536-0.9589 |
| PRR11_DNAI7_GINS4_TXNDC11_MAPRE3_GPR107 | 0.8913 | 0.8255-0.9498 |
| CCL23_DNAI7_GINS4_TXNDC11_MAPRE3_GPR107 | 0.8866 | 0.8047-0.9472 |
| CCL23_PRR11_GINS4_TXNDC11_MAPRE3_GPR107 | 0.8965 | 0.8307-0.9571 |
| PRR11_DNAI7_GALM_TXNDC11_MAPRE3_GPR107 | 0.9013 | 0.842-0.9498 |
| CCL23_DNAI7_GALM_TXNDC11_MAPRE3_GPR107 | 0.903 | 0.8463-0.9533 |
| CCL23_PRR11_GALM_TXNDC11_MAPRE3_GPR107 | 0.919 | 0.8667-0.9645 |
| CCL23_PRR11_DNAI7_TXNDC11_MAPRE3_GPR107 | 0.8926 | 0.8273-0.9528 |
| PRR11_DNAI7_GALM_GINS4_MAPRE3_GPR107 | 0.881 | 0.8147-0.9407 |
| CCL23_DNAI7_GALM_GINS4_MAPRE3_GPR107 | 0.8788 | 0.8073-0.9416 |
| CCL23_PRR11_GALM_GINS4_MAPRE3_GPR107 | 0.8996 | 0.8377-0.9459 |
| CCL23_PRR11_DNAI7_GINS4_MAPRE3_GPR107 | 0.89 | 0.8225-0.9455 |
| CCL23_PRR11_DNAI7_GALM_MAPRE3_GPR107 | 0.8853 | 0.8207-0.9364 |
| PRR11_DNAI7_GALM_GINS4_TXNDC11_GPR107 | 0.8939 | 0.8294-0.9468 |
| CCL23_DNAI7_GALM_GINS4_TXNDC11_GPR107 | 0.9009 | 0.8359-0.9541 |
| CCL23_PRR11_GALM_GINS4_TXNDC11_GPR107 | 0.9126 | 0.8554-0.9593 |
| CCL23_PRR11_DNAI7_GINS4_TXNDC11_GPR107 | 0.8922 | 0.8225-0.9533 |
| CCL23_PRR11_DNAI7_GALM_TXNDC11_GPR107 | 0.9009 | 0.8424-0.9502 |
| DNAI7_GALM_GINS4_TXNDC11_MAPRE3_DIO3 | 0.9104 | 0.8498-0.9602 |
| PRR11_GALM_GINS4_TXNDC11_MAPRE3_DIO3 | 0.9277 | 0.8792-0.968 |
| CCL23_GALM_GINS4_TXNDC11_MAPRE3_DIO3 | 0.9203 | 0.864-0.9662 |
| PRR11_DNAI7_GINS4_TXNDC11_MAPRE3_DIO3 | 0.9069 | 0.8432-0.955 |
| CCL23_DNAI7_GINS4_TXNDC11_MAPRE3_DIO3 | 0.9043 | 0.8407-0.9576 |
| CCL23_PRR11_GINS4_TXNDC11_MAPRE3_DIO3 | 0.9126 | 0.8489-0.9649 |
| PRR11_DNAI7_GALM_TXNDC11_MAPRE3_DIO3 | 0.9165 | 0.8575-0.9654 |
| CCL23_DNAI7_GALM_TXNDC11_MAPRE3_DIO3 | 0.9078 | 0.8455-0.9533 |
| CCL23_PRR11_GALM_TXNDC11_MAPRE3_DIO3 | 0.9203 | 0.8628-0.9632 |
| CCL23_PRR11_DNAI7_TXNDC11_MAPRE3_DIO3 | 0.9069 | 0.842-0.9593 |
| CCL23_PRR11_DNAI7_GALM_GINS4_MAPRE3_DIO3 | 0.9091 | 0.848-0.9598 |
| PRR11_DNAI7_GALM_GINS4_MAPRE3_DIO3 | 0.8944 | 0.829-0.945 |
| CCL23_DNAI7_GALM_GINS4_MAPRE3_DIO3 | 0.8853 | 0.8203-0.9429 |
| CCL23_PRR11_GALM_GINS4_MAPRE3_DIO3 | 0.9087 | 0.8459-0.9571 |
| PRR11_DNAI7_GALM_GINS4_TXNDC11_DIO3 | 0.9173 | 0.8628-0.9615 |
| CCL23_DNAI7_GALM_GINS4_TXNDC11_DIO3 | 0.9117 | 0.8485-0.9611 |
| CCL23_PRR11_GALM_GINS4_TXNDC11_DIO3 | 0.9277 | 0.8783-0.9684 |
| CCL23_PRR11_DNAI7_GINS4_TXNDC11_DIO3 | 0.9156 | 0.8502-0.9645 |
| CCL23_PRR11_DNAI7_GALM_TXNDC11_DIO3 | 0.9199 | 0.8619-0.9649 |
| CCL23_PRR11_DNAI7_GALM_GINS4_DIO3_ | 0.903 | 0.8351-0.955 |
| PRR11_DNAI7_GALM_GINS4_TXNDC11_MAPRE3 | 0.8991 | 0.8359-0.952 |
| CCL23_DNAI7_GALM_GINS4_TXNDC11_MAPRE3 | 0.9013 | 0.8394-0.955 |
| CCL23_PRR11_GALM_GINS4_TXNDC11_MAPRE3 | 0.9143 | 0.8545-0.9641 |
| CCL23_PRR11_DNAI7_GINS4_TXNDC11_MAPRE3 | 0.8896 | 0.8199-0.9528 |
| CCL23_PRR11_DNAI7_GALM_TXNDC11_MAPRE | 0.9048 | 0.8459-0.9532 |
| CCL23_PRR11_DNAI7_GALM_GINS4_MAPRE3 | 0.8779 | 0.8077-0.9372 |
| CCL23_PRR11_DNAI7_GALM_GINS4_TXNDC11 | 0.9004 | 0.8398-0.9545 |

| **Combinations of seven biomarkers** | | |
|---|---|---|
| CCL23_GALM_TXNDC11_ELL2_DIO3_UAP1_CDCA2 | 0.9281 | 0.8688-0.9732 |
| PRR11_GAS6_GALM_TXNDC11_CAV1_DIO3_CADM1 | 0.926 | 0.8727-0.9688 |
| CCL23_GAS6_GALM_TXNDC11_CAV1_DIO3_CADM1 | 0.9277 | 0.8701-0.9753 |
| CCL23_PRR11_GALM_TXNDC11_CAV1_DIO3_CADM1 | 0.939 | 0.8874-0.9792 |
| CCL23_PRR11_GAS6_TXNDC11_CAV1_DIO3_CADM1 | 0.9113 | 0.8494-0.9611 |
| CCL23_PRR11_DNAI7_GINS4_TXNDC11_DIO3_PTGDR2 | 0.9212 | 0.8636-0.968 |
| CCL23_PRR11_GAS6_GALM_CAV1_DIO3_CADM1 | 0.9338 | 0.8818-0.9771 |
| CCL23_PRR11_GAS6_GALM_TXNDC11_DIO3_CADM1 | 0.9398 | 0.8827-0.9827 |
| CCL23_PRR11_GAS6_GALM_TXNDC11_CAV1_CADM1 | 0.9026 | 0.8424-0.9524 |
| CCL23_PRR11_GAS6_GALM_TXNDC11_CAV1_DIO3 | 0.9351 | 0.8805-0.9753 |
| DNAI7_GALM_GINS4_TXNDC11_CAV1_DIO3_PTGDR2 | 0.916 | 0.8589-0.9645 |
| PRR11_GALM_GINS4_TXNDC11_CAV1_DIO3_PTGDR2 | 0.9255 | 0.8727-0.9662 |
| CCL23_GALM_GINS4_TXNDC11_CAV1_DIO3_PTGDR2 | 0.913 | 0.8485-0.9645 |
| PRR11_DNAI7_GINS4_TXNDC11_CAV1_DIO3_PTGDR2 | 0.9316 | 0.884-0.9719 |
| CCL23_DNAI7_GINS4_TXNDC11_CAV1_DIO3_PTGDR2 | 0.9195 | 0.8489-0.9723 |
| CCL23_PRR11_GINS4_TXNDC11_CAV1_DIO3_PTGDR2 | 0.9277 | 0.8719-0.9715 |
| PRR11_DNAI7_GALM_TXNDC11_CAV1_DIO3_PTGDR2 | 0.9255 | 0.8727-0.9662 |
| CCL23_DNAI7_GALM_TXNDC11_CAV1_DIO3_PTGDR2 | 0.9108 | 0.8484-0.9615 |
| CCL23_PRR11_GALM_TXNDC11_CAV1_DIO3_PTGDR2 | 0.9281 | 0.8744-0.9719 |
| CCL23_PRR11_DNAI7_TXNDC11_CAV1_DIO3_PTGDR2 | 0.9325 | 0.8783-0.9766 |
| PRR11_DNAI7_GALM_GINS4_CAV1_DIO3_PTGDR2 | 0.9364 | 0.887-0.974 |
| CCL23_DNAI7_GALM_GINS4_CAV1_DIO3_PTGDR2 | 0.9299 | 0.8697-0.974 |
| CCL23_PRR11_GALM_GINS4_CAV1_DIO3_PTGDR2 | 0.9364 | 0.8887-0.9745 |
| CCL23_PRR11_DNAI7_GINS4_CAV1_DIO3_PTGDR2 | 0.9372 | 0.8831-0.9779 |
| CCL23_PRR11_DNAI7_GALM_CAV1_DIO3_PTGDR2 | 0.9385 | 0.8935-0.9766 |
| PRR11_DNAI7_GALM_GINS4_TXNDC11_DIO3_PTGDR2 | 0.9208 | 0.8688-0.9649 |
| CCL23_DNAI7_GALM_GINS4_TXNDC11_DIO3_PTGDR2 | 0.887 | 0.8121-0.9472 |
| CCL23_PRR11_GALM_GINS4_TXNDC11_DIO3_PTGDR2 | 0.919 | 0.8554-0.9472 |
| CCL23_PRR11_DNAI7_GALM_TXNDC11_DIO3_PTGDR2 | 0.9143 | 0.8558-0.9593 |
| CCL23_PRR11_DNAI7_GALM_GINS4_DIO3_PTGDR2 | 0.9255 | 0.8697-0.968 |
| PRR11_DNAI7_GALM_GINS4_TXNDC11_CAV1_PTGDR2 | 0.897 | 0.8377-0.9511 |
| CCL23_DNAI7_GALM_GINS4_TXNDC11_CAV1_PTGDR2 | 0.8991 | 0.8363-0.9494 |
| CCL23_PRR11_GALM_GINS4_TXNDC11_CAV1_PTGDR2 | 0.9035 | 0.8428-0.9524 |
| CCL23_PRR11_DNAI7_GINS4_TXNDC11_CAV1_PTGDR2 | 0.8991 | 0.8325-0.9502 |
| CCL23_PRR11_DNAI7_GALM_TXNDC11_CAV1_PTGDR2 | 0.9056 | 0.8472-0.9563 |
| CCL23_PRR11_DNAI7_GALM_GINS4_CAV1_PTGDR2 | 0.9104 | 0.8528-0.9597 |
| CCL23_PRR11_DNAI7_GALM_GINS4_TXNDC11_PTGDR2 | 0.8844 | 0.8025-0.9438 |
| PRR11_DNAI7_GALM_GINS4_TXNDC11_CAV1_DIO3 | 0.926 | 0.8723-0.9663 |
| CCL23_DNAI7_GALM_GINS4_TXNDC11_CAV1_DIO3 | 0.9312 | 0.8762-0.9727 |
| CCL23_PRR11_GALM_GINS4_TXNDC11_CAV1_DIO3 | 0.9372 | 0.8896-0.974 |
| CCL23_PRR11_DNAI7_GINS4_TXNDC11_CAV1_DIO3 | 0.9394 | 0.8913-0.9779 |
| CCL23_PRR11_DNAI7_GALM_TXNDC11_CAV1_DIO3 | 0.9398 | 0.8952-0.9749 |
| CCL23_PRR11_DNAI7_GALM_GINS4_CAV1_DIO3 | 0.9455 | 0.903-0.978 |
| CCL23_PRR11_DNAI7_GALM_GINS4_TXNDC11_DIO3 | 0.9312 | 0.8766-0.9719 |
| CCL23_PRR11_DNAI7_GALM_GINS4_TXNDC11_CAV1 | 0.9156 | 0.8597-0.9606 |
| GALM_GINS4_TXNDC11_MAPRE3_DIO3_GPR107_CADM1 | 0.9255 | 0.8719-0.9688 |
| DNAI7_GINS4_TXNDC11_MAPRE3_DIO3_GPR107_CADM1 | 0.91 | 0.8428-0.9632 |
| PRR11_GINS4_TXNDC11_MAPRE3_DIO3_GPR107_CADM1 | 0.9169 | 0.8502-0.9632 |
| CCL23_GINS4_TXNDC11_MAPRE3_DIO3_GPR107_CADM1 | 0.9095 | 0.8424-0.9662 |
| DNAI7_GALM_TXNDC11_MAPRE3_DIO3_GPR107_CADM1 | 0.9195 | 0.861-0.9628 |
| PRR11_GALM_TXNDC11_MAPRE3_DIO3_GPR107_CADM1 | 0.9255 | 0.8731-0.9693 |
| CCL23_GALM_TXNDC11_MAPRE3_DIO3_GPR107_CADM1 | 0.9199 | 0.8632-0.9636 |
| PRR11_DNAI7_TXNDC11_MAPRE3_DIO3_GPR107_CADM1 | 0.9169 | 0.8489-0.9654 |
| CCL23_DNAI7_TXNDC11_MAPRE3_DIO3_GPR107_CADM1 | 0.9082 | 0.8346-0.9662 |
| CCL23_PRR11_TXNDC11_MAPRE3_DIO3_GPR107_CADM1 | 0.9087 | 0.8376-0.9641 |
| DNAI7_GALM_GINS4_MAPRE3_DIO3_GPR107_CADM1 | 0.9017 | 0.842-0.9524 |
| PRR11_GALM_GINS4_MAPRE3_DIO3_GPR107_CADM1 | 0.9199 | 0.8615-0.9641 |
| CCL23_GALM_GINS4_MAPRE3_DIO3_GPR107_CADM1 | 0.9134 | 0.8532-0.9597 |
| PRR11_DNAI7_GINS4_MAPRE3_DIO3_GPR107_CADM1 | 0.9152 | 0.8563-0.9602 |
| CCL23_DNAI7_GINS4_MAPRE3_DIO3_GPR107_CADM1 | 0.9069 | 0.8407-0.9545 |
| CCL23_PRR11_GINS4_MAPRE3_DIO3_GPR107_CADM1 | 0.913 | 0.8537-0.9619 |
| PRR11_DNAI7_GALM_MAPRE3_DIO3_GPR107_CADM1 | 0.9035 | 0.842-0.9533 |
| CCL23_DNAI7_GALM_MAPRE3_DIO3_GPR107_CADM1 | 0.8944 | 0.8246-0.9506 |
| CCL23_PRR11_GALM_MAPRE3_DIO3_GPR107_CADM1 | 0.91 | 0.8498-0.9589 |
| CCL23_PRR11_DNAI7_MAPRE3_DIO3_GPR107_CADM1 | 0.9013 | 0.8329-0.952 |
| DNAI7_GALM_GINS4TXNDC11_DIO3_GPR107_CADM1 | 0.9247 | 0.8749-0.9675 |
| PRR11_GALM_GINS4_TXNDC11_DIO3_GPR107_CADM1 | 0.9277 | 0.8766-0.9701 |
| CCL23_GALM_GINS4_TXNDC11_DIO3_GPR107_CADM1 | 0.9242 | 0.8623-0.9675 |
| PRR11_DNAI7_GINS4_TXNDC11_DIO3_GPR107_CADM1 | 0.9152 | 0.8489-0.9667 |
| CCL23_DNAI7_GINS4_TXNDC11_DIO3_GPR107_CADM1 | 0.9095 | 0.8208-0.9662 |
| CCL23_PRR11_GINS4_TXNDC11_DIO3_GPR107_CADM1 | 0.9117 | 0.8436-0.9649 |
| PRR11_DNAI7_GALM_TXNDC11_DIO3_GPR107_CADM1 | 0.9186 | 0.861-0.9654 |
| CCL23_DNAI7_GALM_TXNDC11_DIO3_GPR107_CADM1 | 0.9169 | 0.8576-0.961 |
| CCL23_PRR11_GALM_TXNDC11_DIO3_GPR107_CADM1 | 0.9229 | 0.8723-0.9667 |
| CCL23_PRR11_DNAI7_TXNDC11_DIO3_GPR107_CADM1 | 0.9134 | 0.8437-0.9654 |
| PRR11_DNAI7_GALM_GINS4_DIO3_GPR107_CADM1 | 0.9152 | 0.8541-0.9632 |
| CCL23_DNAI7_GALM_GINS4_DIO3_GPR107_CADM1 | 0.9108 | 0.855-0.9558 |
| CCL23_PRR11_GALM_GINS4_DIO3_GPR107_CADM1 | 0.9234 | 0.8675-0.9654 |
| CCL23_PRR11_DNAI7_GINS4_DIO3_GPR107_CADM1 | 0.9195 | 0.8675-0.9654 |
| CCL23_PRR11_DNAI7_GALM_DIO3_GPR107_CADM1 | 0.9104 | 0.8541-0.9606 |
| DNAI7_GALM_GINS4_TXNDC11_MAPRE3_GPR107_CADM1 | 0.9017 | 0.8368-0.9546 |
| PRR11_GALM_GINS4_TXNDC11_MAPRE3_GPR107 _CADM1 | 0.9078 | 0.8481-0.9589 |
| CCL23_GALM_GINS4 _TXNDC11_MAPRE3_GPR107_CADM1 | 0.9108 | 0.8524-0.958 |
| PRR11_DNAI7_GINS4_TXNDC11_MAPRE3_GPR107_CADM1 | 0.8909 | 0.819-0.9507 |
| CCL23_DNAI7_GINS4_TXNDC11_MAPRE3_GPR107_CADM1 | 0.8879 | 0.8143-0.9519 |
| CCL23_PRR11_GINS4_TXNDC11_MAPRE3_GPR107_CADM1 | 0.8935 | 0.8251-0.9533 |
| PRR11_DNAI7_GALM_TXNDC11_MAPRE3_GPR107_CADM1 | 0.9022 | 0.8385-0.9545 |
| CCL23_DNAI7_GALM_TXNDC11_MAPRE3_GPR107_CADM1 | 0.9026 | 0.839-0.9554 |
| CCL23_PRR11_GALM_TXNDC11_MAPRE3_GPR107_CADM1 | 0.9104 | 0.8515-0.9641 |
| CCL23_PRR11_DNAI7_TXNDC11_MAPRE3_GPR107_CADM1 | 0.8931 | 0.816-0.9485 |
| PRR11_DNAI7_GALM_GINS4_MAPRE3_GPR107_CADM1 | 0.8892 | 0.8247-0.9455 |
| CCL23_DNAI7_GALM_GINS4_MAPRE3_GPR107_CADM1 | 0.8922 | 0.8285-0.9407 |
| CCL23_PRR11_GALM_GINS4_MAPRE3_GPR107_CADM1 | 0.9061 | 0.8484-0.9541 |
| CCL23_PRR11_DNAI7_GINS4_MAPRE3_GPR107_CADM1 | 0.8909 | 0.8277-0.9476 |
| CCL23_PRR11_DNAI7_GALM_MAPRE3_GPR107_CADM1 | 0.8931 | 0.8307-0.9442 |
| PRR11_DNAI7_GALM_GINS4_TXNDC11_GPR107_CADM1 | 0.897 | 0.8342-0.9528 |
| CCL23_DNAI7_GALM_GINS4_TXNDC11_GPR107_CADM1 | 0.9026 | 0.8428-0.9554 |
| CCL23_PRR11_GALM_GINS4_TXNDC11_GPR107_CADM1 | 0.9095 | 0.8454-0.9585 |
| CCL23_PRR11_DNAI7_GINS4_TXNDC11_GPR107_CADM1 | 0.8918 | 0.816-0.9502 |
| CCL23_PRR11_DNAI7_GALM_TXNDC11_GPR107_CADM1 | 0.9065 | 0.8511-0.9546 |
| CCL23_PRR11_DNAI7_GALM_GINS4_GPR107_CADM1 | 0.8983 | 0.8411-0.9498 |
| DNAI7_GALM_GINS4_TXNDC11_MAPRE3_DIO3_CADM1 | 0.9203 | 0.8684-0.9632 |
| PRR11_GALM_GINS4_TXNDC11_MAPRE3_DIO3_CADM1 | 0.932 | 0.8762-0.9719 |
| CCL23_GALM_GINS4_TXNDC11_MAPRE3_DIO3_CADM1 | 0.932 | 0.8835-0.974 |
| PRR11_DNAI7_GINS4_TXNDC11_MAPRE3_DIO3_CADM1 | 0.9195 | 0.8619-0.9675 |
| CCL23_DNAI7_GINS4_TXNDC11_MAPRE3_DIO3_CADM1 | 0.9082 | 0.8363-0.9636 |
| CCL23_PRR11_GINS4_TXNDC11_MAPRE3_DIO3_CADM1 | 0.9169 | 0.8519-0.9662 |
| PRR11_DNAI7_GALM_TXNDC11_MAPRE3_DIO3_CADM1 | 0.9199 | 0.8654-0.9654 |
| CCL23_DNAI7_GALM_TXNDC11_MAPRE3_DIO3_CADM1 | 0.9177 | 0.8602-0.9615 |
| CCL23_PRR11_GALM_TXNDC11_MAPRE3_DIO3_CADM1 | 0.9255 | 0.8701-0.968 |
| CCL23_PRR11_DNAI7_XNDC11_MAPRE3_DIO3_CADM1 | 0.9147 | 0.8454-0.9693 |
| PRR11_DNAI7_GALM_GINS4_MAPRE3_DIO3_CADM1 | 0.9104 | 0.8485-0.9598 |
| CCL23_DNAI7_GALM_GINS4_MAPRE3_DIO3_CADM1 | 0.9017 | 0.8368-0.9511 |
| CCL23_PRR11_GALM_GINS4_MAPRE3_DIO3_CADM1 | 0.9173 | 0.8554-0.9641 |
| CCL23_PRR11_DNAI7_GINS4_MAPRE3_DIO3_CADM1 | 0.913 | 0.8576-0.9628 |
| CCL23_PRR11_DNAI7_GALM_MAPRE3_DIO3_CADM1 | 0.9026 | 0.842-0.9524 |
| PRR11_DNAI7_GALM_GINS4_TXNDC11_DIO3_CADM1 | 0.9251 | 0.8723-0.9671 |
| CCL23_DNAI7_GALM_GINS4_TXNDC11_DIO3_CADM1 | 0.9251 | 0.8731-0.9693 |
| CCL23_PRR11_GALM_GINS4_TXNDC11_DIO3_CADM1 | 0.9316 | 0.8796-0.974 |
| CCL23_PRR11_DNAI7_GINS4_TXNDC11_DIO3_CADM1 | 0.9216 | 0.8588-0.9719 |
| CCL23_PRR11_DNAI7_GALM_TXNDC11_DIO3_CADM1 | 0.926 | 0.8697-0.968 |
| CCL23_PRR11_DNAI7_GALM_GINS4_DIO3_CADM1 | 0.9195 | 0.8654-0.9654 |
| PRR11_DNAI7_GALM_GINS4_TXNDC11_MAPRE3_CADM1 | 0.9017 | 0.8432-0.955 |
| CCL23_DNAI7_GALM_GINS4_TXNDC11_MAPRE3_CADM1 | 0.9017 | 0.8385-0.9524 |
| CCL23_PRR11_GALM_GINS4_TXNDC11_MAPRE3_CADM1 | 0.9095 | 0.848-0.9602 |
| CCL23_PRR11_DNAI7_GINS4_TXNDC11_MAPRE3_CADM1 | 0.887 | 0.8186-0.9481 |
| CCL23_PRR11_DNAI7_GALM_TXNDC11_MAPRE3_CADM1 | 0.9048 | 0.8446-0.9537 |
| CCL23_PRR11_DNAI7_GALM_GINS4_MAPRE3_CADM1 | 0.8892 | 0.819-0.9463 |
| DNAI7_GALM_GINS4_TXNDC11_MAPRE3_DIO3_GPR107 | 0.9208 | 0.8684-0.9619 |
| PRR11_GALM_GINS4_TXNDC11_MAPRE3_DIO3_GPR107 | 0.9316 | 0.8775-0.9719 |
| CCL23_GALM_GINS4_TXNDC11_MAPRE3_DIO3_GPR107 | 0.9251 | 0.8645-0.9645 |
| PRR11_DNAI7_GINS4_TXNDC11_MAPRE3_DIO3_GPR107 | 0.9212 | 0.8636-0.9649 |
| CCL23 _DNAI7_GINS4_TXNDC11_MAPRE3_DIO3_GPR107 | 0.9152 | 0.8489-0.9688 |
| CCL23 _PRR11_GINS4_TXNDC11_MAPRE3 DIO3 GPR107 | 0.9195 | 0.8623-0.968 |
| PRR11_DNAI7_GALM_TXNDC11 _MAPRE3_DIO3_GPR107 | 0.9186 | 0.8636-0.9632 |
| CCL23_DNAI7_GALM_TXNDC11_MAPRE3_DIO3_GPR107 | 0.9169 | 0.8567-0.9628 |
| CCL23_PRR11_GALM_TXNDC11_MAPRE3_DIO3_GPR107 | 0.9277 | 0.8805-0.9684 |
| CCL23 _PRR11_DNAI7_TXNDC11_MAPRE3_DIO3_GPR107 | 0.919 | 0.8515-0.9701 |
| PRR11_DNAI7_GALM_GINS4_MAPRE3_DIO3_GPR107 | 0.91 | 0.8537-0.9571 |
| CCL23_DNAI7_GALM_GINS4_MAPRE3_DIO3_GPR107 | 0.8948 | 0.8316-0.9437 |
| CCL23_PRR11_GALM_GINS4_MAPRE3_DIO3_GPR107 | 0.9212 | 0.8671-0.9654 |
| CCL23_PRR11_DNAI7_GINS4_MAPRE3_DIO3_GPR107 | 0.9126 | 0.8489-0.9598 |
| CCL23_PRR11_DNAI7_GALM_MAPRE3_DIO3_GPR107 | 0.8991 | 0.832-0.9485 |
| PRR11_DNAI7_GALM_GINS4_TXNDC11_DIO3_GPR107 | 0.9255 | 0.8688-0.968 |
| CCL23_DNAI7_GALM_GINS4_TXNDC11_DIO3_GPR107 | 0.9221 | 0.8675-0.9645 |
| CCL23_PRR11_DNAI7_GINS4_TXNDC11_DIO3_GPR107 | 0.9208 | 0.8593-0.9693 |
| CCL23_PRR11_DNAI7_GALM_TXNDC11_DIO3_GPR107 | 0.9216 | 0.8667-0.9671 |
| CCL23_PRR11_DNAI7 _GALM_GINS4_DIO3_GPR107 | 0.9121 | 0.8519-0.9584 |
| PRR11_DNAI7_GALM_GINS4_TXNDC11_MAPRE3_GPR107 | 0.9065 | 0.8424-0.9558 |
| CCL23_DNAI7_GALM_GINS4_TXNDC11_MAPRE3_GPR107 | 0.9082 | 0.8476-0.9585 |
| CCL23_PRR11_GALM_GINS4_TXNDC11_MAPRE3_GPR107 | 0.9173 | 0.8571-0.9658 |
| CCL23_PRR11_DNAI7_GINS4_TXNDC11_MAPRE3_GPR107 | 0.8974 | 0.8281-0.9541 |
| CCL23_PRR11_DNAI7_GALM_TXNDC11_MAPRE3_GPR107 | 0.9091 | 0.8493-0.958 |
| CCL23_PRR11_DNAI7_GALM_GINS4_MAPRE3_GPR107 | 0.8935 | 0.8285-0.945 |
| CCL23_PRR11_DNAI7_GALM_GINS4_TXNDC11_GPR107 | 0.9078 | 0.8467-0.9558 |
| PRR11_DNAI7_GALM_GINS4_TXNDC11_MAPRE3_DIO3 | 0.9212 | 0.8688-0.9615 |
| CCL23_DNAI7_GALM_GINS4_TXNDC11_MAPRE3_DIO3 | 0.9199 | 0.864-0.9641 |
| CCL23_PRR11_GALM_GINS4_TXNDC11_MAPRE3_DIO3 | 0.9364 | 0.8913-0.974 |
| CCL23_PRR11_DNAI7_GINS4_TXNDC11_MAPRE3_DIO3 | 0.9234 | 0.8679-0.9701 |
| CCL23_PRR11_DNAI7_GALM_TXNDC11_MAPRE3_DIO3 | 0.9242 | 0.8706-0.9688 |
| CCL23_PRR11_DNAI7_GALM_GINS4_TXNDC11_DIO3_ | 0.9303 | 0.8796-0.9697 |
| CCL23_PRR11_DNAI7_GALM_GINS4_TXNDC11_MAPRE3 | 0.9069 | 0.8389-0.9619 |

| **Combinations of eight biomarkers** | | |
|---|---|---|
| CCL23_PRR11_GAS6_GALM_TXNDC11_CAV1_DIO3_CADM1 | 0.9394 | 0.8827-0.9818 |
| PRR11_DNAI7_GALM_GINS4_TXNDC11_CAV1_DIO3 PTGDR2 | 0.9364 | 0.8866-0.9753 |
| CCL23_DNAI7_GALM_GINS4_TXNDC1_CAV1_DIO3_PTGDR2 | 0.929 | 0.8693-0.9775 |
| CCL23_PRR11_GALM_GINS4_TXNDC11_CAV1_DIO3 PTGDR2 | 0.9368 | 0.887-0.9753 |
| CCL23_PRR11_DNAI7_GINS4_TXNDC11_CAV1_DIO3_PTGDR2 | 0.9368 | 0.8853-0.9784 |
| CCL23_PRR11_DNAI7_GALM_TXNDC11_CAV1_DIO3_PTGDR2 | 0.939 | 0.89-0.9775 |
| CCL23_PRR11_DNAI7_GALM_GINS4_CAV1_DIO3_PTGDR2 | 0.9446 | 0.8956-0.981 |
| CCL23_PRR11_DNAI7_GALM_GINS4_TXNDC11_DIO3_PTGDR2 | 0.9268 | 0.8762-0.9684 |
| CCL23_PRR11_DNAI7_GALM_GINS4_TXNDC11_CAV1_PTGDR2 | 0.9113 | 0.8502-0.9615 |
| CCL23_PRR11_DNAI7_GALM_GINS4_TXNDC11_CAV1_DIO3 | 0.945 | 0.9017-0.9771 |
| DNAI7_GALM_GINS4_TXNDC11_MAPRE3_DIO3_GPR107_CADM1 | 0.932 | 0.8823-0.9715 |
| PRR11_GALM_GINS4_TXNDC11_MAPRE3_DIO3_GPR107_CADM1 | 0.9377 | 0.8857-0.9758 |
| CCL23_GALM_GINS4_TXNDC11_MAPRE3_DIO3_GPR107_CADM1 | 0.9377 | 0.8827-0.9775 |
| PRR11_DNAI7_GINS4_TXNDC11_MAPRE3_DIO3_GPR107_CADM1 | 0.9268 | 0.8688-0.974 |
| CCL23_DNAI7_GINS4_TXNDC11_MAPRE3_DIO3_GPR107_CADM1 | 0.9238 | 0.8558-0.9727 |
| CCL23_PRR11_GINS4_TXNDC11_MAPRE3_DIO3_GPR107_CADM1 | 0.9229 | 0.8662-0.9701 |
| PRR11_DNAI7_GALM_TXNDC11_MAPRE3_DIO3_GPR107_CADM1 | 0.9351 | 0.8801-0.9732 |
| CCL23_DNAI7_GALM_TXNDC11_MAPRE3_DIO3_GPR107_CADM1 | 0.9303 | 0.8736-0.9714 |
| CCL23_PRR11_GALM_TXNDC11_MAPRE3_DIO3_GPR107_CADM1 | 0.9316 | 0.8809-0.9727 |
| CCL23_PRR11_DNAI7_TXNDC11_MAPRE3_DIO3_GPR107_CADM1 | 0.9242 | 0.858-0.9732 |
| PRR11_DNAI7_GALM_GINS4_MAPRE3_DIO3_GPR107_CADM1 | 0.9229 | 0.8697-0.9641 |
| CCL23_DNAI7_GALM_GINS4_MAPRE3_DIO3_GPR107CADM1 | 0.9156 | 0.8619-0.9602 |
| CCL23_PRR11_GALM_GINS4_MAPRE3_DIO3_GPR107_CADM1 | 0.9294 | 0.8757-0.9684 |
| CCL23_PRR11_DNAI7_GINS4_MAPRE3_DIO3_GPR107_CADM1 | 0.9286 | 0.8693-0.9701 |
| CCL23_PRR11_DNAI7_GALM_MAPRE3_DIO3_GPR107_CADM1 | 0.9139 | 0.8571-0.9619 |
| PRR11_DNAI7_GALM_GINS4_TXNDC11_DIO3_GPR107_CADM1 | 0.9338 | 0.884-0.9736 |
| CCL23_DNAI7_GALM_GINS4_TXNDC11_DIO3_GPR107_CADM1 | 0.9333 | 0.8818-0.9749 |
| CCL23_PRR11_GALM_GINS4_TXNDC11_DIO3_GPR107_CADM1 | 0.9368 | 0.8865-0.9758 |
| CCL23_PRR17_DNAI7_GINS4_TXNDC11_DIO3_GPR107_CADM1 | 0.9273 | 0.8649-0.9758 |
| CCL23_PRR11_DNAI7_GALM_TXNDC11_DIO3_GPR107_CADM1 | 0.932 | 0.8792-0.9697 |
| CCL23_PRR11_DNAI7_GALM_GINS4_DIO3_GPR107_CADM1 | 0.9273 | 0.874-0.9697 |
| PRR11_DNAI7_GALM_GINS4_TXNDC11_MAPRE3_GPR107_CADM1 | 0.9039 | 0.8437-0.9532 |
| CCL23_DNAI7_GALM_GINS4_TXNDC11_MAPRE3_GPR107_CADM1 | 0.9108 | 0.8485-0.9615 |
| CCL23_PRR11_GALM_GINS4_TXNDC11_MAPRE3_GPR107_CADM1 | 0.9134 | 0.8532-0.9632 |
| CCL23_PRR11_DNAI7_GINS4_TXNDC11_MAPRE3_GPR107_CADM1 | 0.8978 | 0.8242-0.955 |
| CCL23_PRR11_DNAI7_GALM_TXNDC11_MAPRE3_GPR107_CADM1 | 0.9117 | 0.8554-0.9576 |
| CCL23_PRR11_DNAI7_GALM_GINS4_MAPRE3_GPR107_CADM1 | 0.897 | 0.8389-0.9494 |
| CCL23_PRR11_DNAI7_GALM_GINS4_TXNDC11_GPR107_CADM1 | 0.9082 | 0.8481-0.9584 |
| PRR11_DNAI7_GALM_GINS4_TXNDC11_MAPRE3_DIO3_CADM1 | 0.9294 | 0.8762-0.9715 |
| CCL23_DNAI7_GALM_GINS4_TXNDC11_MAPRE3_DIO3_CADM1 | 0.9333 | 0.8809-0.9749 |
| CCL23_PRR11_GALM_GINS4_TXNDC11_MAPRE3_DIO3_CADM1 | 0.9403 | 0.8948-0.9784 |
| CCL23_PRR11_DNAI7_GINS4_TXNDC11_MAPRE3_DIO3_CADM1 | 0.929 | 0.8705-0.9766 |
| CCL23_PRR11_DNAI7_GALM_TXNDC11_MAPRE3_DIO3_CADM1 | 0.932 | 0.8801-0.9745 |
| CCL23_PRR11_DNAI7_GALM_GINS4_MAPRE3_DIO3_CADM1 | 0.9221 | 0.8658-0.9667 |
| CCL23_PRR11_DNAI7_GALM_GINS4_TXNDC11_DIO3_CADM1 | 0.9364 | 0.8883-0.974 |
| CCL23_PRR11_DNAI7_GALM_GINS4_TXNDC11_MAPRE3_CADM1 | 0.9091 | 0.851-0.9589 |
| PRR11_DNAI7_GALM_GINS4_TXNDC11_MAPRE3_DIO3_GPR107 | 0.9359 | 0.8874-0.9758 |
| CCL23_DNAI7_GALM_GINS4_TXNDC11_MAPRE3_DIO3_GPR107 | 0.9264 | 0.8731-0.968 |
| CCL23_PRR11_GALM_GINS4_TXNDC11_MAPRE3_DIO3_GPR107 | 0.9381 | 0.8909-0.9784 |
| CCL23_PRR11_DNAI7_GINS4_TXNDC11_MAPRE3_DIO3_GPR107 | 0.932 | 0.8709-0.9779 |
| CCL23_PRR11_DNAI7_GALM_TXNDC11_MAPRE3_DIO3_GPR107 | 0.9307 | 0.8857-0.971 |
| CCL23_PRR11_DNAI7_GALM_GINS4_MAPRE3_DIO3_GPR107 | 0.9177 | 0.8649-0.9628 |
| CCL23_PRR11_DNAI7_GALM_GINS4_TXNDC11_DIO3_GPR107 | 0.9342 | 0.8861-0.9736 |
| CCL23_PRR11_DNAI7_GALM_GINS4_TXNDC11_MAPRE3_GPR107 | 0.9152 | 0.8567-0.9641 |
| CCL23_PRR11_DNAI7_GALM_GINS4_TXNDC11_MAPRE3_DIO3 | 0.9333 | 0.8835-0.974 |

| **Combinations of nine biomarkers** | | |
|---|---|---|
| CCL23_PRR11_DNAI7_GALM_GINS4_TXNDC11_CAV1_DIO3_PTGDR2 | 0.9446 | 0.9008-0.9788 |
| PRR11_DNAI7_GALM_GINS4_TXNDC11_MAPRE3_DIO3_GPR107_CADM1 | 0.9407 | 0.8931-0.9797 |
| CCL23_DNAI7_GALM_GINS4_TXNDC11_MAPRE3_DIO3_GPR107_CADM1 | 0.9416 | 0.89-0.9801 |
| CCL23_PRR11_GALM_GINS4_TXNDC11_MAPRE3_DIO3_GPR107_CADM1 | 0.9446 | 0.8956-0.9801 |
| CCL23_PRR11_DNAI7_GINS4_TXNDC11_MAPRE3_DIO3_GPR107_CADM1 | 0.9372 | 0.8801-0.9797 |
| CCL23_PRR11_DNAI7_GALM_TXNDC11_MAPRE3_DIO3_GPR107_CADM1 | 0.9429 | 0.8961-0.9801 |
| CCL23_PRR11_DNAI7_GALM_GINS4_MAPRE3_DIO3_GPR107_CADM1 | 0.929 | 0.8784-0.9693 |
| CCL23_PRR11_DNAI7_GALM_GINS4_TXNDC11_DIO3_GPR107_CADM1 | 0.945 | 0.9004-0.9814 |
| CCL23_PRR11_DNAI7_GALM_GINS4_TXNDC11_MAPRE3_GPR107_CADM1 | 0.9152 | 0.8528-0.9645 |
| CCL23_PRR11_DNAI7_GALM_GINS4_TXNDC11_MAPRE3_DIO3_CADM1 | 0.9372 | 0.8913-0.9779 |
| CCL23_PRR11_DNAI7_GALM_GINS4_TXNDC11_MAPRE3_DIO3_GPR107 | 0.9433 | 0.9004-0.9805 |

| **Combination of ten biomarkers** | | |
|---|---|---|
| CCL23_PRR11_DNAI7_GALM_GINS4_TXNDC11_MAPRE3_DIO3_GPR107_CADM1 | 0.9528 | 0.9121-0.9874 |

So, the first embodiment of present invention refers to an *in vitro* method for selecting or identifying biomarkers signatures for the diagnosis of a *Mycoplasma pneumoniae* infection and/or for the differential diagnosis between a *Mycoplasma pneumoniae* pneumonia and other bacterial or viral pneumonias which comprises: a) Assessing the level of expression of at least one gene, or any combination thereof comprising between two and eighteen genes, selected from the list consisting of: PRR11, FKBP11, TXNDC11, DIO3, GAS6, HYOU1, CAV1, GINS4, GALM, DNAI7, CCL23, ELL2, UAP1, CDCA2, CADM1, PTGDR2, GPR107 and/or MAPRE3 in a biological sample obtained from the subject, wherein the identification of a statistically significant variation of the level of expression with respect a pre-established threshold level determined in subjects with viral pneumonia, is an indication that the biomarkers signatures may be used for the diagnosis of a *Mycoplasma pneumoniae* infection and/or for the differential diagnosis between a *Mycoplasma pneumoniae* pneumonia and other bacterial or viral pneumonias.

The second embodiment refers to an *in vitro* method for the diagnosis of a *Mycoplasma pneumoniae* infection and/or for the differential diagnosis between a *Mycoplasma pneumoniae* pneumonia and other bacterial or viral pneumonias, the method comprising determining the level of expression of at least one gene, or any combination thereof comprising between two and eighteen genes, selected from the list consisting of: PRR11, FKBP11, TXNDC11, DIO3, GAS6, HYOU1, CAV1, GINS4, GALM, DNAI7, CCL23, ELL2, UAP1, CDCA2, CADM1, PTGDR2, GPR107 and/or MAPRE3, in a biological sample obtained from the subject.

The third embodiment of the present invention refers to an *in vitro* method for selecting the antibiotic treatment for a patient suffering from pneumonia which comprises determining whether the patient is suffering from *Mycoplasma pneumoniae* pneumonia by following the method of the invention, wherein, if the patient is suffering from *Mycoplasma pneumoniae* pneumonia a treatment with a macrolide antibiotic may be recommended and a treatment with beta-lactam antibiotic can be initially discarded, and/or wherein if the patient is not suffering from *Mycoplasma pneumoniae* pneumonia other clinical decisions could be considered.

In a preferred embodiment, the beta-lactam antibiotic is selected, for instance, from: Amoxicillin, ampicillin, or cefotaxime and/or the macrolide antibiotic is selected, for instance, from: Azithromycin, clarithromycin or erythromycin.

The fourth embodiment of the present invention refers to the *in vitro* use of at least one gene, or any combination thereof comprising between two and eighteen genes, selected from the list consisting of: PRR11, FKBP11, TXNDC11, DIO3, GAS6, HYOU1, CAV1, GINS4, GALM, DNAI7, CCL23, ELL2, UAP1, CDCA2, PTGDR2, CADM1, GPR107 and/or MAPRE3; or of a kit comprising reagents for the determining the level of expression of the genes; for the diagnosis of a *Mycoplasma pneumoniae* infection and/or for the differential diagnosis between a *Mycoplasma pneumoniae* pneumonia and other bacterial or viral pneumonias and/or for selecting the antibiotic treatment for a patient suffering from pneumonia.

In a preferred embodiment, the method comprises determining the level of expression of at least a combination of genes selected from the list shown in **Table 1** or **Table 2.**

In a preferred embodiment, the method comprises determining the level of expression of at least one combination of genes selected from: DIO3, GALM and TXNDC11; FKBP11, TXNDC11 and DIO3; GAS6, HYOU1, CAV1 and DIO3; or PRR11, DNAI7, GALM, GINS4, TXNDC11 and CAV1.

In a preferred embodiment, the method comprises determining the level of expression of at least one combination of genes that comprises the genes DIO3, GALM, and TXNDC11 selected from: DNAI7, GALM, GINS4, TXNDC11 and DIO3; CCL23, GALM, TXNDC11, ELL2, DIO3, UAP1 and CDCA2; CCL23, PRR11, GAS6, GALM, TXNDC11, CAV1, DIO3 and CADM1; CCL23, PRR11, DNAI7, GALM, GINS4, TXNDC11, CAV1, DIO3 and PTGDR2; or CCL23, PRR11, DNAI7, GALM, GINS4, TXNDC11, MAPRE3, DIO3, GPR107 and CADM1.

In a preferred embodiment, the identification of a higher level of expression with respect a pre-established threshold level determined in subjects with viral pneumonia, is an indication that the subject has a *Mycoplasma pneumoniae* infection and/or that the subject has a *Mycoplasma pneumoniae* pneumonia rather than another bacterial or viral pneumonias and/or that a treatment with a macrolide antibiotic may be recommended and a treatment with beta-lactam antibiotic can be initially discarded.

In a preferred embodiment, the biological sample is selected from blood, plasma or serum.

In a preferred embodiment, the present invention is a computer-implemented invention, wherein a processing unit (hardware) and a software are configured to: a) Receive the expression level values of any of the above cited biomarkers or signatures, b) process the concentration level values received for finding substantial variations or deviations, and c) provide an output through a terminal display of the variation or deviation of the expression level, wherein the variation or deviation of the expression level indicates that the subject may be suffering from *Mycoplasma pneumoniae* pneumonia.

For the purposes of the present invention the following terms defined:
- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of". Thus, the phrase "consisting of" indicates that the listed elements are required or mandatory, and that no other elements may be present.
- According to the present invention, a reference value can be a "pre-established threshold value" or a "cut-off value". Typically, a "threshold value" or "cut-off value" can be determined experimentally, empirically, or theoretically. According to the present invention, the "pre-established threshold" value refers to a value previously determined in subjects with viral pneumonia. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.

### Description of the figures

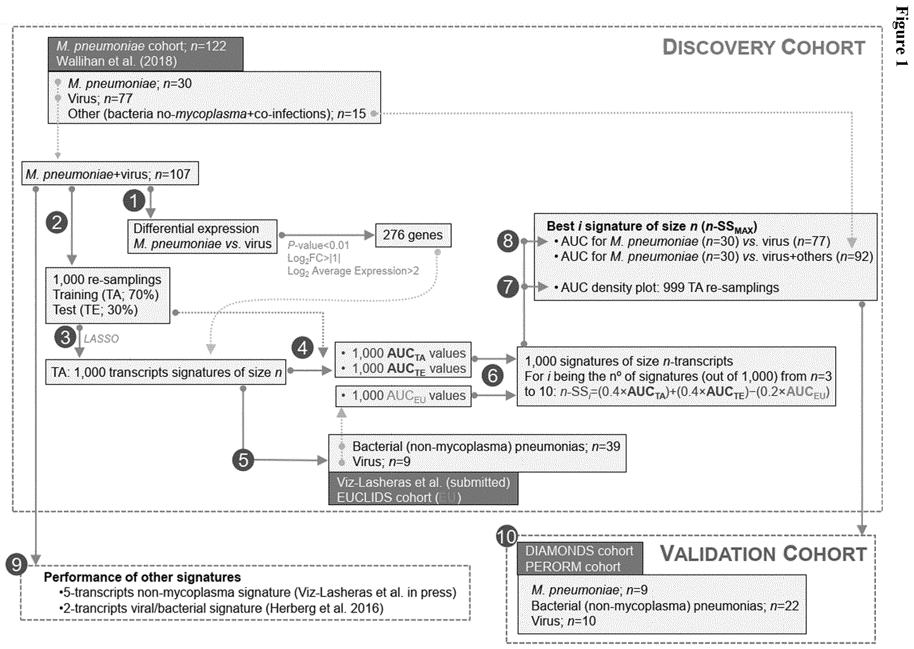

**Figure 1****.** Overall study design. Differential expression was investigated between *M*. *pneumoniae* and virus datasets from Wallihan et al. (2018). We employed a re-sampling approach to generate 1,000 transcript signatures of size n. These signatures were utilized to compute 1,000 AUC values on both the training (TA) and test (TE) sets, as well as on the non-mycoplasma bacteria and virus datasets from the EUCLIDS cohort. Next, Signature Scores (SS) were obtained for the different n-transcript signatures (from n=3 to 10) based on AUCTA, AUCTE, and AUCEU values, allowing to derive the best signature of size n; n-SSMAX. AUC values were then computed with the best n-transcript signatures for the comparison between *M. pneumoniae* vs. virus and vs. virus + other pathogens from Wallihan et al. For comparative purposes, we also assessed *M*. *pneumoniae* the performance of other available signatures in the literature. Finally, the best signatures were subsequently validated in the DIAMOND/PERFORM cohorts.

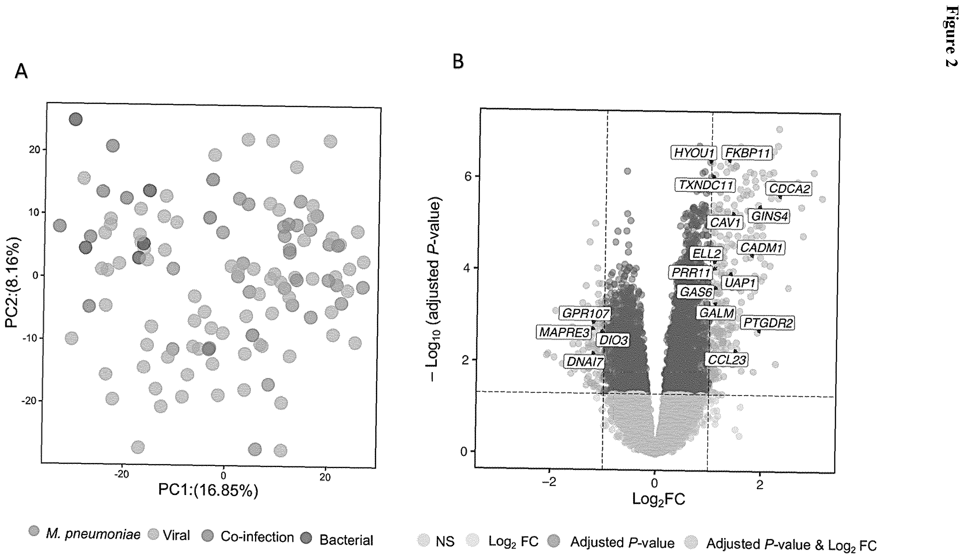

**Figure 2****.** PCA of transcriptomic profiles of different pneumonia cohorts and differential expression analysis. **A)** PCA of transcriptome profiles from blood samples of viral, M. pneumoniae, co-infections or other bacteria pneumonia infections. The two first principal components (PC1 and PC2) are shown. **B)** Volcano plot showing the DEGs between conditions: M. pneumoniae pneumonia vs. viral pneumonia. Genes with an adjusted P-value < 0.05 and a log2FC > |1| were colored in orange, genes with an adjusted P-value < 0.05 but a log2FC < |1| were colored in dark blue, genes with an adjusted P-value >0.05 but a log2FC > |1| were colored in light blue and non-significant genes were colored in grey. Only genes included in the final signatures were labeled in the graphic.

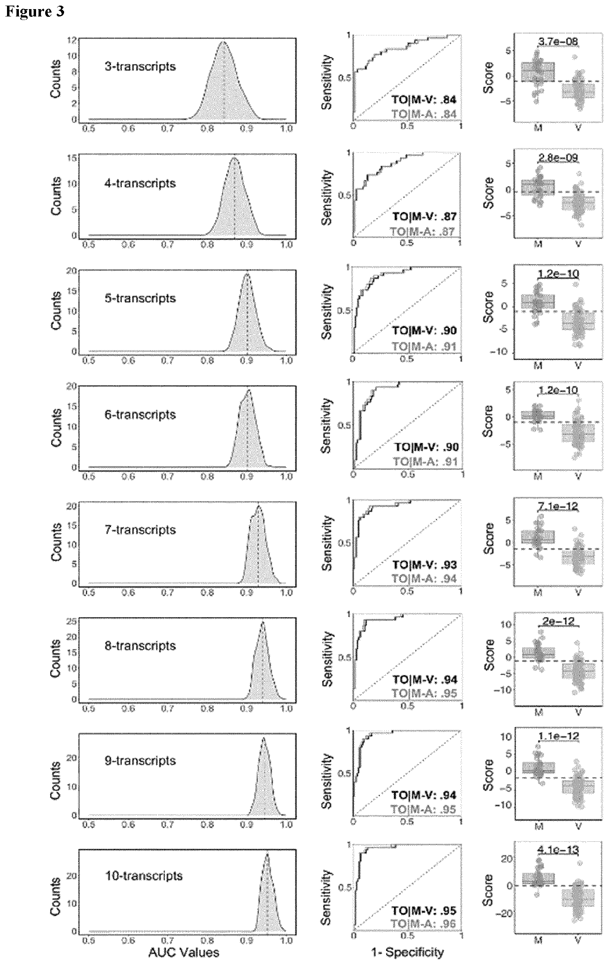

**Figure 3****.** Transcriptomic signatures obtained from the discovery cohort. AUCs and ROC curves from the density plots (left panels) of the AUC values computed on the 999 training resamples and using the optimal n-transcript signature; red dashed vertical lines in the left panels indicate the median values. ROC curves and AUC values (central panels) for the total cohort of *M. pneumoniae* vs. viral pneumonia dataset (black line; AUCTO|M-V) and for the total cohort of *M. pneumoniae* vs. all non-mycoplasma pneumonias (violet line; AUCTO|MA). Boxplots of the predicted values using each optimal model in the total cohort with Wilcoxon P-values (right panels). Red dashed line represents the optimal cutpoint. Abbreviations: A: all non-mycoplasma pneumonias; B: bacterial; M: *M. pneumoniae;* TO: total sample; V: virus.

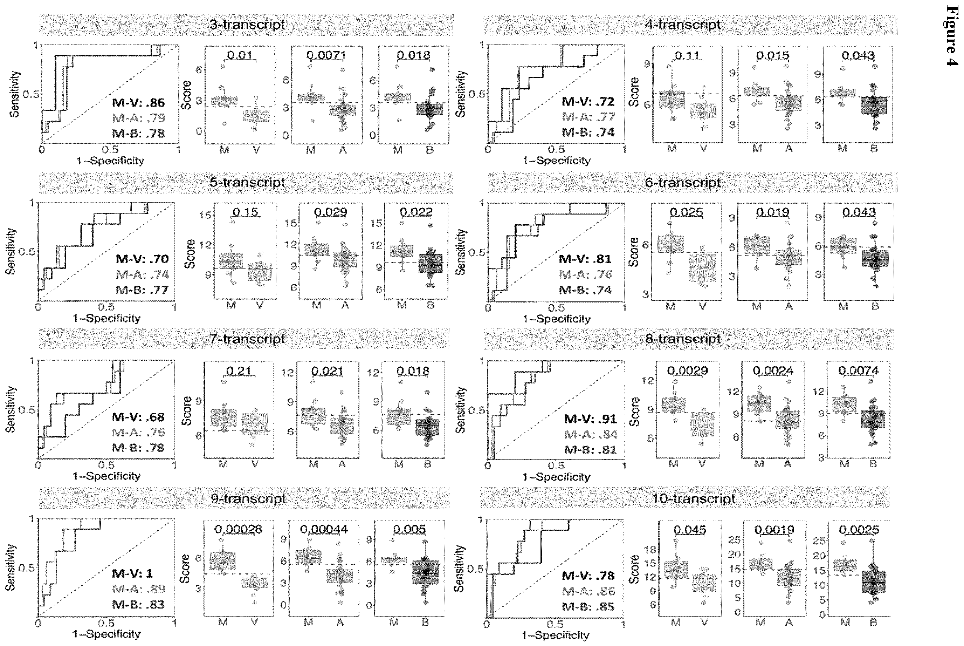

**Figure 4****.** Validation of the transcriptomic signatures. ROC curves with AUC values for the three tested validation subsets: *M. pneumoniae* vs. viral pneumonias (black line; AUCM-V), *M. pneumoniae* vs. all non-mycoplasma pneumonias (violet line; AUCM-A) and *M. pneumoniae* vs. non-mycoplasma bacterial pneumonias (red line; AUCM-B) (left panels). Boxplots of the predicted values using each model in the three validation subsets (right panels). Wilcoxon P-values are also displayed. The red dashed line represents the optimal cutpoint. Abbreviations as in legend of **Figure 3****.**

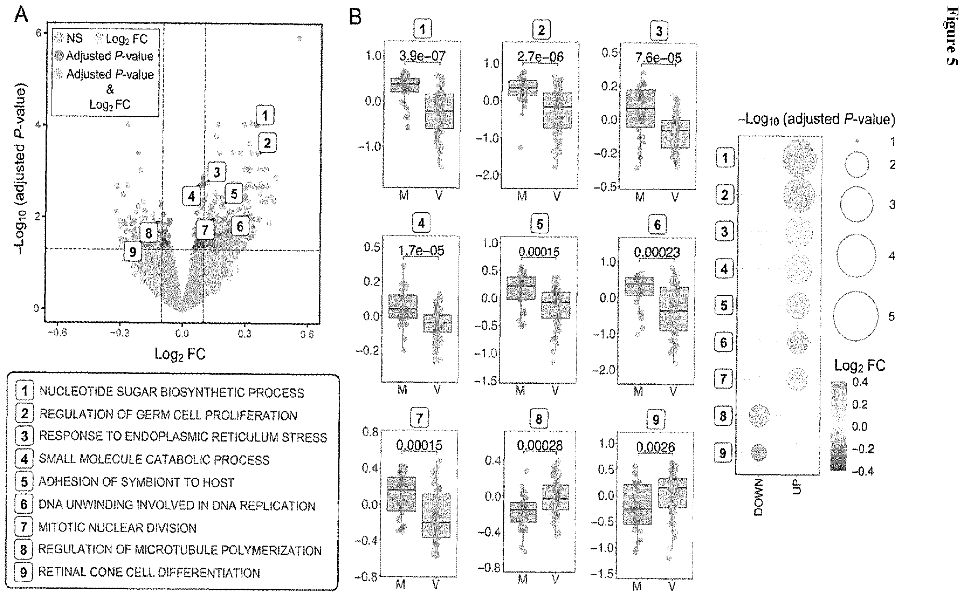

**Figure 5****.** Differential pathways analysis. **A)** Volcano plot showing the genes in DEPs between conditions: *M. pneumoniae* pneumonia vs. viral pneumonia. Pathways meeting the criteria of an adjusted P-value < 0.05 and a log2FC > 10.11 were colored in yellow, pathways with an adjusted P-value < 0.05 but a log2FC < |0.1| were colored in dark blue, genes with an adjusted P-value >0.05 but a log2FC > 10.11 were colored in light blue and non-significant genes were colored in grey. **B)** Boxplots representing the pathway activity of the DEPs for the mycoplasma and viral groups in which genes from the transcriptomic signatures are involved (left panel). Bubble plot of the DEPs in which genes included in the transcriptomic signatures are involved (right panel). Numeric identifies indicate DEPs in which genes from the transcriptomic signatures are involved.

### Detailed description of the invention

The present invention is illustrated by means of the Examples shown below without the intention of limiting the scope of protection.

### Example 1. Material and methods

### Example 1.1. Samples and study design

We investigated the microarray blood transcriptomic profiles of 122 pneumonia children and 20 healthy controls. These cases involved pneumonia caused by *M. pneumoniae* (*n* = 30), various viral infections (*n* = 77), pyogenic bacteria (*n* = 5) and different co-infections (*n* = 10). Samples from coinfected pneumonias caused by *M. pneumoniae* with other pathogens and samples from non-etiology pneumonias were disregarded for downstream analysis. Data were downloaded from the Gene Expression Onmibus (GEO) database with accession number GSE103119.

Additionally, we have used RNAseq data from blood of other pediatric patients with pneumonia not caused by *M. pneumoniae* recruited in the European Union Childhood Life-threatening Infectious Diseases Study (EUCLIDS). The EUCLIDS cohort contains RNAseq data for 39 definitive non-mycoplasma bacterial and 9 definitive viral pneumonia infections. For validation purposes, we have generated new RNAseq data from an additional pediatric cohort comprising blood samples obtained from children infected with *M*. *pneumoniae* (*n* = 9), along with samples from viral (*n* = 10) and bacterial (*n* = 22) pneumonias. These samples were recruited under the umbrella of the PErsonalised Risk assessment in Febrile illness to Optimise Real-life Management across the European Union (PERFORM - https://www.perform2020.org/) and the Diagnosis and Management of Febrile Illness using RNA Personalised Molecular Signature Diagnosis (DIAMONDS - https://www.diamonds2020.eu) consortiums.

### Example 1.2. RNAseq analysis

Whole blood was collected into PAXgene blood RNA tubes (PreAnalytiX), and stored at - 80 °C. Total RNA was isolated using PAXgene blood miRNA isolation kit according to the manufacturer's instructions (Qiagen). An additional DNAse treatment was carried out with the RNA clean & concentrator kit (Zymo Research) prior to sequencing. RNA was quantified using RiboGreen (Invitrogen) on the FLUOstar OPTIMA plate reader (BMG Labtech) and the integrity analyzed on the TapeStation 2200(Agilent, RNA ScreenTape). After a normalization step, a strand specific library preparation was completed using NEBNext^{®} Ultra^{™} II mRNA kit (NEB) and NEB rRNA/globin depletion probes following manufacturer's recommendations. Individual libraries were normalized using Qubit, pooled together and diluted. The sequencing was performed using a 150 paired-end configuration in a Novaseq6000 platform. Quality control of raw data was carried out using *FastQC,* alignment and read counting were performed using *STAR,* alignment filtering was done with *SAMtools* and read counting was carried out using *FeatureCounts.*

### Example 1.3. Statistical analysis

Microarray data pre-process and normalization was performed using the *illuminaHumanv4.db* and *limma* packages. RNAseq data was processed for batch correction using control samples and COMBAT-Seq package. Data was subsequently normalized with DESeq2 package. Principal component analysis (PCA) was conducted to explore the different groups in the data and check for potential outliers. A differential expression (DE) analyses was carried out using *limma* package and accounting for differences in age and sex, in order to compare the blood transcriptome of pneumonia patients with *M. pneumoniae* infection (*n* = 30) *vs.* viral pneumonia infections (*n* = 77).

In order to identify subsets of genes that could serve as predictive transcriptomic signatures differentiating *M. pneumonia* from other pneumonias of viral etiology, we randomly split the dataset (*n* = 107) into 1,000 independent subsets comprising 70% of the samples (training datasets; TA) and other 1,000 subsets containing the remaining 30% of the samples (test datasets; TE); step 2 in **Figure 1****.** A predictive transcriptomic signature was computed using the R package *glmnet* for each of the 1,000 TA. To do that, a logistic LASSO regression model was fitted with the alpha parameter set to 1 and a 10-fold cross validation (step 3 in **Figure 1**); 276 differentially expressed genes (DEGs) were included as input for the logistic regression based on |Log₂FC| > 1, adjusted *P*-value < 0.01, and a Log₂ average expression > 2.

The accuracy of the predictive transcriptomic signatures was measured by calculating the area under the receiver operating characteristic curve (AUC) with 95% confidence intervals (CI) using the *pROC* package. The optimal cut-point value (cut-off) that maximize sensitivity and specificity, was calculated using the *OptimalCutPoints* R package.

AUC values were computed for the 1,000 TA and the corresponding TE datasets (step 4 in **Figure 1**), and also for the non-M. *pneumoniae* pneumonia infections (EUCLIDS cohort); step 5 in **Figure 1****.** Among the 1,000 signatures of size *n* transcripts, we selected those (*i*) of size *n* = 3 to 10 transcripts. Next, on these selected *i* signatures, we computed the score *n*-SS*ᵢ* = (0.4 × AUC_{TA}) + (0.4 × AUC_{TE})- (0.2 × AUC_{EU}) (step 6 in **Figure 1**); this score considers the AUC value on training and test samples but also simultaneously penalize the performance of the signature in other non-*M*. *pneumoniae* pneumonia infections (EUCLIDS cohort). We selected the best transcriptomic signature of size *n* = 3 to 10 (*n*-SS_{MAX}) among the different signatures with the same number of transcripts. The AUC values for the signatures with the *n*-SS_{MAX} values, were then obtained in the 999 complete training resamples (1,000 minus the one used to generate the *n*-SS_{MAX}) (step 7 in **Figure 1**). In addition, AUC values were also calculated for the full pneumonia datasets of *i*) mycoplasmas vs. virus, and *ii*) mycoplasmas vs. virus+others (step 8 in **Figure 1**).

The performance of other signatures to differentiate *M. pneumoniae* from viral pneumonias were also investigated. In particular, we tested the 5-transcript signature recently developed to differentiate non-Mycoplasma pneumonia from viral pneumonia, and the 2-transcript signature developed to differentiate viral from bacterial infections; step 9 in **Figure 1****.**

### Example 1.4. Validation of the best signatures

The performance and accuracy of the predictive transcriptomic signatures were validated using new blood RNAseq data generated from an additional pediatric cohort (PERFORM-DIAMONDS cohort); step 10 in **Figure 1****.** Coefficients and intercepts from each LASSO model were applied to the new data to perform ROC analysis and calculate the AUC, sensitivity, and specificity of the signatures. Three data comparisons were tested: mycoplasma vs. viral pneumonia, mycoplasma vs. bacterial pneumonia, and mycoplasma vs. all other pneumonias including both viral and non-mycoplasma bacterial.

### Example 1.5. GSVA pathway analysis

Biological pathways differentially involved in viral and *M. pneumoniae* pneumonia were inferred from gene expression data using the GSVA algorithm included in the Gene Set Variation Analysis (*GSVA*) R package. Gene Ontology (GO) biological pathways gene set collection from the Molecular Signatures Database (MSigDB) was used as reference database. Significantly differentially expressed pathways (DEPs) were determined using the *limma* package with the viral groups as a reference and a threshold of adjusted P-value < 0.05. All graphics were created using R software v.4.3.2 (www-r-project.org).

### Example 2. Results

### Example 2.1. Transcriptomic analysis

We conducted a PCA on a subset of the 500 most variable genes with the entire pneumonia cohort; which comprised four different groups: bacterial infection, viral infection, *M*. *pneumoniae* infection and co-infections. The first principal component (PC1), explaining 16.85 % of the variation, clearly shows the segregation of the *M. pneumoniae* samples from pneumonias caused by other bacteria. As expected, co-infected samples are distributed evenly between the two groups. Viral profiles intermingle with other profiles in the plot, including *M*. *pneumoniae* and bacterial transcriptomes; **Figure 2A****.** PC2 (accounting for 8.16% of the variation) makes a subtle distinction between bacterial infections on one pole of the component, with most co-infections included in this cluster. *M. pneumoniae* samples are slightly displayed towards the center of the component, while viral samples are evenly distributed along the whole component, reproducing the same behavior as in PC1 (**Figure 2A**).

In a comparative analysis of transcriptomes in children with pneumonia caused by *M*. *pneumoniae vs.* viral pneumonia, we identified 3,783 DEGs, using a significance threshold of False Discovery Rate (FDR) 5%. Among these DEGs, 2,288 were found to be upregulated, while 1,495 were downregulated (**Figure 2B**)**.**

### Example 2.2. Diagnostic signature discovery

To identifying the best minimal signatures for differentiating *M. pneumoniae* infection from viral infections, we constructed a LASSO model using the top 276 DEGs, applying the criteria of *P*-value < 0.01, Log₂FC > | 1|, and Log₂ Average Expression > 2. Subsequently, we generated 1,000 different transcriptomic signatures using the 1,000 randomized TA. We then calculated the AUC for each signature in the TA, TE and the EUCLIDS cohort (non-mycoplasma bacterial and viral pneumonias). Among the 1,000 signatures, we computed the signature scores (SS) to obtain the best signatures of size 3 to 10. Eight transcriptomic signatures emerged as the best candidates for distinguishing pneumonia caused by *M. pneumoniae* from viral pneumonia (**Table 1**). These signatures include genes in common, totaling 18 different transcripts, with 15 over-expressed and 3 under-expressed in mycoplasma pneumonias compared to pneumonias from viral etiology. Notably, all these signatures demonstrated reliable performance when employed to compare *M. pneumoniae* samples against all non-mycoplasma samples, including cases of co-infections and other bacterial pneumonias.

We assessed the performance for each of the eight selected signatures across all randomized datasets (TA), excluding those in which each of the signatures were generated (therefore, 999 subsets). The median AUC values ranged from 0.84 (for the 3-transcript signature) to 0.95 (for the 10-transcript signature) **(****Figure 3****),** demonstrating the overall high-accuracy of the signatures to discriminate between both phenotypes. Additionally, we evaluated each transcriptomic signature in the complete dataset to test their performance in differentiating mycoplasma pneumonias from all other pneumonias, represented with a ROC curve and their respective AUC values. We set the optimal cut-off for each signature to optimize the discrimination of the two categories **(****Figure 3****).** The AUC values from this analysis aligned with the AUC median values observed across all TA datasets **(****Figure 3****).**

### Example 2.3. Validation of M. pneumoniae signatures in an independent cohort

The diagnostic accuracy of the proposed RNA signatures was evaluated using additional gene expression data generated from a new paediatric cohort of pneumonia samples and a different technology (RNAseq). The results confirmed that all RNA signatures can discriminate between viral and mycoplasma pneumonias with AUCs higher than 0.68 (7-transcript), being the most predictive the 9-transcript signature (AUC = 1 [CI: 1-1]; sensitivity: 1; specificity: 1); **Figure 4****.** Unexpectedly, the third most predictive model was the 3-transcript signature (AUC = 0.86 [CI: 0.65-1]; sensitivity: 0.89; specificity: 0.9) (**Figure 4**). Remarkably, all signatures can also accurately differentiate mycoplasma pneumonia from other bacterial pneumonias, with AUCs ranging from 0.74 (CI: 0.55-0.92) for the 4-transcript signature to 0.85 (CI: 0.73-0.99) for the 10-transcript signature. Considering all viral and non-mycoplasma bacterial pneumonias against mycoplasma pneumonias, the AUC values were similar to those obtained from the comparison mycoplasma vs. bacterial comparison. In this case, the 9-transcript signature yielded the best performance of all the signatures tested (AUC = 0.89 [CI:0.79-0.99]); **Figure 4****.**

As in the case of the discovery dataset, we determined the optimal cut-off for each signature in the validation that establishes the threshold to discriminate between pneumonias from different aetiologies in the three comparisons groups (**Figure 4**).

### Example 2.4. Performance of other available signatures in M. pneumoniae samples

As *M. pneumoniae* pediatric pneumonias appear to induce a different alteration in the transcriptome compared to other bacterial infections causing pneumonia in children, we opted to examine the performance of two available signatures designed to differentiate viral and bacterial pediatric infections in samples from children with pneumonia caused by *M*. *pneumoniae:* the two-transcript signature (*IFI44L*/*FAM89A*)*,* capable of differentiating between pediatric viral from bacterial infections; and the novel 5-transcript signature, designed to specifically distinguish between viral and bacterial CAP in children. We observed that neither of these two tested signatures could effectively differentiate *M. pneumoniae* as a bacterial infection or a bacterial pneumonia, yielding AUC values of 0.56 and 0.52, respectively.

### Example 2.5. Differentially expressed pathway analysis between M. pneumoniae and viral pneumonia

To detect biological pathways responsible for the different response to *M. pneumoniae* and viral pneumonia in our study cohort, we performed a GSVA analysis directly from gene expression data. We identify 525 significantly (adjusted P-value < 0.05) DEPs between both categories, with over half upregulated in *M. pneumoniae* pneumonia (358/525; 68%); **Figure 5A****.** Among the top 20 pathways, the most notable changes in pathways activity were mainly represented by up-regulated processes in atypical pneumonia (18/20; 90%).

The most differentially activated pathway (DEP) was "SRP dependent co-translational protein targeting to membrane signal sequence recognition" (adjusted *P*-value = 1×10⁻¹⁶) followed by "interleukin-12 mediated signaling" pathways (adjusted *P*-value= 2×10⁻⁰⁸), and a group of biological routes related to polysaccharides and glycolipids synthesis, including "nucleotide sugar biosynthetic process" (adjusted *P*-value = 9×10⁻⁰⁵), "UDP N-Acetylglucosamine metabolic process" (adjusted *P*-value = 9×10⁻⁰⁵), "amino sugar biosynthetic process" (adjusted *P*-value = 2×10⁻⁰⁵), "UDP N-Acetylglucosamine biosynthetic process" (adjusted P-value = 4×10⁻⁰⁵) and "GDP-mannose metabolic process" (adjusted *P*-value = 9×10⁻⁰⁵).

Afterwards, we investigated the functional involvement of the genes included in the transcriptomic signatures by examining the DEPs related to these genes. We found that 10 out of the 18 genes (*UAP1, PTGDR2, CAV1, HYOU1, GALM, GAS6, GINS4, CDCA2, MAPRE3, DIO3*; **Figure 5B**) participate in 9 significantly DEPs. Notably, among these pathways, "the nucleotide sugar biosynthetic process" emerged as one of the most significant pathways in the overall analysis (adjusted P-value = 9×10⁻⁰⁵), **Figure 5B****.** Additionally, the involvement of two of the predictive genes in the same DEP (*CAV1* and *HYOU1*) was only detected for the "response to endoplasmic reticulum stress process" (adjusted *P*-value = 0.002).

## Claims

1. *In vitro* method for selecting or identifying biomarkers signatures for the diagnosis of a *Mycoplasma pneumoniae* infection and/or for the differential diagnosis between a *Mycoplasma pneumoniae* pneumonia and other bacterial or viral pneumonias which comprises: a) Assessing the level of expression of at least one gene, or any combination thereof comprising between two and eighteen genes, selected from the list consisting of: PRR11, FKBP11, TXNDC11, DIO3, GAS6, HYOU1, CAV1, GINS4, GALM, DNAI7, CCL23, ELL2, UAP1, CDCA2, CADM1, PTGDR2, GPR107 and/or MAPRE3 in a biological sample obtained from the subject, wherein the identification of a statistically significant variation of the level of expression with respect a pre-established threshold level determined in subjects with viral pneumonia, is an indication that the biomarkers signatures may be used for the diagnosis of a *Mycoplasma pneumoniae* infection and/or for the differential diagnosis between a *Mycoplasma pneumoniae* pneumonia and other bacterial or viral pneumonias.

2. *In vitro* method for the diagnosis of a *Mycoplasma pneumoniae* infection and/or for the differential diagnosis between a *Mycoplasma pneumoniae* pneumonia and other bacterial or viral pneumonias, the method comprising determining the level of expression of at least one gene, or any combination thereof comprising between two and eighteen genes, selected from the list consisting of: PRR11, FKBP11, TXNDC11, DIO3, GAS6, HYOU1, CAV1, GINS4, GALM, DNAI7, CCL23, ELL2, UAP1, CDCA2, CADM1, PTGDR2, GPR107 and/or MAPRE3, in a biological sample obtained from the subject.

3. *In vitro* method for selecting the antibiotic treatment for a patient suffering from pneumonia which comprises determining whether the patient is suffering from *Mycoplasma pneumoniae* pneumonia by following the method of claim 2, wherein, if the patient is suffering from *Mycoplasma pneumoniae* pneumonia a treatment with a macrolide antibiotic may be recommended and a treatment with beta-lactam antibiotic can be initially discarded, and/or wherein if the patient is not suffering from *Mycoplasma pneumoniae* pneumonia other clinical decisions could be considered.

4. *In vitro* method, according to claim 3, wherein the beta-lactam antibiotic is amoxicillin, ampicillin or cefotaxime and/or the macrolide antibiotic is azithromycin, clarithromycin or erythromycin.

5. *In vitro use* of at least one gene, or any combination thereof comprising between two and eighteen genes, selected from the list consisting of: PRR11, FKBP11, TXNDC11, DIO3, GAS6, HYOU1, CAV1, GINS4, GALM, DNAI7, CCL23, ELL2, UAP1, CDCA2, CADM1, PTGDR2, GPR107 and/or MAPRE3; or of a kit comprising reagents for the determining the level of expression of the genes; for the diagnosis of a *Mycoplasma pneumoniae* infection and/or for the differential diagnosis between a *Mycoplasma pneumoniae* pneumonia and other bacterial or viral pneumonias and/or for selecting the antibiotic treatment for a patient suffering from pneumonia.

6. *In vitro* method, or *in vitro* use, according to any of the claims 2 to 5, the method comprising determining the level of expression of at least a combination of genes selected from the list shown in Table 1 or Table 2.

7. *In vitro* method, or *in vitro* use, according to any of the claims 2 to 6, which comprises determining the level of expression of at least one combination of genes selected from: DIO3, GALM and TXNDC11; FKBP11, TXNDC11 and DIO3; GAS6, HYOU1, CAV1 and DIO3; or PRR11, DNAI7, GALM, GINS4, TXNDC11 and CAV1.

8. *In vitro* method, or *in vitro* use, according to any of the claims 2 to 7, which comprises determining the level of expression of at least one combination of genes that comprises the genes DIO3, GALM, and TXNDC11 selected from: DNAI7, GALM, GINS4, TXNDC11 and DIO3; CCL23, GALM, TXNDC11, ELL2, DIO3, UAP1 and CDCA2; CCL23, PRR11, GAS6, GALM, TXNDC11, CAV1, DIO3 and CADM1; CCL23, PRR11, DNAI7, GALM, GINS4, TXNDC11, CAV1, DIO3 and PTGDR2; or CCL23, PRR11, DNAI7, GALM, GINS4, TXNDC11, MAPRE3, DIO3, GPR107 and CADM1.

9. *In vitro* method, or *in vitro* use, according to any of the claims 2 to 8, wherein the identification of a higher level of expression with respect a pre-established threshold level determined in subjects with viral pneumonia, is an indication that the subject has a *Mycoplasma pneumoniae* infection and/or that the subject has a *Mycoplasma pneumoniae* pneumonia rather than another bacterial or viral pneumonias and/or that a treatment with a macrolide antibiotic may be recommended and a treatment with beta-lactam antibiotic can be initially discarded.

10. *In vitro* method, or *in vitro* use, according to any of the claims 2 to 9, wherein the biological sample is selected from blood, plasma or serum.
